(19) Europäisches Patentamt
European Patent Office
Office européen des brevets

(11) **EP 4 225 934 B1**

(12) **EUROPEAN PATENT SPECIFICATION**

(45) Date of publication and mention
of the grant of the patent:
**24.07.2024 Bulletin 2024/30**

(21) Application number: **21802873.6**

(22) Date of filing: **08.10.2021**

(51) International Patent Classification (IPC):
*C12Q 1/6804* (2018.01)     *C12Q 1/6869* (2018.01)

(52) Cooperative Patent Classification (CPC):
(C-Sets available)
**C12Q 1/6804; C12Q 1/6869**          (Cont.)

(86) International application number:
**PCT/US2021/054305**

(87) International publication number:
**WO 2022/076912 (14.04.2022 Gazette 2022/15)**

(54) **METHODS AND COMPOSITIONS FOR ANALYZING ANTIGEN BINDING MOLECULES**

VERFAHREN UND ZUSAMMENSETZUNGEN ZUR ANALYSE ANTIGENBINDENDER MOLEKÜLE

PROCÉDÉS ET COMPOSITIONS POUR L'ANALYSE DES MOLÉCULES DE LIAISON AUX ANTIGÈNES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **09.10.2020 US 202063090137 P**

(43) Date of publication of application:
**16.08.2023 Bulletin 2023/33**

(73) Proprietor: **10X Genomics, Inc.
Pleasanton, CA 94588-3260 (US)**

(72) Inventors:
• **MCDONNELL, Wyatt, James
Pleasanton, California 94588-3260 (US)**
• **STUBBINGTON, Michael, John, Terry
Bristol, Bridgewater House Counterslip Bristol
BS1 6BX (GB)**
• **MCDERMOTT, Geoffrey
Pleasanton, California 94588-3260 (US)**

(74) Representative: **J A Kemp LLP
80 Turnmill Street
London EC1M 5QU (GB)**

(56) References cited:
**WO-A1-2018/075693     WO-A1-2019/118355**

• **PAYAM SHAHI ET AL: "Abseq:
Ultrahigh-throughput single cell protein profiling
with droplet microfluidic barcoding", SCIENTIFIC
REPORTS, vol. 7, 14 March 2017 (2017-03-14),
pages 1 - 12, XP055586462, DOI:
10.1038/srep44447**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**C12Q 1/6804, C12Q 2563/149, C12Q 2563/179,
C12Q 2565/514, C12Q 2565/629;
C12Q 1/6869, C12Q 2563/149, C12Q 2563/179,
C12Q 2565/514, C12Q 2565/629**

## Description

## FIELD

The present disclosure relates in some aspects to methods and compositions for analyzing an antigen binding molecule including a secreted antigen receptor, e.g., secreted antibodies.

## BACKGROUND

Methods for antibody discovery typically rely on lower throughput immortalization (e.g., by fusing a primary antibody-producing cell with a myeloma cell to generate a hybridoma) and culturing methods that lead to antibody secretion and testing of a small (typically three or fewer) antigen panel. The hybridomas can be grown in culture, each culture starting with one viable hybridoma cell, producing cultures each of which consists of genetically identical hybridomas which produce one antibody per culture (monoclonal) rather than mixtures of different antibodies (polyclonal). An antigenic bait coupled to a detection reagent such as a fluorophore may be used to detect antibody-antigen interactions in the clonally expanded cells. However, single cell analysis of antibody secretion, e.g., from a primary cell, remains challenging. Methods for efficient analysis of secreted antibodies and antibody-secreting cells, particularly high throughput screening in a heterogeneous population of antibody-secreting cells, are needed. The present disclosure addresses these and other needs.

## BRIEF SUMMARY

The present disclosure provides methods and systems for single cell analyte detection and measurement comprising nucleotide barcoded polypeptides that may be coupled to secreted analytes (e.g., antibodies) from single cells (e.g., antibody-producing cells). The methods and systems described herein may comprise performing next generation sequencing for measuring and analyzing analyte molecules (e.g., antibodies, cytokines, mRNAs, etc.) that may be secreted from and/or present in a single cell. The herein disclosed methods and systems may increase the number of different molecules or analytes that can be measured from a single cell compared to other cell analysis methods. Moreover, the disclosed methods and systems may allow simultaneous measurement of a plurality of cellular molecules (e.g., secreted and/or intracellular molecules), such as secreted analytes (e.g., antibodies,), mRNAs, cell surface proteins, antigen receptor sequences, and antigen binding specificity, etc.

In some embodiments, provided herein are methods and compositions for analyzing a secreted analyte from a single cell (e.g., a primary cell in a heterogeneous population of cells from a subject), such as an antigen binding molecule, e.g., an antibody, for example using a capture agent and/or a cell bead to associate the secreted analyte with the cell from which it is secreted. In some embodiments, secreted antibodies from a cell (e.g., a plasma cell) can be tethered to a surface of the cell (e.g., via a cell-surface bispecific antibody capture agent) and/or to a matrix encapsulating the cell (e.g., a cell bead), and screened for multiple antigen specificities using nucleic-acid based-barcoded antigens. This approach is compatible with a large array of antigens/epitopes and directly pairs antibody heavy chains and/or light chains with the antigens/epitopes, enabling an antibody with an antigen/epitope specificity of interest to be rapidly cloned and converted into a format amenable to further therapeutic development and testing.

In some aspects, provided herein are methods and compositions for analyzing a secreted analyte from a single cell among a plurality of cells, wherein the secreted analyte comprises an antigen binding molecule, such as a secreted antigen receptor, e.g., secreted antibodies. In some aspects, the analysis of the single cell further comprises analyzing one or more other analytes, including protein analytes (e.g., one or more cell surface protein analytes such as receptors and/or ligands, and/or one or more other secreted analytes such as cytokines), nucleic acid analytes (e.g., genomic DNA and/or RNA such as mRNA), carbohydrate analytes, and/or lipid analytes.

Provided herein in one aspect is a method for single cell analysis, comprising: (a) contacting a cell, e.g., antibody-secreting cell, with a reporter agent comprising a reporter nucleic acid molecule to provide a labelled cell, wherein the reporter nucleic acid molecule comprises a reporter sequence corresponding to the reporter agent, wherein the labelled cell comprises a complex coupled to a surface of the cell, and wherein the complex comprises (i) a capture agent, (ii) a secreted antigen binding molecule (e.g., a secreted antigen receptor, such as a secreted antibody or antigen binding fragment thereof), and (iii) the reporter agent; (b) partitioning the labelled cell in a partition, wherein the partition comprises a plurality of barcode nucleic acid molecules which comprise a plurality of partition barcode sequences (e.g., which may be or include a common partition-specific barcode sequence); and (c) in the partition, generating a barcoded nucleic acid molecule from a barcode nucleic acid molecule of the plurality of barcode nucleic acid molecules and the reporter nucleic acid molecule, wherein the barcoded nucleic acid molecule comprises the reporter sequence or a complement thereof and a partition barcode sequence (e.g., the common partition-specific barcode sequence) or a complement thereof.

In any of the preceding embodiments, the cell, e.g., antibody-secreting cell, can be of B-cell lineage, e.g., a plasma cell.

In any of the preceding embodiments, the capture agent can be configured to couple to a cell surface molecule.

In any of the preceding embodiments, said cell surface molecule can be a cell surface protein.

In any of the preceding embodiments, the capture agent can be configured to couple to the secreted antibody or antigen binding fragment thereof.

In any of the preceding embodiments, said capture agent can be configured to couple to both the cell surface protein and said secreted antibody or antigen binding fragment thereof.

In any of the preceding embodiments, said capture agent can be a first antibody binding agent.

In any of the preceding embodiments, the reporter agent can be configured to couple to the secreted antibody or antigen binding fragment thereof.

In any of the preceding embodiments, said reporter agent can be a second antibody binding agent.

In any of the preceding embodiments, said reporter nucleic acid molecule can further comprise a sequence configured to couple to the barcode nucleic acid molecule.

In any of the preceding embodiments, said labelled cell can further comprise a second reporter agent comprising a second reporter nucleic acid molecule.

In any of the preceding embodiments, said second reporter agent can be configured to couple to a second cell surface protein of the cell.

In any of the preceding embodiments, said second reporter nucleic acid molecule can comprise a second reporter sequence corresponding to the second reporter agent.

In any of the preceding embodiments, the partition can further comprise a second barcode nucleic acid molecule of a plurality of second barcode nucleic acid molecules. The second barcode nucleic acid molecule of the plurality of second barcode nucleic acid molecules may include the common partition-specific barcode sequence.

In any of the preceding embodiments, said second reporter nucleic acid molecule can comprise a second sequence configured to couple to the second barcode nucleic acid molecule.

In any of the preceding embodiments, step (c) can further comprise generating a second barcoded nucleic acid molecule from a second barcode nucleic acid molecule from the plurality of second barcode nucleic acid molecules and the second reporter nucleic acid molecule, wherein the second barcoded nucleic acid molecule can comprise sequences from the second reporter nucleic acid molecule, (e.g., the second reporter sequence), and the second barcode nucleic acid molecule, (e.g., the common partition-specific barcode sequence), or complements thereof.

In any of the preceding embodiments, said labelled cell can further comprise a plurality of nucleic acid analytes, wherein a nucleic acid analyte of said plurality of nucleic acid analytes can comprise a nucleic acid analyte sequence.

In any of the preceding embodiments, the partition can further comprise a plurality of third barcode nucleic acid molecules. The plurality of third barcode nucleic acid molecules may include the common partition-specific barcode sequence.

In any of the preceding embodiments, a third barcode nucleic acid molecule of said plurality of third barcode nucleic acid molecules can comprise a third sequence configured to couple to the nucleic acid analyte sequence.

In any of the preceding embodiments, step (c) can further comprise generating a third barcoded nucleic acid molecule from a third barcode nucleic acid molecule from the plurality of third barcode nucleic acid molecules and the nucleic acid analyte, wherein the third barcoded nucleic acid molecule can comprise sequences from the nucleic acid analyte and the third barcode nucleic acid molecule (e.g., the common partition-specific barcode sequence), or complements thereof.

In any of the preceding embodiments, said partition can comprise a support that comprises the plurality of barcode nucleic acid molecules.

In any of the preceding embodiments, said support can be a bead.

In any of the preceding embodiments, said bead can be a gel bead.

In any of the preceding embodiments, said plurality of barcode nucleic acid molecules can be releasably attached to the support.

In any of the preceding embodiments, the partition can be from a plurality of partitions.

In any of the preceding embodiments, the partition can be a droplet or a microwell.

In another aspect, provided herein is a method of single cell analysis, comprising: (a) contacting a cell bead with a reporter agent comprising a reporter nucleic acid molecule to provide a labelled cell bead, wherein the cell bead comprises a cell, e.g., an antibody-secreting cell, in a matrix, wherein the reporter nucleic acid molecule comprises a reporter sequence corresponding to the reporter agent, wherein the labelled cell bead comprises a complex in or on the cell bead, and wherein the complex comprises (i) a capture agent, (ii) a secreted antibody or antigen binding fragment thereof, and (iii) the reporter agent; (b) partitioning the labelled cell bead in a partition, wherein the partition comprises a plurality of barcode nucleic acid molecules which comprise a plurality of partition barcode sequences (e.g., which may be or include a common partition-specific barcode sequence); and (c) in the partition, generating a barcoded nucleic acid molecule from a barcode nucleic acid molecule of the plurality of barcode nucleic acid molecules and the reporter nucleic acid molecule, wherein the barcoded nucleic acid molecule comprises the reporter sequence or a complement thereof and a partition barcode sequence (e.g., the common partition-specific barcode sequence) or a complement thereof.

In any of the preceding embodiments, the cell, e.g., antibody-secreting cell, can be of B-cell lineage, e.g., a plasma cell.

In any of the preceding embodiments, the capture agent can be configured to couple to the matrix of the cell bead.

In any of the preceding embodiments, said matrix can be a degradable polymer matrix.

In any of the preceding embodiments, the capture agent can be configured to couple to the secreted antibody or antigen binding fragment thereof.

In any of the preceding embodiments, said capture agent can be configured to couple to both the matrix and said secreted antibody or antigen binding fragment thereof.

In any of the preceding embodiments, said capture agent can be a first antibody binding agent.

In any of the preceding embodiments, the reporter agent can be configured to couple to the antibody or antigen binding fragment thereof.

In any of the preceding embodiments, said reporter agent can be a second antibody binding agent.

In any of the preceding embodiments, said reporter nucleic acid molecule can further comprise a sequence configured to couple to the barcode nucleic acid molecule.

In any of the preceding embodiments, said cell or said labelled cell bead can further comprise a second reporter agent comprising a second reporter nucleic acid molecule.

In any of the preceding embodiments, said second reporter agent can be configured to couple to a cell surface protein of the cell.

In any of the preceding embodiments, said second reporter nucleic acid molecule can comprise a second reporter sequence corresponding to the second reporter agent.

In any of the preceding embodiments, the partition can further comprise a second barcode nucleic acid molecule of a plurality of second barcode nucleic acid molecules. The second barcode nucleic acid molecule of the plurality of second barcode nucleic acid molecules may include the common partition-specific barcode sequence

In any of the preceding embodiments, said second reporter nucleic acid molecule can comprise a second sequence configured to couple to the second barcode nucleic acid molecule.

In any of the preceding embodiments, step (c) can further comprise generating a second barcoded nucleic acid molecule from a second barcode nucleic acid molecule from the plurality of second barcode nucleic acid molecules and the second reporter nucleic acid molecule, wherein the second barcoded nucleic acid molecule can comprise sequences from the second reporter nucleic acid molecule (e.g., the second reporter sequence) and the second barcode nucleic acid molecule (e.g., the common partition-specific barcode sequence), or complements thereof.

In any of the preceding embodiments, said cell can further comprise a plurality of nucleic acid analytes, wherein a nucleic acid analyte of said plurality of nucleic acid analytes can comprise a nucleic acid analyte sequence.

In any of the preceding embodiments, the partition can further comprise a plurality of third barcode nucleic acid molecules. The plurality of third barcode nucleic acid molecules may include the common partition-specific barcode sequence.

In any of the preceding embodiments, a third barcode nucleic acid molecule of said plurality of third barcode nucleic acid molecules can comprise a third sequence configured to couple to the nucleic acid analyte sequence.

In any of the preceding embodiments, step (c) can further comprise generating a third barcoded nucleic acid molecule from a third barcode nucleic acid molecule from the plurality of third barcode nucleic acid molecules and the nucleic acid analyte, wherein the third barcoded nucleic acid molecule can comprise sequences from the nucleic acid analyte and the third barcode nucleic acid molecule (e.g., the common partition-specific barcode sequence), or complements thereof.

In any of the preceding embodiments, said partition can comprise a support that comprises the plurality of barcode nucleic acid molecules.

In any of the preceding embodiments, said support can be a bead.

In any of the preceding embodiments, said bead can be a gel bead.

In any of the preceding embodiments, said plurality of barcode nucleic acid molecules can be releasably attached to the support.

In any of the preceding embodiments, the partition can be from a plurality of partitions.

In any of the preceding embodiments, the partition can be a droplet or a microwell.

In any of the preceding embodiments, the methods may further include a step (d), in which the generated barcoded nucleic acid molecule is sequenced. If the generated barcoded nucleic acid molecule is sequenced, the cell, e.g., antibody-secreting cell, may be analyzed as secreting an antibody capable of binding the reporter agent, e.g., via detection of the reporter sequence. If the method further (d), sequences the generated barcoded nucleic acid molecule, the generated barcoded nucleic acid molecule may be amplified prior to its being sequenced.

BRIEF DESCRIPTION OF THE DRAWINGS

The drawings illustrate certain embodiments of the features and advantages of this disclosure. These embodiments are not intended to limit the scope of the appended claims in any manner.

**FIG. 1** shows an exemplary microfluidic channel structure for partitioning individual biological particles.

**FIG. 2** shows an exemplary microfluidic channel structure for the controlled partitioning of beads into discrete droplets.

**FIG. 3** shows an exemplary barcode carrying bead.

**FIG. 4** shows another exemplary barcode carrying bead.

**FIG. 5** shows an exemplary microarray schematic.

**FIG. 6** shows an exemplary workflow for processing nucleic acid molecules.

**FIG. 7** shows exemplary methods disclosed herein.

**FIG. 8** shows an exemplary method using a capture agent (e.g., a cell-surface capture agent such as a bispecific antibody).

**FIG. 9** shows exemplary methods disclosed herein.

**FIG. 10A** shows an exemplary method using a cell bead. Secreted antibodies are shown exiting the cell surface and conjugated to the cell bead matrix.

**FIG. 10B** shows an exemplary cell bead with secreted antibody molecules conjugated to the matrix.

**FIG. 11** shows an exemplary illustration of the conversion of barcoded analytes into sequencing libraries.

**FIG. 12** shows an exemplary illustration of the simultaneous measurement of secreted analysts (e.g., secreted antibodies or antigen binding fragment thereof), mRNAs, cell surface proteins, and antigen binding specificity.

**FIG. 13** shows and exemplary schematic of labelling agents.

**FIG. 14** shows another exemplary barcode carrying bead.

FIGS. 15A-D show an exemplary workflow for processing nucleic acid molecules.

**FIG. 16** shows an exemplary computer system that is programmed or otherwise configured to implement methods provided herein.

**DETAILED DESCRIPTION**

The practice of the techniques described herein may employ, unless otherwise indicated, conventional techniques and descriptions of organic chemistry, polymer technology, molecular biology (comprising recombinant techniques), cell biology, biochemistry, and sequencing technology, which are within the skill of those who practice in the art. Such conventional techniques comprise polymer array synthesis, hybridization and ligation of polynucleotides, and detection of hybridization using a label. Specific illustrations of suitable techniques can be had by reference to the examples herein. However, other equivalent conventional procedures can, of course, also be used. Such conventional techniques and descriptions can be found in standard laboratory manuals such as Green, et al., Eds. (1999), Genome Analysis: A Laboratory Manual Series (Vols. I-IV); Weiner, Gabriel, Stephens, Eds. (2007), Genetic Variation: A Laboratory Manual*;* Dieffenbach, Dveksler, Eds. (2003), PCR Primer: A Laboratory Manual; Bowtell and Sambrook (2003), DNA Microarrays: A Molecular Cloning Manual; Mount (2004), Bioinformatics: Sequence and Genome Analysis; Sambrook and Russell (2006), Condensed Protocols from Molecular Cloning: A Laboratory Manual; and Sambrook and Russell (2002), Molecular Cloning: A Laboratory Manual (all from Cold Spring Harbor Laboratory Press); Stryer, L. (1995) Biochemistry (4th Ed.) W. H. Freeman, New York N.Y.; Gait, "Oligonucleotide Synthesis: A Practical Approach" 1984, IRL Press, London; Nelson and Cox (2000), Lehninger, Principles of Biochemistry 3rd Ed., W. H. Freeman Pub., New York, N.Y.; and Berg et al. (2002) Biochemistry, 5th Ed., W. H. Freeman Pub., New York, N.Y..

If a definition set forth herein is contrary to or otherwise inconsistent with a definition set forth in the patents, applications, published applications and other publications, the definition set forth herein prevails over the definition that is incorporated herein by reference.

The section headings used herein are for organizational purposes only and are not to be construed as limiting the subject matter described.

**I. OVERVIEW**

Provided herein are methods and compositions for analyzing an antigen binding molecule including a secreted antigen receptor, e.g., secreted antibodies. While secreted antibodies are discussed as exemplary analytes throughout, it should be appreciated that other antigen binding molecules are encompassed by the present disclosure. In some embodiments, provided herein are methods and systems for profiling immune cells, e.g., cells of the B-lineage, and analyte detection and measurement, utilizing nucleotide barcoded polypeptides (e.g., reporter agents, e.g., antibody binding agents, e.g., antigens) to screen these cells for multiple antigen specificities, e.g., antigen specificities of secreted antibodies. The methods and systems described herein may comprise performing next generation sequencing for measuring and analyzing analyte molecules (e.g., antibodies) that may be secreted from and/or present in a single cell. The herein disclosed methods and systems may increase the number of different molecules or analytes that can be measured from a single cell compared to other cell analysis methods. Moreover, the disclosed methods and systems may allow simultaneous measurement of a plurality of cellular molecules (e.g., secreted and/or intracellular molecules), such as secreted analytes (e.g., antibodies), mRNAs, cell surface proteins, and antigen binding specificity, etc.

B cells produce biomolecules with extremely potent (e.g., sub-picomolar) binding capacity to their targets - antibodies. Antibodies produced by B cells are either tethered to the surface of the B cell (e.g., as in the case of naive or memory B cells, in the form of BCRs) or secreted by the B cell (e.g., as in the case of plasma cells or other antibody-secreting effector cells). In some aspects, this interaction is determined by which exon of the antibody heavy chain constant region is present in the transcribed RNA, as BCRs comprise a transmembrane domain that is absence in secreted antibodies. Secreted antibodies (e.g., by plasma cells) include antibodies that are detectable in serum, which typically comprise antibodies that are highly affinity matured, mirroring the mature developmental status of a plasma cell.

For BCRs tethered to the cell surface (e.g., as in the case of memory B cells), the BCR-antigen interaction can be detected and used to enrich for antigen-binding B cells (e.g., using flow cytometry or magnetic enrichment) as the antigen is tightly bound to the cell surface-bound BCRs. However, in the case of secreted antibodies, this interaction is disrupted and difficult to stably measure, and a secreted antibody may quickly float away from the secreting cell and mix with antibodies secreted by other cells. In some embodiments disclosed herein, a secreted antibody is captured, e.g., on a surface of the cell from which the antibody is secreted, in a droplet (e.g., containing a bead such as a gel bead, magnetic bead or paramagnetic bead) containing the cell, and/or in a matrix surrounding (e.g., encapsulating) the cell, for subsequent single cell analysis of the cell and the antibody molecules that it secretes. In some embodiments, a method disclosed herein is used for screening single cells in a heterogeneous population of cells, such as plasma cells in a subject.

In some embodiments herein, the method comprises contacting a cell, such as an isolated cell of B-cell lineage (e.g., a plasma cell), with a reporter agent, comprising a reporter nucleic acid molecule. In some embodiments, the reporter nucleic acid molecule comprises a reporter sequence corresponding to or identifying the reporter agent. For example, the reporter sequence may be an oligonucleotide DNA barcode sequence, creating a labelled cell. The reporter agent may be one or more reporter barcoded second antibody binding agents (e.g., antigens), wherein a reporter barcode sequence, e.g., reporter sequence, is a unique oligonucleotide that can be amplified downstream. In some embodiments, the labelled cell comprises a complex coupled to a surface of the cell, and wherein the complex comprises (i) a capture agent, (ii) a secreted antibody or antigen binding fragment thereof, and (iii) the reporter agent.

In some embodiments herein, the method may further comprise enriching for antigen-specific cells (e.g., plasma cells) by secondary enrichment against the antigen, e.g., targeting the fluorophore or another part of the antigen (as a target for enrichment) and use this to produce a single cell suspension for partitioning, or by forming cell beads in a selective fashion around only cells binding the antigens of interest.

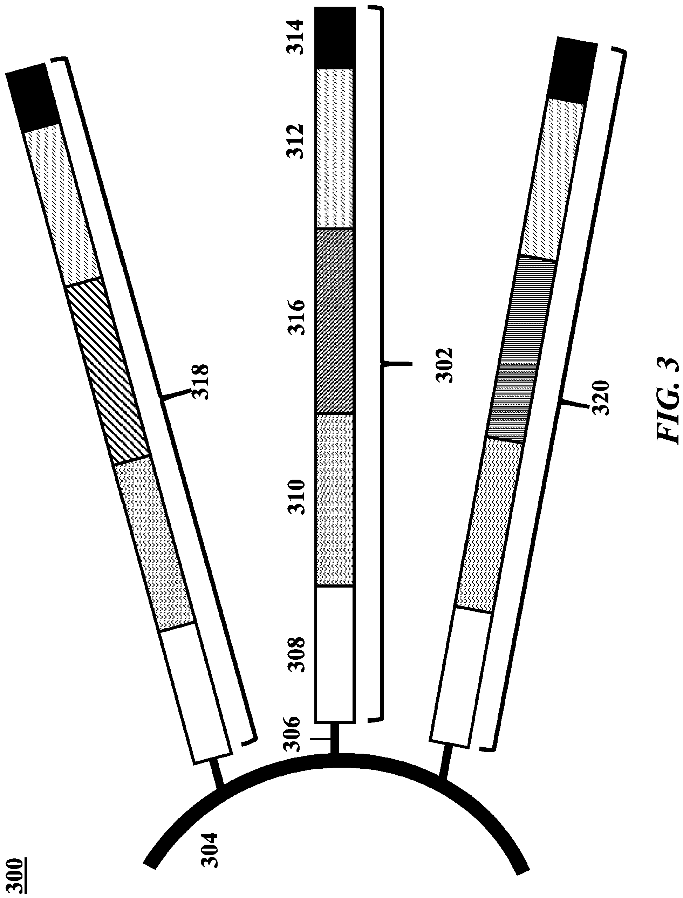

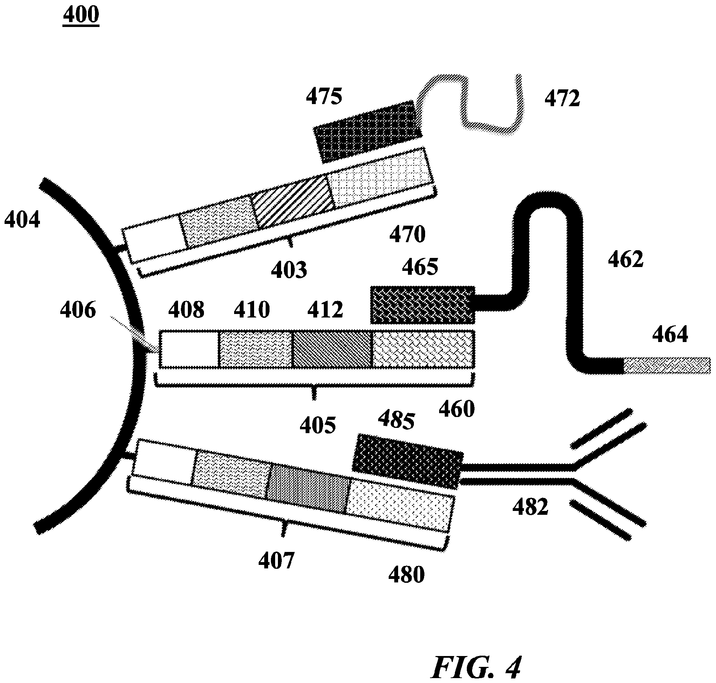

In some embodiments herein, the labelled cell (or a cell bead formed around a labelled cell) is partitioned in a partition (e.g., a droplet such as an emulsion droplet), wherein the partition comprises a plurality of barcode nucleic acid molecules which comprise a plurality of partition barcode sequences. As described for **FIG. 4,** e.g.,

paragraph 322, the plurality of partition barcode sequences may be a common partition-specific sequence to the partition **(410)**. See also description of **FIG. 3** at, e.g., paragraph 319, in reference to element **310.** Use of a common partition-specific barcode sequence, as described in paragraph 340, can be useful to associate one or more analytes with the labelled cell partitioned in the partition.

In some embodiments, a barcoded nucleic acid molecule is generated from a barcode nucleic acid molecule of the plurality of barcode nucleic acid molecules and the reporter nucleic acid molecule, wherein the barcoded nucleic acid molecule comprises the reporter sequence or a complement thereof and a partition barcode sequence or a complement thereof.

In some embodiments, the capture agent is configured to couple to a cell surface molecule. In some embodiments, the cell surface molecule is a cell surface protein, e.g., a cell surface receptor such as CD19, CD20, CD45, or CD22. In some embodiments, the capture agent is configured to couple to the secreted antibody or antigen binding fragment thereof. For example, the capture agent may comprise one or more immunoglobulin (e.g., antibody) molecules (e.g., IgA, IgE, IgG, or IgM molecules) or any fragments (e.g., epitope-binding fragments) or derivatives thereof, and in any suitable combination. In some embodiments, the capture agent may be configured to couple to both the cell surface protein and the secreted antibody or antigen binding fragment thereof. In some examples, the capture agent is a first antibody binding agent. For example, a capture agent may be a multi-specific antibody, e.g., a bispecific antibody capable of binding to a cell surface molecule and to the secreted antibody or antigen binding fragment thereof.

In some embodiments, the capture agent may be tethered to a lipid, enabling insertion of the biomolecule into a cellular membrane where it could then tether the secreted antibody to the surface of the cell. In some embodiments, the capture agent may be configured to couple to both the cell surface protein and the secreted antibody or antigen binding fragment thereof. For example, a capture agent may be tethered to a lipid and comprise an antibody capable of binding to the secreted antibody or antigen binding fragment thereof.

In some examples, the method further comprises enriching for antigen-specific cells (e.g., plasma cells) by secondary enrichment against the antigen, e.g., by forming cell beads in a selective fashion around cells secreting the antibodies binding the antigens of interest. For example, a hydrogel matrix may be formed by addition of hydrogen peroxide if the antigen has a horseradish peroxidase or similar domain conjugated, thus forming cell beads around only plasma cells binding the antigens of interest.

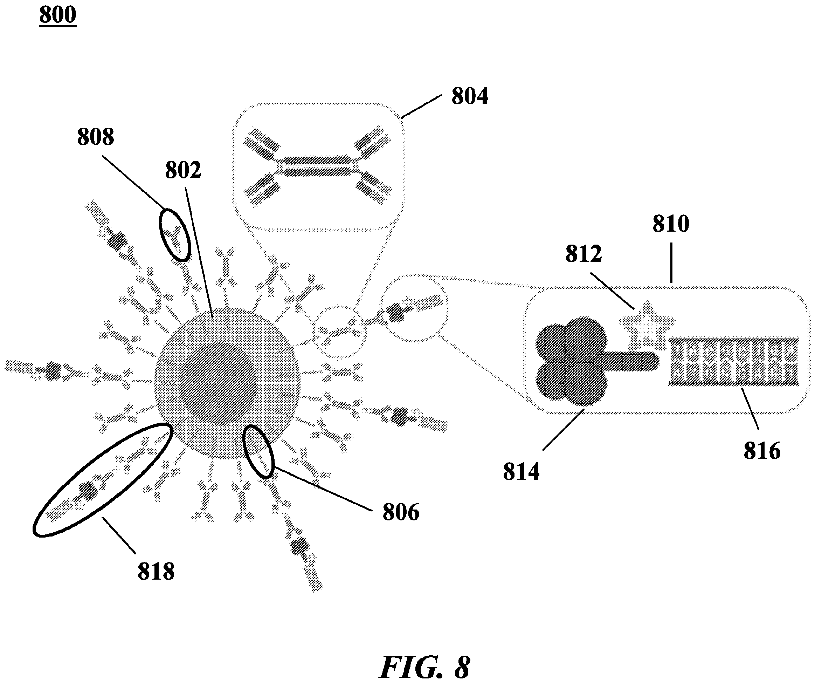

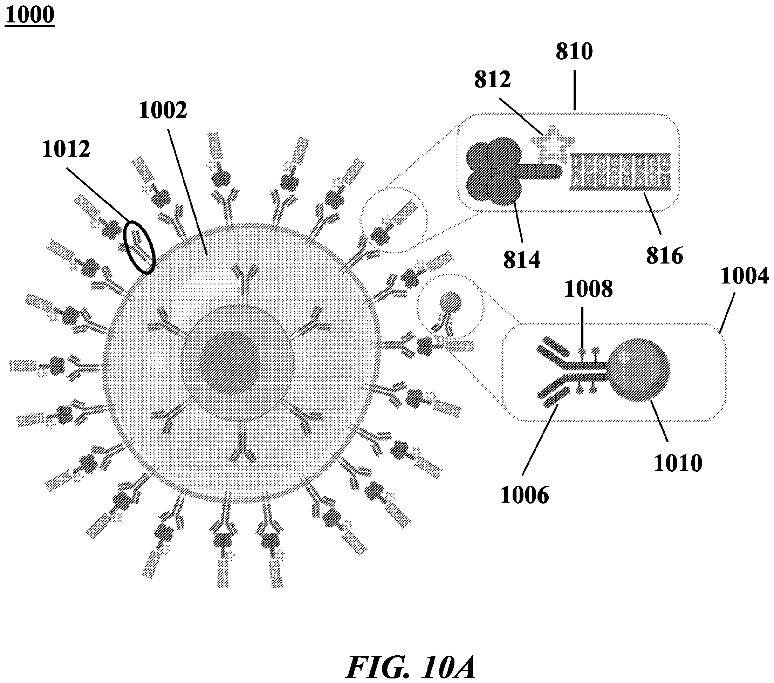

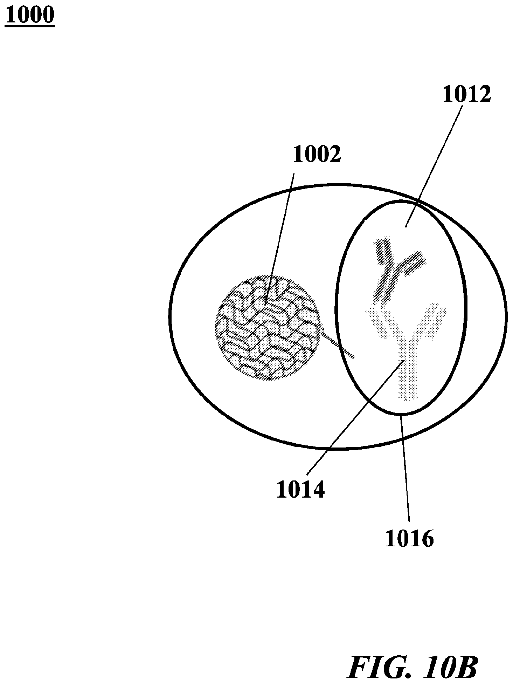

In some embodiments, the reporter agent may include a nucleic acid molecule and is configured to couple to the secreted antibody or antigen binding fragment thereof. In some examples, the reporter agent is a second antibody binding agent, e.g., an antigen or epitope conjugated with a fluorophore or enrichment target (e.g., **812** shown in **FIG. 8** or **FIG. 10**). In some examples, said reporter nucleic acid molecule further comprises a sequence configured to couple to a barcode nucleic acid molecule, e.g., hybridizing the barcode nucleic acid molecule to the reporter nucleic acid molecule, optionally followed by a ligation reaction or a nucleic acid extension reaction. In some embodiments, inclusion of controls such as oligonucleotide, oligonucleotide-barcoded tetramerized fluorophore, fluorophore, e.g., phycoerythrin (PE) or allophycocyanin (APC) or capture agent could be used to filter antigen binding cells not truly binding the antigen or epitope, or to remove cells, if the cells bound to an irrelevant antigen such as hemoglobin, myoglobin, or an irrelevant antigen. Examples of control antigens that may be used to filter antigen binding cells whose secreted antibodies do not truly bind the reporter agents' antibody binding agent, e.g, antigen or epitope, may be off-target antigens or antigens to which a subject (e.g., a human subject or subject from whom antibody-secreting cells are isolated from) would not be expected to develop an antibody response to or to have antibodies with a specificity for. Examples of such non-target antigens may be antigens endogenous to and abundantly expressed in a subject, e.g., a human subject, e.g., human serum albumin (HSA). The control antigens may be coupled to biotin, streptavidin or biotin conjugated with streptavidin. The control antigens may further be detectably labeled or may be detectable labels, e.g., detectable labels that may optionally be included as part of the reporter agent. In some embodiments, the single cell suspensions and/or cell bead-gel beads of interest, e.g., including one or more labelled cells, are captured and analyzed, for example, using barcoding and/or V(D)J amplification using the Immune Profiling Solution of the 10× Genomics Chromium™ system. This produces a vector of antigen-specific counts for each antigen, for each captured B cell, enabling the identification of the antigen specificity of that cell using approaches for count transformation and imputation appreciable by those knowledgeable in the art.

In some embodiments, a method disclosed herein comprises a multi-omic approach for the analysis of a secreted antibody or antigen binding fragment thereof, as well the analysis of a cell surface molecule (e.g., protein) of interest. In some embodiments, the labelled cell further comprises a second reporter agent comprising a second reporter nucleic acid molecule. In some embodiments, the second reporter agent is configured to couple to a second cell surface protein of the cell, and said second reporter nucleic acid molecule comprises a second reporter sequence corresponding to the second reporter agent. The second reporter sequence may be different than the first reporter sequence, in order to distinguish between the two reporter agents during the detection and analysis processes.

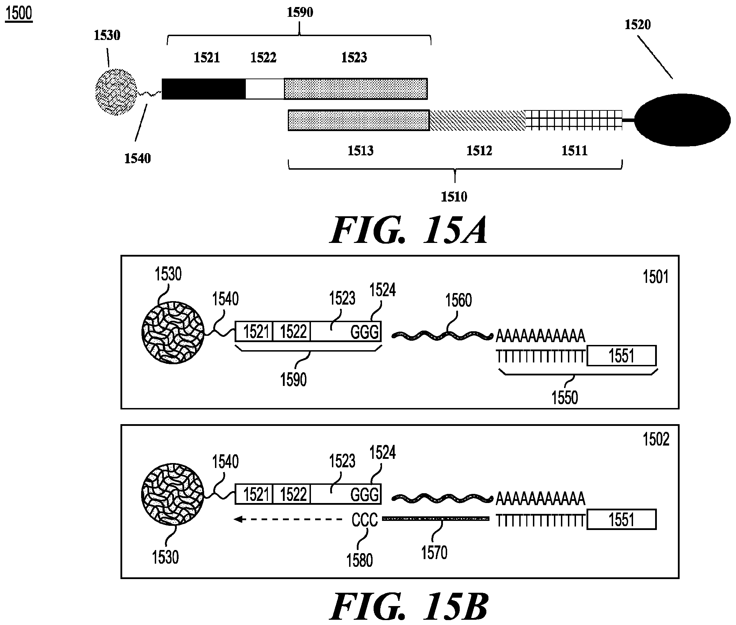

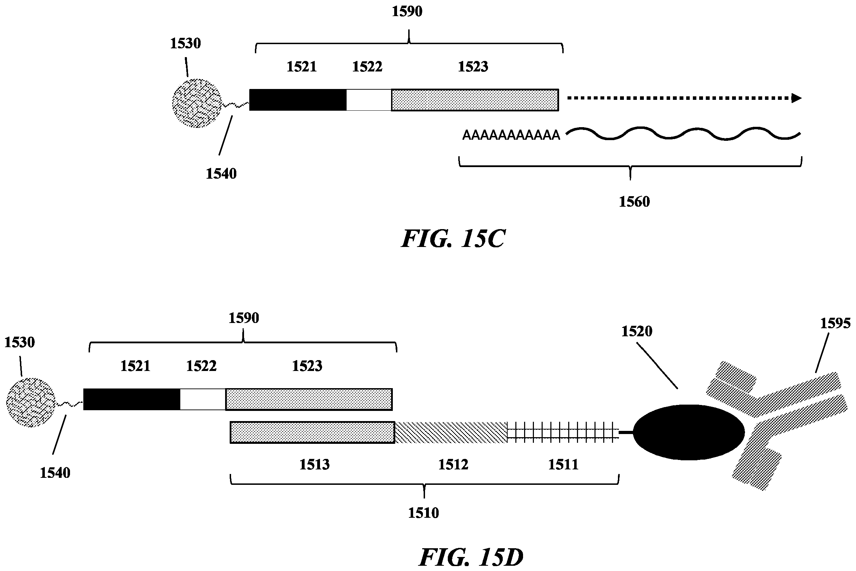

In some embodiments, the partition further comprises a second barcode nucleic acid molecule of a plurality of second barcode nucleic acid molecules. In some embodiments, the second reporter nucleic acid molecule comprises a second sequence configured to couple to the second barcode nucleic acid molecule, e.g., hybridizing the second barcode nucleic acid molecule to the second reporter nucleic acid molecule, optionally followed by a ligation reaction or a nucleic acid extension reaction. In some embodiments, a method further comprises generating a second barcoded nucleic acid molecule from a second barcode nucleic acid molecule from the plurality of second barcode nucleic acid molecules and the second reporter nucleic acid molecule, wherein the second barcoded nucleic acid molecule comprises sequences from the second reporter nucleic acid molecule and the second barcode nucleic acid molecule, or complements thereof. The second barcoded nucleic acid molecule may include the second reporter sequence of the second reporter nucleic acid molecule and a partition specific barcode sequence of the second barcode nucleic acid molecule, or complements thereof, as described in as described in the description references to **FIG. 3** and **FIG. 15 (1522, 1512)**, e.g., at paragraph 360-362. As later discussed in **"A. Generation of Barcoded Molecules"** and **"B. Multiplexing Methods"** of **"VII. DETECTION AND ANALYSIS"**, e.g., paragraphs 319 and 340, the second barcode nucleic acid molecule of the plurality of second barcode nucleic acid molecules may be or include a partition or cell barcode that is the same as the partition or cell barcode of the barcode nucleic acid molecule of the plurality of barcode nucleic acid molecules so as to associate one or more analytes with the labelled cell partitioned in the partition.

In some embodiments, a method disclosed herein comprises a multi-omic approach for the identification of a secreted antibody or antigen binding fragment thereof, as well the analysis of a cell surface molecule (e.g., protein) of interest and/or a nucleic acid molecule (e.g., an RNA) of interest. In some embodiments, the labelled cell comprises a plurality of nucleic acid analytes, wherein a nucleic acid analyte of said plurality of nucleic acid analytes comprises a nucleic acid analyte sequence (e.g., a VDJ sequence or a VJ sequence for the heavy chain variable and light chain variable regions, respectively, of the antibody secreted by the cell). In some embodiments, the partition further comprises a third barcode nucleic acid molecule of a plurality of third barcode nucleic acid molecules. In some examples, a third barcode nucleic acid molecule of said plurality of third barcode nucleic acid molecules comprises a third sequence configured to couple to the nucleic acid analyte sequence, e.g., hybridizing the third barcode nucleic acid molecule to the nucleic acid analyte sequence, optionally followed by a ligation reaction or a nucleic acid extension reaction. In some embodiments, a method further comprises generating a third barcoded nucleic acid molecule from a third barcode nucleic acid molecule from the plurality of third barcode nucleic acid molecules and the nucleic acid analyte, wherein the third barcoded nucleic acid molecule comprises sequences from the nucleic acid analyte and the third barcode nucleic acid molecule, or complements thereof. The third barcoded nucleic acid molecule may include sequences from the nucleic acid analyte and a partition specific barcode sequence of the third barcode nucleic acid molecule, or complements thereof, as described herein in the description references to **FIG. 3.** As later discussed in **"A. Generation of Barcoded Molecules"** and **"B. Multiplexing Methods"** of **"VII. DETECTION AND ANALYSIS",** e.g., paragraphs 319 and 340, the third barcode nucleic acid molecule of the plurality of third barcode nucleic acid molecules may include a partition or cell barcode that is the same as the partition or cell barcode of the barcode nucleic acid molecule of the plurality of barcode nucleic acid molecules so as to associate one or more analytes with the labelled cell partitioned in the partition.

In some embodiments, the partition comprises a support that comprises or is coupled to the plurality of barcode nucleic acid molecules. In some examples, the support is a bead. In some examples, the bead is a gel bead. The gel bead may include a polymer matrix (e.g., matrix formed by polymerization or cross-linking), that includes one or more polymers comprising different functional groups. In some embodiments, the plurality of barcode nucleic acid molecules are releasably attached to the support, and/or randomly distributed in or on the support matrix. In some embodiments, the partition is from a plurality of partitions. In some embodiments, the partitions is a droplet or a microwell.

In some alternative embodiments, the method comprises contacting a cell bead with a reporter agent comprising a reporter nucleic acid molecule to provide a labelled cell bead, wherein the cell bead comprises a cell in a matrix, e.g., a hydrogel/polymer matrix. In some examples, the matrix is collagen, laminin, and/or fibronectin. The cell bead may be generated, for example, by fixing a cell in a fixation medium or by cross-linking elements of the cell, such as the cell membrane or cell cytoskeleton.

In some embodiments herein, the method may further comprise enriching antigen-specific cells (e.g., plasma cells) and/or cell beads containing antigen-specific cells by enrichment against the antigen, e.g., targeting the fluorophore or another part of the antigen (as a target for enrichment) and use this to produce a suspension of single cells or cell beads for partitioning, or by forming cell beads in a selective fashion around only cell beads having cells secreting antibodies that bind the antigens of interest.

In some embodiments, the reporter nucleic acid molecule comprises a reporter sequence corresponding to the reporter agent, wherein the labelled cell bead comprises a complex in or on the cell bead, and wherein the complex comprises (i) a capture agent, (ii) a secreted antibody or antigen binding fragment thereof, and (iii) the reporter agent. In some embodiments, the method further comprises partitioning the labelled cell bead in a partition, wherein the partition comprises a plurality of barcode nucleic acid molecules, which comprise a plurality of partition barcode sequences, e.g., barcodes unique to each partition. As described for **FIG. 4,** e.g., paragraph 322, the plurality of partition barcode sequences may be a common partition-specific sequence to the partition **(410).** See also description of **FIG. 3** at, e.g., paragraph 319, in reference to element **310.** Use of a common partition-specific barcode sequence, as described in paragraph 340, can associate one or more analytes with the labelled cell partitioned in the partition.

In some embodiments, the method further comprises, within the partition, generating a barcoded nucleic acid molecule from a barcode nucleic acid molecule of the plurality of barcode nucleic acid molecules and the reporter nucleic acid molecule, wherein the barcoded nucleic acid molecule comprises the reporter sequence or a complement thereof and a partition barcode sequence or a complement thereof. In some embodiments, the cell encapsulated within the cell bead is of a B-cell lineage, e.g., a plasma cell.

In some embodiments, the capture agent is configured to couple to the matrix, of the cell bead. In some examples, a cell bead may be a macromolecule formed of nucleic acid molecules bound together. In some embodiments, the matrix of the cell bead is a degradable polymer matrix, e.g., matrix formed by polymerization or cross-linking that are randomly arranged, such as in random copolymers, and/or have ordered structures, such as in block copolymers. In some embodiments, the capture agent is configured to couple to the secreted antibody or antigen binding fragment thereof. In some embodiments, said capture agent is configured to couple to both the matrix and said secreted antibody or antigen binding fragment thereof. In some embodiments, said capture agent is a first antibody binding agent.

In some embodiments, the reporter agent includes a nucleic acid molecule and is configured to couple to the antibody or antigen binding fragment thereof. In some embodiments, said reporter agent includes a second antibody binding agent, e.g., and antigen conjugated with a fluorophore or enrichment target. In some embodiments, said reporter nucleic acid molecule, e.g., a reporter barcode sequence identifying the reporter agent, further comprises a sequence configured to couple to the barcode nucleic acid molecule. In some embodiments, inclusion of controls, disclosed in paragraph 89, such as an oligonucleotide-barcoded tetramerized fluorophore could be used to filter antigen binding cells not truly binding the antigen, or to remove cells, or prevent cell bead formation, if the cells bound to an irrelevant antigen such as hemoglobin, myoglobin, or an irrelevant antigen. In some embodiments, the cell beads of interest are captured and analyzed, for example, using barcoding and/or V(D)J amplification using the Immune Profiling Solution of the $10\times$ Genomics Chromium™ system. This produces a vector of antigen-specific counts for each antigen, for each captured B cell, enabling the identification of the antigen specificity of that cell using approaches for count transformation and imputation appreciable by those knowledgeable in the art.

In some embodiments, a method disclosed herein comprises a multi-omic approach for the analysis of a secreted antibody or antigen binding fragment thereof, as well as the analysis of a cell surface molecule (e.g., protein). In some embodiments, said cell or said labelled cell bead further comprises a second reporter agent comprising a second reporter nucleic acid molecule. In some embodiments, said second reporter agent is configured to couple to a cell surface protein of the cell, e.g. a CD surface marker such as CD45. In some embodiments, said second reporter nucleic acid molecule comprises a second reporter sequence corresponding to the second reporter agent.

In some embodiments, the partition further comprises a second barcode nucleic acid molecule of a plurality of second barcode nucleic acid molecules, e.g., cell or partition-specific barcode, such as a barcode attached to a bead, e.g., a gel bead. In some embodiments, said second reporter nucleic acid molecule comprises a second sequence configured to couple to the second barcode nucleic acid molecule. In some embodiments, the method further comprises generating a second barcoded nucleic acid molecule from a second barcode nucleic acid molecule from the plurality of second barcode nucleic acid molecules and the second reporter nucleic acid molecule, wherein the second barcoded nucleic acid molecule comprises sequences from the second reporter nucleic acid molecule and the second barcode nucleic acid molecule, or complements thereof. The second barcoded nucleic acid molecule may include the second reporter sequence of the second reporter nucleic acid molecule and a cell or partition specific barcode sequence of the second barcode nucleic acid molecule, or complements thereof, as described in as described in the description references to **FIG. 3** and **FIG. 15 (1522, 1512)**, e.g., at paragraph 360-362. As later discussed in **"A. Generation of Barcoded Molecules"** and **"B. Multiplexing Methods"** of **"VII. DETECTION AND ANALYSIS"**, e.g., paragraphs 319 and 341, the second barcode nucleic acid molecule of the plurality of second barcode nucleic acid molecules may be or may include a partition or cell barcode that is the same as the partition or cell barcode of the barcode nucleic acid molecule of the plurality of barcode nucleic acid molecules so as to associate one or more analytes with the labelled cell partitioned in the partition.

In some embodiments, a method disclosed herein comprises a multi-omic approach for the analysis of a secreted antibody or antigen binding fragment thereof, as well as the analysis of a cell surface molecule (e.g., protein) and/or a nucleic acid molecule (e.g., an RNA). In some embodiments, said cell, e.g., comprised in a cell bead, further comprises a plurality of nucleic acid analytes (e.g., mRNA transcripts containing a VDJ sequence or a VJ sequence for the heavy chain variable and light chain variable regions, respectively, of the antibody secreted by the cell), wherein a nucleic acid analyte of said plurality of nucleic acid analytes comprises a nucleic acid analyte sequence. In some embodiments, the partition further comprises a plurality of third barcode nucleic acid molecules. In some embodiments, a third barcode nucleic acid molecule of said plurality of third barcode nucleic acid molecules comprises a third sequence configured to couple to the nucleic acid analyte sequence. In some embodiments, the method further comprises generating a third barcoded nucleic acid molecule from a third barcode nucleic acid molecule from the plurality of third barcode nucleic acid molecules and the nucleic acid analyte, wherein the third barcoded nucleic acid molecule comprises sequences from the nucleic acid analyte and the third barcode nucleic acid molecule, or complements thereof. The third barcoded nucleic acid molecule may include sequences from the nucleic acid analyte and a partition specific barcode sequence of the third barcode nucleic acid molecule, or complements thereof, as described herein in the description references to **FIG. 3**. As later discussed in **"A. Generation of Barcoded Molecules"** and **"B. Multiplexing Methods"** of **"VII. DETECTION AND ANALYSIS"**, e.g., paragraphs 319 and 340, the third barcode nucleic acid molecule of the plurality of third barcode nucleic acid molecules may include a partition or cell barcode that is the same as the partition or cell barcode of the barcode nucleic acid molecule of the plurality of barcode nucleic acid molecules so as to associate one or more analytes with the labelled cell partitioned in the partition.

In some embodiments, the partition comprises a support that comprises the plurality of barcode nucleic acid molecules. In some examples, the support is a bead. In some examples, the bead is a gel bead. The gel bead may include a polymer matrix (e.g., matrix formed by polymerization or cross-linking), that includes one or more polymers comprising different functional groups. In some embodiments, the plurality of barcode nucleic acid molecules are releasbly attached to the support, and randomly distributed in or on the support matrix. In some embodiments, the partition is from a plurality of partitions. In some embodiments, the partitions is a droplet or a microwell.

In some embodiments, the analytical procedure of the captured cell beads or single cell suspensions of interest include all, or some, of the following approaches: 1) identifying cells with a reasonable number of antigen-binding counts, 2) identifying of cells which also have additional multiomic counts such as cellular RNA or surface protein as a filtering step, 3) transforming the antigen-binding count distribution by subtracting the median or mean background for each antigen as detected in empty droplets, 4) excluding cells that bind the antigen of interest and the control antigen, or just the control antigen, from further analysis 5) transforming the antigen count vector using any number of approaches (e.g., centered log ratio, Principal Component Analysis (PCA), generalized PCA (GLMPCA), more complex machine learning models or neural networks), and 6) choose antibodies with antigen fingerprints for therapeutic evaluation. In some embodiments, the cDNA library is used to selectively amplify plasma cell or secreted antibodies of interest by using primers hybridizing to a given cell barcode/cell of interest and the heavy chain and light chain constant regions. These heavy and light chains may be amplified separately, and overlap extension PCR may combine them into a single continuous molecule.

## II. CELLS AND ANALYTES

In some embodiments, a cell disclosed herein may include a naturally antigen receptor (e.g., antibody) secreting cell or derivatives (e.g., fused, transformed, or immortalized cells), a cell engineered or modified to secrete an antigen receptor (e.g., antibody) or antigen-binding fragment thereof. An antigen receptor disclosed herein may include naturally occurring and engineered antibodies (including fragments, fusions, and variants thereof), naturally occurring and engineered B-cell receptors (BCRs) (including fragments, fusions, and variants thereof), naturally occurring and engineered T-cell receptors (TCRs) (including fragments, fusions, and variants thereof, such as soluble TCRs), and chimeric antigen receptors (CARs) (including fragments, fusions, and variants thereof). In addition, an antigen receptor disclosed herein may be soluble or be part of an extracellular vesicle such as an exosome or a microvesicle from a cell.

A key aspect of the adaptive immune response is the rapid production of high-affinity antibodies (Abs), such as plasma cells. This antibody production is the function of an antibody secreting cell, which arises from naive (or memory) B cells as they encounter antigen, activate, proliferate, and differentiate. Antibodies secreted by plasma cells is the set of antibodies that are detectable in serum, and typically is comprised of antibodies that are highly affinity matured (mirroring the mature developmental status of a plasma cell).

In some embodiments, a cell disclosed herein may include any type of antibody secreting cell, including without limitation a B cell, such as an antigen-specific memory B cell, plasma cell isolated after immunization or vaccination, or a memory B cell co-incubate with the antigen of interest, anti-CD40L under sufficient conditions to antigen-specific memory B cells to antigen-specific plasma cells.

In some embodiments herein, prior to analysis, the cell populations of interest, e.g., B cells, are isolated. For example, isolated cell populations may be: antigen-specific memory B cells isolated using flow cytometry or magnetic enrichment with an antigen bound to the surface, plasma cells isolated after immunization or vaccination and captured by flow cytometry, magnetic enrichment, buoyancy-activated cell sorting (BACS), or another method, or memory B cells co-incubated with the antigen of interest, anti-CD40L, and appropriate culture medium (converting antigen-specific memory B cells to antigen-specific plasma cells).

In some embodiments, a sample disclosed herein comprises one or more cells (e.g., immune cells such as a T cells, B cells, plasma cells, and/or dendritic cells) and are analyzed using the herein disclosed methods and systems. The sample may be a cell line or cell culture sample. The sample can include one or more microbes. The sample may be derived from another sample. The sample may be a tissue sample, such as a biopsy, core biopsy, needle aspirate, or fine needle aspirate. The sample may be a fluid sample, such as blood, urine, saliva, plasma, serum, mucosal excretion, sputum, stool and tears. A sample may be isolated in various ways. For example, a cell sample may be isolated from a biological sample such as a bodily fluid, such as plasma or may be isolated from a specific tissue or organ from a subject. In some embodiments, B cell populations of interest are isolated using flow cytometry (e.g., FACS), magnetic enrichment with an antigen bound to the surface (MACS), BACS, or another method, as described herein.

In some embodiments, an analyte disclosed herein may include any molecule that specifically binds an antigenic determinant. Examples of antigen binding molecules are immunoglobulins and derivatives, e.g. fragments, thereof, and generally refer to a portion of a complete antibody (e.g., comprising each domain of the light and heavy chains respectively) capable of binding the same epitope/antigen as the complete antibody, albeit not necessarily to the same extent. Although multiple types of antigen binding molecules are possible, an antigen binding fragment typically comprises at least one pair of heavy and light chain variable regions (VH and VL, respectively) held together (e.g., by disulfide bonds) to preserve the antigen binding site and does not contain all or a portion of the Fc region. Antigen binding fragments of an antibody can be obtained from a given antibody by any suitable technique (e.g., recombinant DNA technology or enzymatic or chemical cleavage of a complete antibody), and typically can be screened for specificity in the same manner in which complete antibodies are screened. In some embodiments, an antigen binding fragment comprises an F(ab')$_2$ fragment, Fab' fragment, Fab fragment, Fd fragment, or Fv fragment. In some embodiments, an antibody disclosed herein may include antibody-derived polypeptides, such as single chain variable fragments (scFv), diabodies or other multimeric scFvs, heavy chain antibodies, single domain antibodies, or other polypeptides comprising a sufficient portion of an antibody (e.g., one or more complementarity determining regions (CDRs)) to confer specific antigen binding ability to the polypeptide. In some embodiments, an analyte is secreted antibody or antigen binding fragment thereof. In some examples, secreted antibodies may be produced by B cells and may be secreted by the B cell (e.g., plasma cells, wherein antibodies are detectable in serum).

In some embodiments, an analyte is a secreted antibody comprising an immunoglobulin molecule, e.g., a protein having the structure of a naturally occurring antibody. For example, immunoglobulins of the IgG class are heterotetrameric glycoproteins of about 150,000 daltons, composed of two light chains and two heavy chains that are disulfide-bonded. From N- to C-terminus, each heavy chain has a variable domain (VH), also called a variable heavy domain or a heavy chain variable region, followed by three constant domains (CH1, CH2, and CH3), also called a heavy chain constant region. Similarly, from N- to C-terminus, each light chain has a variable domain (VL), also called a variable light domain or a light chain variable region, followed by a constant light (CL) domain, also called a light chain constant region. The heavy chain of an immunoglobulin may be assigned to one of five types, called $\alpha$ (IgA), $\delta$ (IgD), $\varepsilon$ (IgE), $\gamma$ (IgG), or $\mu$ (IgM), some of which may be further divided into subtypes, e.g. $\gamma_1$ (IgG$_1$), $\gamma_2$ (IgG$_2$), $\gamma_3$ (IgG$_3$), $\gamma_4$ (IgG$_4$), $\alpha_1$ (IgA$_1$) and $\alpha_2$ (IgA$_2$). The light chain of an immunoglobulin may be assigned to one of two types, called kappa ($\kappa$) and lambda ($\lambda$), based on the amino acid sequence of its constant domain. An immunoglobulin essentially consists of two Fab molecules and an Fc domain, linked via the immunoglobulin hinge region.

In some embodiments, an analyte produced by a cell disclosed herein is an antibody targeting any suitable antigen or epitope. For example, the antigens can include, but are not limited to, any antigens associated with a pathogen, including viral antigens, fungal antigens, bacterial antigens, helminth antigens, parasitic antigens, ectoparasite antigens, protozoan antigens, or antigens from any other infectious agent. Antigens can also include any antigens associated with a particular disease or condition, whether from pathogenic or cellular sources, including, but not limited to, cancer antigens, antigens associated with an autoimmune disease (e.g., diabetes antigens), allergy antigens (allergens), mammalian cell molecules harboring one or more mutated amino acids, proteins normally expressed pre- or neo-natally by mammalian cells, proteins whose expression is induced by insertion of an epidemiologic agent (e.g. virus), proteins whose expression is induced by gene translocation, and proteins whose expression is induced by mutation of regulatory sequences. These antigens can be native antigens or genetically engineered antigens which have been modified in some manner (e.g., sequence change or generation of a fusion protein). It will be appreciated that in some embodiments (i.e., when the antigen is expressed by the yeast vehicle from a recombinant nucleic acid molecule), the antigen can be a protein or any epitope of immunogenic domain thereof, a fusion protein, or a chimeric protein, rather than an entire cell or microorganism. An antigen may be one of any associated with infectious disease, cancer, or autoimmune disease. An antigen may be of native biological origin or genetically engineered. Antigens related to infectious disease include but are not limited to viral, bacterial, fungal, parasitic antigens, or immunogenic portions thereof. Antigens related to cancer include but are not limited to tumor-specific or tumor-associated antigens. Antigens related to autoimmune diseases include but are not limited to any antigen known in the art that stimulates production of autoantibodies, e.g., a self-antigen.

In some embodiments, an analyte produced by a cell disclosed herein is an anti-idiotypic (Anti-ID) antibody targeting idiotype of another antibody, such as an antibody drug. An idiotype can be defined as the specific combination of idiotopes present within an antibodies complement determining regions (CDRs). A single idiotope, is a specific region within an antibodies Fv region which binds to the paratope (antigenic epitope binding site) of a different antibody. In some embodiments, an idiotope is an antigenic determinant of an antibody. In some embodiments, an analyte produced by a cell disclosed herein is an antigen blocking anti-ID antibody, named so because the targeting antibodies paratope and idiotope overlap with one another. Because of this, the antibody drug's target antigen and the anti-ID antibody compete with one another. Therefore, this form of anti-ID antibody only detects free antibody drug. In some embodiments, an analyte produced by a cell disclosed herein is a non-blocking where the antibody drug's paratope and idiotope do not overlap. Therefore, the anti-ID antibody and the antigen can simultaneously bind to the antibody drug without affecting one another's binding capability. In some embodiments, non-blocking anti-ID antibodies bind all forms of available antibody drug (free or antigen bound). In some embodiments, an analyte produced by a cell disclosed herein is a complex specific anti-ID, which cannot bind to the antibody drug unless the drug is already bound to its antigen. Therefore, this type of anti-ID antibody is only able to detect bound antibody drug.

In some embodiments, an analyte (e.g., secreted antibody) is tethered to the surface of a cell (e.g., a B cell or plasma cell, via a capture agent or matrix described herein) and screened for the ability to couple to a plurality of targets. In some embodiments, an analyte is screened for multiple antigen specificities, for example, using a reporter agent coupled to an antibody binding agent, described herein.

Following maturation in the bone marrow and spleen, immunocompetent B cells remain in peripheral tissues until they encounter an antigen and are activated. B cell activation requires two distinct signals, and results in B cell differentiation into memory B cells or plasma cells. The first activation signal occurs upon antigen binding to B cell receptors (BCRs). Upon binding to the BCR, the antigen is internalized by receptor-mediated endocytosis, digested, and complexed with MHC II molecules on the B cell surface. The second activation signal occurs via either a thymus-dependent or a thymus-independent mechanism. Most B cell responses to antigen require the interaction of B cells with T helper cells (thymus-dependent activation). Presentation of an antigen-class II MHC complex on a B cell enables it to act as an antigen-presenting cell (APC) to T cells. T cell receptors (TCR) on T helper cells bind to the antigen-complexed class II MHC molecule on the B cell surface resulting in T cell activation. The activated T cell then provides a second activation signal to the B cell, which can occur through a variety of proteins. Alternatively, there are a few types of antigens that can directly provide the second B cell activation signal (thymus-independent activation). These antigens include components of some bacterial cell wall components (e.g., lipopolysaccharide) or antigens containing highly repetitious molecules (e.g., bacterial flagellin). Upon activation, B cells proliferate and form germinal centers where they differentiate into memory B cells or plasma cells. Following differentiation into plasma cells, additional signals initiate plasma cell antibody class switching and regulate antibody secretion. The primary function of plasma cells is the secretion of B cell clone-specific antibodies. Each plasma cell secretes antibodies containing a clonally-unique antigen-binding region joined to a constant immunoglobulin (Ig) isotype-defining region.

The antigen binding portion of a BCR is composed of a membrane-bound antibody that, like most antibodies (e.g., immunoglobulins), has a unique and randomly determined antigen-binding site. The antigen binding portion of a BCR includes membrane-bound immunoglobulin molecule of one isotype (e.g., IgD, IgM, IgA, IgG, or IgE). When a B cell is activated by its first encounter with a cognate antigen, the cell proliferates and differentiates to generate a population of antibody-secreting plasma B cells and memory B cells. The various immunoglobulin isotypes differ in their biological features, structure, target specificity and distribution. A variety of molecular mechanisms exist to generate initial diversity, including genetic recombination at multiple sites.

The BCR is composed of two genes IgH and IgK (or IgL) coding for antibody heavy and light chains. Immunoglobulins are formed by recombination among gene segments, sequence diversification at the junctions of these segments, and point mutations throughout the gene. Each heavy chain gene contains multiple copies of three different gene segments-a variable 'V' gene segment, a diversity 'D' gene segment, and a joining 'J' gene segment. Each light chain gene contains multiple copies of two different gene segments for the variable region of the protein-a variable 'V' gene segment and a joining 'J' gene segment. The recombination can generate a molecule with one of each of the V, D, and J segments. Furthermore, several bases may be deleted and others added (called N and P nucleotides) at each of the two junctions, thereby generating further diversity. After B cell activation, a process of affinity maturation through somatic hypermutation occurs. In this process progeny cells of the activated B cells accumulate distinct somatic mutations throughout the gene with higher mutation concentration in the CDR regions leading to the generation of antibodies with higher affinity to the antigens. In addition to somatic hypermutation activated B cells undergo the process of isotype switching. Antibodies with the same variable segments can have different forms (isotypes) depending on the constant segment. Whereas all naive B cells express IgM (or IgD), activated B cells mostly express IgG but also IgM, IgA and IgE. This expression switching from IgM (and/or IgD) to IgG, IgA, or IgE occurs through a recombination event causing one cell to specialize in producing a specific isotype. A unique nucleotide sequence that arises during the gene arrangement process can similarly be referred to as a clonotype.

In some embodiments, the methods, compositions and systems disclosed herein are utilized to analyze the various sequences of BCRs and secreted antibodies from immune cells, for example various clonotypes. In some embodiments, methods, compositions and systems disclosed herein are used to analyze the sequence of an antibody or any fragment thereof (e.g., variable regions including VDJ or VJ regions, constant regions, transmembrane regions, fragments thereof, combinations thereof, and combinations of fragments thereof). In some embodiments, methods, compositions and systems disclosed herein are used to analyze the sequence of a B cell receptor heavy chain, B cell receptor light chain, or any fragment thereof (e.g., variable regions including VDJ or VJ regions, constant regions, transmembrane regions, fragments thereof, combinations thereof, and combinations of fragments thereof). Primer sequences useful in any of the various operations for attaching barcode sequences and/or amplification reactions may comprise gene specific sequences which target genes or regions of genes of antibody or BCR proteins.

Generally, the methods and systems described herein may comprise stimulating a cell (e.g., an immune cell, i.e., B cell or plasma cell) to induce secretion of one or more analytes (e.g., secreted antibodies) from the cell. Stimulating a cell (e.g., an immune cell such as a T cell, B cell, plasma cell, or dendritic cell) to induce secretion of one or more analytes (e.g., secreted antibodies) may include subjecting (e.g., contacting) the cell to specific molecules (e.g., stimulatory or co-stimulatory molecules). Specific molecules that may be used to induce secretion of an analyte from the cell may include antigens such as Pattern recognition receptor (PRR) ligands (e.g., Toll-like receptors (TLR) ligands, NOD-like receptor (NLRs) ligands, RIG-I-like receptor (RLR) ligands, C-type lectin receptor (CLR) ligands, cytosolic dsDNA sensor (CDS) ligands, etc.), lipopolysaccharide (LPS), double-stranded DNA (dsDNA), double-stranded RNA (dsRNA), a synthetic dsRNA (e.g., polyinosinic-polycytidylic acid (poly I:C) or polyadenylic-polyuridylic acid (poly(A:U)), CpG oligodeoxynucleotides (CpG ODN), or any combination thereof. The methods described herein may include providing a plurality of one or more different stimulatory molecules to the cell at a concentration that is sufficient to induce secretion of the one or more secreted molecule (e.g., antibodies). Regulators of plasma cell activity are known and include, for example, CD23/Fc epsilon RII, CD25/IL-2R alpha, FCRLB/FCRY, IFN-gamma R1/CD119, IFN-gamma R2, IL-2 R beta, IL-5 R alpha/CD125, IL-27 R alpha/WSX-1/TCCR, IL-4R alpha, IL-6R alpha, NTB-A/SLAMF6, SIT1, and SWAP70, any one or more of which may be targeted, modulated, and/or used to induce antibody secretion from a plasma cell.

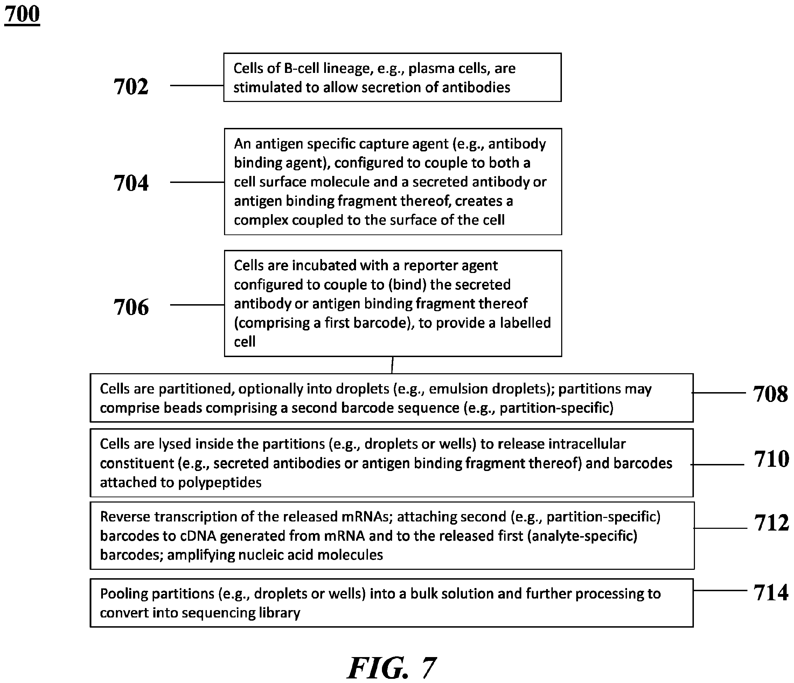

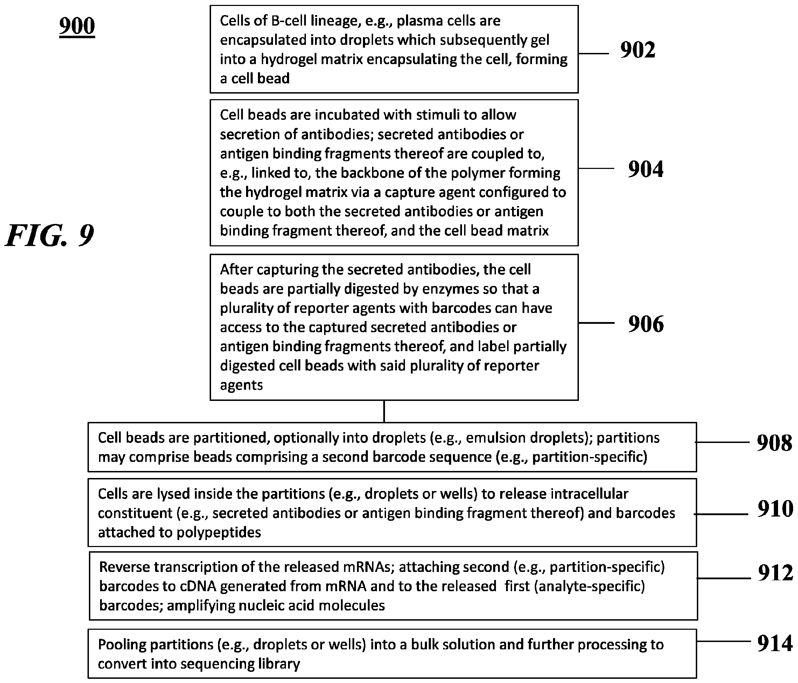

Referring to operation **700** or **900** in **FIG. 7** and **FIG. 9,** respectively, immune cells (e.g., B cells or plasma cells) or other cell types of interest are incubated with a suitable concentration of one or more stimulatory molecules to induce secretion of antibodies or other analytes. Any suitable concentration of stimulatory molecules sufficient to induce antibody secretion from the cells (e.g., B cells or plasma cells) may be used herein.

### III. ANALYTE CAPTURE

A secreted analyte (e.g., an antibody) may quickly float away from the secreting cell and mix with analytes (e.g., antibodies) secreted by other cells, rendering single cell analysis of analyte secretion challenging. In some embodiments disclosed herein, a secreted analyte (e.g., an antibody) is captured, e.g., on a surface of the cell from which the analyte is secreted, in a droplet (e.g., containing a bead such as a magnetic or paramagnetic bead) containing the cell, and/or in a matrix surrounding (e.g., encapsulating) the cell, for subsequent single cell analysis of the cell and the analytes (e.g., antibodies) that it secretes. In some embodiments, a method disclosed herein is used for screening single cells in a heterogeneous population of cells, such as plasma cells in a subj ect.

A. Analyte Capture on Cell Surface

In certain embodiments, a method for processing a secreted analyte (e.g., antibody) from a cell (e.g., B cell or plasma cell) may comprise coupling the secreted analyte to a capture agent. The cell (e.g., B cell or plasma cell) may be incubated with a capture agent, under a sufficient condition that the capture agent couples to the surface of the cell, to form a complex couple to a surface of the cell.

Generally, as disclosed herein, a capture agent may comprise one or more portions. In some cases, a capture agent comprises at least one, at least two, at least three, or at least four or more portions. Each portion may comprise a polypeptide. The polypeptide of a specific portion may be capable of coupling one or more molecules. For example, a polypeptide of a specific portion may coupling to a molecule located on a surface of a cell, such as a cell surface protein or receptor (e.g., a CD surface marker such as CD45). A polypeptide of another portion of a capture agent may bind a molecule that may be secreted from a cell (e.g., T cell, B-cell, dendritic cell, etc.).

The one or more portions of a capture agent may be directly or indirectly linked to, conjugated to, or fused to one another. For example, a first portion of a capture agent may be directly or indirectly linked to, conjugated to, or fused to a second portion of the capture agent. In some cases, the first portion is chemically (e.g., covalently or non-covalently) bound or linked to the second portion. In some cases, the first portion and the second portion are connected via a linker (e.g., an amino acid linker). In some cases, the first portion and the second portion are connected via one or more domains or polypeptide chains. A capture agent as described herein may comprise one or more immunoglobulin (or antibody) molecules (e.g., IgA, IgE, IgG, or IgM molecules) or any fragments (e.g., antigen binding fragments) or derivatives thereof, and in any suitable combination.

For example, a capture agent may be a bispecific antibody, e.g., **804** in **FIG. 8.** A bispecific antibody may comprise a first portion comprising a first polypeptide capable of binding a cell surface molecule (e.g., a CD receptor), and a second portion comprising a second polypeptide capable of binding one or more molecules of a cell (e.g., one or more antibodies secreted from the cell). In some cases, the first polypeptide of a first portion of a capture agent may comprise a first immunoglobulin molecule (e.g., IgA, IgE, IgG, or IgM), or fragment(s) or derivative(s) thereof (e.g., a Fab, $F(ab')_2$, bispecific $Fab_2$, scFv, diabodies, etc.), and the second polypeptide of a second portion of a binding agent may comprise a second immunoglobulin molecule (e.g., IgA, IgE, IgG, or IgM), or fragment(s) or derivative(s) thereof (e.g., a Fab, $F(ab')_2$, bispecific $Fab_2$, scFv, diabodies, etc.)

The first polypeptide (e.g., the first immunoglobulin molecule) of a capture agent may be capable of binding a molecule on the surface of a cell, and the second polypeptide (e.g., the second immunoglobulin molecule) may be capable of binding one or more molecules secreted from the cell. The one or more portions of a capture agent comprising one or more polypeptides, respectively, may be associated, e.g., bound to, linked to, or coupled to one another. For example, a first portion of a capture agent comprising a first polypeptide may be linked to a second portion of a capture agent comprising a second polypeptide via a linker. The linker may be a flexible linker, allowing efficient binding of the first and second polypeptide to their respective binding partners. The linker may be an amino acid linker such as a glycine-rich linker. A linker may be a cleavable or a non-cleavable linker.

In some embodiments, a capture agent **804** shown in **FIG. 8** may comprise a polypeptide, such as a capture antibody or a bispecific molecule, e.g., anti-CD45-streptavidin (for binding to CD45 on the cell surface) conjugated to biotin-labeled $F(ab')_2$ anti-IgG Fc (for binding to a secreted IgG antibody). A capture agent **804** may be a bispecific antibody (or another antibody construct such as a tri-specific antibody) comprising a first portion capable of binding a molecule 808 (e.g., an antibody, cytokine, a hormone, or a growth factor) secreted from the cell **802** (e.g., an immune cell such as a T cell, a B cell, a cell of B cell lineage, or a dendritic cell), and a second portion capable of binding a cell surface molecule such as a cell surface receptor **806** (e.g., CD45). Cells (e.g., B cells or plasma cells) may be incubated with the capture agent in a solution for a certain amount of time under a reasonable and sufficient condition to allow coupling of the capture agent to the cell surface. In certain embodiments, capture agent **804** is a bispecific antibody comprising a first portion and a second portion.

In some embodiments, the first portion comprises an antibody or an antigen binding fragment thereof that has a binding affinity for one or more molecules, such as for one or more different types of antibodies, i.e., the first portion may bind one or more molecules (e.g., one or more antibodies) that come into its vicinity. The first portion may be selected such that it binds to a particular secreted analyte (e.g., antibody) of interest from the cell or cells (e.g., immune cells, i.e., B cells or plasma cells). In other embodiments, the first portion comprises a plurality of antibodies or fragments thereof (e.g., an antigen binding fragment) each may be selective for a certain analyte such as an antibody. For example, the first portion may comprise two antibodies or fragments thereof (e.g., antigen binding fragments) that each have a binding affinity for a different analyte. The first portion may comprise one antibody, two antibodies, three antibodies, four antibodies, five antibodies, six antibodies, seven antibodies, or eight antibodies or fragments thereof, wherein each antibody or fragments thereof has a binding affinity for a different analyte. In other cases, the one or more antibodies and/or antibody fragments (e.g., antigen binding fragments) have a binding affinity for the same analyte (e.g., the same antibody).

Further, in certain embodiments, the second portion comprises an antibody or an antigen binding fragment thereof. The second portion may be configured such that it targets, binds, or otherwise associates with one or more cell surface proteins, such as receptor tyrosine kinases (RTKs), a G-protein-coupled receptors (GPCRs), cluster of differentiation (CD) proteins, (such as CD45, etc.), etc., or any combination thereof. In some embodiments, the second portion is configured to couple (e.g., covalently or non-covalently bind) to the cell surface and allow the first portion to couple (e.g., covalently or non-covalently) to one or more secreted analytes (e.g., antibodies) as described herein. A capture agent (or a polypeptide or portion thereof) may couple to a cell surface in a variety of ways. For example, the second portion of polypeptide may be attached to the cell surface using any suitable approach, such as attachment through cysteine residues or through unnatural amino acids having reactive functionality. In some cases, the second portion is attached to the surface via its carbohydrate groups (i.e., glycosylation sites), for example, on the constant region (i.e., Fc) of the antibody, or via lipid-lipid, or lipid-protein interactions. In some examples, the capture agent could also be lipid-conjugated **806,** enabling insertion of the capture agent into the cell membrane of the cells **802** (e.g., immune cells), as shown in **FIG. 8.** Moreover, in some embodiments, the second portion comprises two antibodies or epitope binding fragments thereof, each having a binding affinity to a different cell surface molecule, respectively. In other embodiments, the second portion comprises three, four, five, six, seven, eight or more antibodies or epitope binding fragments thereof, each selectively targeting a specific cell surface molecule (e.g., the same or different cell surface molecules).

In some embodiments, a first portion of a capture agent (e.g., those comprising polypeptide) may be coupled to, linked to, or conjugated to (e.g., chemically coupled, linked, or conjugated to) a second portion. Alternatively, a first portion of a capture agent may be fused (e.g., recombinantly fused) to a second portion. Thus, in some embodiments, capture agent is a fusion protein comprising the first portion and the second portion. The fusion polypeptide may be generated by recombinant DNA technology, e.g., translation of fusion genes (one or more genes encodes for the first portion and the other one or more genes encodes for the second portion) results in a fusion polypeptide. In certain embodiments, one or more linker (e.g., amino acid linker such as polypeptide linker) link the first portion to the second portion to, e.g., ensure proper folding of the first and second portion and/or to ensure effective cell surface and/or analyte binding. Furthermore, polypeptide linker may allow important domain interactions, reinforce stability, and reduce steric hindrance. In other embodiments, the first portion and the second portion are linked or conjugated post-translationally. For example, a polypeptide of a first portion of a capture agent may be conjugated to a second portion using any suitable bio-conjugation strategy including but not limited to activated esters (e.g., NHS esters), cycloaddition reactions, Staudinger ligation, click chemistry etc. In yet other embodiments, the first portion and the second portion can be connected end-to-end via linkage of N or C termini between the first portion and the second portion, providing a flexible bridge structure for proper folding and reduced steric hindrance.

As shown in **FIG. 8,** binding or coupling of an analyte (e.g., a secreted antibody) to the capture agent **804** and reporter agent **810,** comprising an analyte specific antigen **814** and a nucleic acid molecule comprising a barcode sequence (i.e., reporter barcode sequence) **816,** forms a complex **818.**

## B. Analyte Capture on Matrix

In another aspect, the present disclose provides methods of processing a secreted molecule (e.g., secreted antibody) from a cell (e.g., B cell or plasma cell), the methods comprise (a) generating a cell bead, wherein the cell bead comprises a cell encapsulated by a polymer matrix, wherein the polymer matrix comprises a plurality of capture agents; and (b) coupling a molecule secreted from the cell to a first capture agent of the plurality of capture agents to form a complex.

The present disclosure provides methods that comprise use of macromolecules (e.g., polymers) capable of forming a matrix such as a polymer and/or crosslinked matrix. In some cases, a polymer precursors are polymerized to form a polymer gel matrix. In some embodiments, the polymer gel may be a hydrogel thus forming a hydrogel matrix. The polymer molecules used to form the hydrogel matrix may comprise a plurality of capture agents. The plurality of capture agents may be coupled to or linked to (e.g., covalently or non-covalently bound or linked to the backbone of the polymer. See, e.g., U.S. Pat. Pub. 2019/0100632 (now U.S. Pat. 10,590,244), for exemplary cell bead functionalization strategies. A first capture agent of the plurality of capture agents may comprise a polypeptide capable of binding a specific molecule (e.g., a secreted molecule, such as an antibody). The capture agent comprising the polypeptide capable of binding the specific molecule may be an antibody or an antigen-binding fragment or derivative thereof. The specific molecule that the capture agent may be capable of binding may be an analyte (e.g., antibody) of a cell (e.g., an immune cell, i.e., B cell or plasma cell). The analyte may be a molecule that may be secreted by the cell upon stimulation, e.g., with one or more stimulatory molecules or one or more APCs.

A plurality of cells (e.g., immune cells such as a T cell, B cell, plasma cell, and/or dendritic cells) may be co-partitioned (e.g., encapsulated into droplets) such that a partition (e.g., a droplet or a well) may comprise a cell of the plurality of cells. The partition may further comprise components capable of forming a polymeric or crosslinked matrix such as a hydrogel matrix inside the partition, resulting in the formation of a cell bead. The gel matrix (e.g., hydrogel matrix) and thus the cell bead may be formed using a reversible gel (e.g., hydrogel). The gel matrix may be a hydrogel matrix and the backbones of the polymer molecules forming the hydrogel matrix may comprise a plurality of capture agents comprising a polypeptide capable of binding a specific molecule (e.g., an antibody).

The methods disclosed herein may comprise stimulating the cell (e.g., B cell or plasma cell) forming the cell bead such that the cell secretes one or more analytes. The analytes secreted by the cell may be coupled (e.g., covalently or non-covalently bound or linked) to a first capture agent of a plurality of capture agents attached (e.g., coupled or linked) to the backbones of the polymer molecules forming the cell bead hydrogel matrix, e.g., **FIG. 10A.** In some embodiments, the polymer matrix is a hydrogel matrix. In some embodiments, the polymer matrix comprises collagen, laminin, and/or fibronectin. In some embodiments, the plurality of first capture agents are coupled to a polymer backbone of the polymer matrix. In some embodiments, the bead may comprise covalent or ionic bonds between polymeric precursors (e.g., monomers, oligomers, linear polymers), oligonucleotides, primers, and other entities. In some cases, the covalent bonds comprise carbon-carbon bonds or thioether bonds. In some cases, a bead may comprise an acrydite moiety or click chemistry moiety, which in certain aspects may be used to attach one or more agents such as capture agents. See, e.g., U.S. Pat. Pub. 2019/0100632 (now U.S. Pat. 10,590,244), for exemplary cell bead functionalization strategies.

In some instances, a chemical conjugation method is utilized to attach molecules (e.g., capture agent) to the cell bead hydrogel matrix. In some instances, a capture agent such as an antibody may comprise a chemical modification (e.g., click chemistry precursor such as an alkene/alkyne) that is configured to react with a chemical modification (e.g., click chemistry precursor such as an azide/alkyne) present in the polymer matrix or polymer precursors of a cell bead. In certain embodiments, the capture agents comprising the secreted antibody is coupled to the polymer backbone of the polymer matrix as shown in **FIGS. 10A and 10B.**

Disclosed herein are compositions comprising a cell bead comprising a polymerized or cross-linked polymer network comprising a cell or a lysed cell generated from the cell, wherein the polymerized or cross-linked polymer network is electrically charged. See, e.g., U.S. Pat. Pub. 2018/0216162 (now U.S. Pat. 10,428,326), U.S. Pat. Pub. 2019/0100632 (now U.S. Pat. 10,590,244), and U.S. Pat. Pub. 2019/0233878, for exemplary compositions for cellular analyses. The cell bead may comprise a component (e.g., secreted antibody or antigen binding fragment thereof) from a cell. A component may be a nucleic acid. A nucleic acid may be DNA (e.g., genomic DNA) or RNA (e.g., mRNA, miRNA). A component may be a protein. The polymer network may be positively charged. The polymer network may comprise chitosan. The polymer network may comprise PEI. The polymer network may be negatively charged. The polymer network may comprise alginate. A component from a cell may be capable of being retained within the cell bead for about 1 hour, about 2 hours, about 3 hours, about 4 hours, about 5 hours, about 6 hours, about 12 hours, about 24 hours, about 48 hours, about 72 hours, or more. A component from a cell may be capable of being retained within the cell bead for at least 1 hour, at least 2 hours, at least 3 hours, at least 4 hours, at least 5 hours, at least 6 hours, at least 12 hours, at least 24 hours, at least 48 hours, at least 72 hours, or more. A component from a cell may be capable of being retained within the cell bead for at most 1 hour, at most 2 hours, at most 3 hours, at most 4 hours, at most 5 hours, at most 6 hours, at most 12 hours, at most 24 hours, at most 48 hours, or at most 72 hours.

Also disclosed herein are compositions comprising a cell bead comprising a polymerized or cross-linked polymer network comprising a cell or a lysed cell generated from the cell and a charged species. The cell bead may comprise a component from the cell. A component may be a nucleic acid. A nucleic acid may be DNA (e.g., genomic DNA) or RNA (e.g., mRNA, miRNA). A component may be a protein. The charged species may be positively charged. The charged species may comprise trimethylammonium . The charged species may be (2-Aminoethyl)trimethylammonium . The charged species may be (3-Acrylamidopropyl)trimethylammonium. The charged species may be negatively charged. The charged species may comprise phosphate. A component from a cell may be capable of being retained within the cell bead for about 1 hour, about 2 hours, about 3 hours, about 4 hours, about 5 hours, about 6 hours, about 12 hours, about 24 hours, about 48 hours, about 72 hours, or more. A component from a cell may be capable of being retained within the cell bead for at least 1 hour, at least 2 hours, at least 3 hours, at least 4 hours, at least 5 hours, at least 6 hours, at least 12 hours, at least 24 hours, at least 48 hours, at least 72 hours, or more. A component from a cell may be capable of being retained within the cell bead for at most 1 hour, at most 2 hours, at most 3 hours, at most 4 hours, at most 5 hours, at most 6 hours, at most 12 hours, at most 24 hours, at most 48 hours, or at most 72 hours.

## C. Cell Beads

Cell beads may be generated by methods as described herein, for example by polymerization of molecular precursors (e.g., polymer or gel precursors) in a partition comprising a cell or constituents from a cell. Cell beads can comprise one or more different types of components from a cell, including, for example, DNA (e.g., gDNA, chromatin, etc.), RNA (e.g., mRNA, miRNA), proteins, secreted antibodies or antigen binding fragments thereof, and/or metabolites. Components may be comprised in and/or attached to cell beads (e.g., via a capture agent). Cell beads can be generated by encapsulating a cell in a polymer or gel matrix and lysing the cell in the gel or polymer matrix, lysing the cell while it is being encapsulated in the polymer or gel matrix, or lysing the cell so that its constituents are encapsulated in the polymer or gel matrix. The polymer or gel matrix may comprise one or more charged species configured to interact with a component from a cell (e.g., DNA, RNA, proteins, secreted antibodies or antigen binding fragments thereof, etc.).

The partition used in generating a cell bead may comprise one or more reagents for conducting one or more reactions. Species may include, for example, reagents for a nucleic acid amplification or extension reaction (e.g., primers, polymerases, nucleotides, co-factors (e.g., ionic co-factors), buffers, etc.) including those described herein, reagents for enzymatic reactions (e.g., enzymes, co-factors, substrates, buffers, etc.), reagents for nucleic acid modification reactions, such as polymerization, ligation, or digestion, and/or reagents for template preparation.

Reagents may comprise reagents for minimizing damage of nucleic acids resulting from a click chemistry reaction. For example, a radical scavenger may be added to a partition, thereby reducing the risk of damage to nucleic acids caused by free radicals generated during a click chemistry reaction. In some cases, the radical scavenger comprises dimethyl sulfoxide (DMSO). DMSO may be added to a partition used in generating a cell bead at a sufficient concentration for preventing nucleic acid damage. In some embodiments, DMSO is added to a partition at an amount of at least about 1%, about 2%, about 3%, about 4%, about 5%, about 6%, about 7%, about 8%, about 9%, about 10%, or greater.

One or more reagents within a partition may be attached to precursors (e.g., polymer or gel precursors). Reagents may be covalently attached to precursors. Reagents may be reversibly or irreversible attached to precursors. Reagents may be attached to precursors via an acrydite moiety.

In some cases, oligonucleotides may be attached to the precursors. Oligonucleotides attached to precursors may be useful in, for example, capturing RNA and/or performing a reverse transcription reaction. Oligonucleotides may comprise a poly-T sequence or a poly-U sequence (e.g., may be a poly-T primer). In some embodiments, a poly-T sequence is used to hybridize to a poly-A sequence, for example, from mRNA of a cell. In some embodiments, a poly-U sequence is used to hybridize to a poly-A sequence, for example, from mRNA of a cell.

In some cases, an oligonucleotide, such as a poly-T sequence, can be attached to a precursor (e.g., polymer) via an irreversible click chemistry reaction. In some embodiments, this click chemistry attachment of an oligonucleotide can be carried out during the crosslinking of the polymers that results in a gel matrix. For example, a propargylated poly-T oligonucleotide introduced into an emulsion droplet together with the polymers modified with azide and alkyne click chemistry groups is attached via CuAAC click chemistry resulting in a 1,2,3-triazole linkage with some of the azide-modified linker sites of the azide-modified polymer. The other sites form crosslinks with the alkyne-modified polymers resulting in a gel matrix comprising covalently attached poly-T reagents capable of capturing polyadenylated RNA transcripts.

A partition used in generating a cell bead may comprise one or more particles (e.g., magnetic particles). One or more reagents within a partition may be attached to the particle. Reagents may be covalently attached to the particle. Reagents may be reversibly or irreversibly attached to the particle. Regents may be attached to the particle via an acrydite moiety. In some cases, oligonucleotides may be attached to the particle. Oligonucleotides attached to the particle may be useful in, for example, capturing RNA and performing a reverse transcription reaction. In some embodiments, the particles (which are optionally magnetic particles) comprise oligonucleotides attached thereto that comprise a poly-T sequence capable of hybridizing to a poly-A sequence, for example, from mRNA of a cell.

A cell (e.g., labelled B cell or plasma cell) within a partition may be lysed as described herein, thereby releasing constituents from the cell into the partition. Constituents may include multiple types of cellular components, including proteins, metabolites, and/or nucleic acid molecules (e.g., DNA, RNA (e.g. messenger RNA), etc.). Alternatively, or in addition, a cell within a partition may by permeabilized. Permeabilization may allow for transfer of certain reagents, species, constituents, etc. into and/or out of a cell with or without complete cellular lysis. In some embodiments, the cell is lysed or permeabilized prior to the polymerization or gelling of the cell bead. In other embodiments, the cell is lysed or permeabilized concurrent with the polymerization or gelling of the cell bead. In some embodiments, the cell is lysed or permeabilized subsequent to the polymerization or gelling of the cell bead. In still other embodiments, the cell is not lysed or permeabilized while in the cell bead.

Reagents can be included within a partition, including reagents attached to precursors, particles, etc., and may be used to perform one or more reactions on the cell or constituents from or derived from a cell (e.g., labelled B cell or plasma cell). A reaction may be, for example, amplification, reverse transcription, or deamination reaction. In some embodiments, the one or more reactions are performed prior to the polymerization or gelling of the cell bead. In some embodiments, the one or more reactions are performed concurrent with the polymerization or gelling of the cell bead. In some embodiments, the one or more reactions are performed subsequent to the polymerization or gelling of the cell bead. In some cases, oligonucleotides (e.g., primers) are used to perform a reverse transcription reaction on messenger RNA from a cell, thereby generating complementary DNA (cDNA). Reverse transcription may comprise the addition of additional nucleotides, e.g., a polynucleotide such as polyC, to the cDNA. In some cases, template switching may be performed to further extend the cDNA. Template switching may append one or more additional sequences to the cDNA. Additional sequences may, in some cases, be used to facilitate nucleic acid extension/amplification and/or barcoding, as described herein. cDNA may be attached to precursors and/or particles. In some cases, oligonucleotides are used to capture messenger RNA from a cell, (e.g., via hybridization) prior to generation of a cell bead.

In some embodiments, a partition is subjected to conditions sufficient to generate a cell bead comprising one or more reagents. For example, a partition droplet comprising polymer precursors attached to reagents (e.g., primers, nucleic acid molecules, etc.) may be polymerized or gelled such that the reagents are attached to the polymer or gel matrix (i.e., attached to a cell bead). In some instances, the reagents are releasably attached to the gel precursor via a labile bond (e.g., a chemically labile bond, thermally labile bond, or photo-labile bond). Reagents may be covalently attached to a cell bead. Reagents may be reversible or irreversibly attached to a cell bead. Reagents may be attached to the surface of a gel bead. Reagents may be attached to the inside of a cell bead. In some cases, mRNA is attached to a cell bead. For example, polymer precursors attached to mRNA from a cell may be polymerized or gelled to generate a cell bead such that the mRNA is attached to the cell bead. In some cases, cDNA is attached to a cell bead. For example, polymer precursors attached to cDNA derived from a cell may be polymerized to generate a cell bead such that the cDNA is attached to the cell bead. In some cases, one or more oligonucleotides are attached to a cell bead. For example, polymer precursors attached to the oligonucleotides may be polymerized or gelled to generate a cell bead such that the oligonucleotides are attached to the cell bead.

Attaching macromolecular constituents (e.g., nucleic acid molecules, protein, secreted antibodies or antigen binding fragments thereof, etc.) to a cell bead or a particle within a cell bead may be useful in preparing the species for further processing. For example, nucleic acid molecules attached to a cell bead or particle may be processed while remaining attached to the cell bead or particle. Following processing, a nucleic acid may be released (e.g., released into a partition) from a cell bead and/or particle for analysis. In some cases, it may be useful to attach one type of cellular component or derivative thereof (e.g., mRNA, cDNA, secreted antibodies or antigen binding fragments thereof) to a cell bead or a particle within a cell bead, while encapsulating but not attaching another type of cellular component (e.g., genomic DNA). This may be useful in, for example, facilitating separate processing of multiple types of components. For example, following cell bead formation, cell beads may be transferred to an aqueous solution and subjected to additional processing as described herein. For example, cell beads may be subjected, in bulk, to reverse transcription to generate cDNA from captured mRNA (e.g., hybridized to an oligonucleotide attached to the cell bead matrix or a particle, such as a magnetic particle).

### D. Methods for Cell Bead Generation

Methods of the present disclosure may comprise generation of one or more cell beads comprising one or more of the polymers disclosed herein. See, e.g., U.S. Pat. Pub. 2018/0216162 (now U.S. Pat. 10,428,326), U.S. Pat. Pub. 2019/0100632 (now U.S. Pat. 10,590,244), and U.S. Pat. Pub. 2019/0233878, for exemplary cell bead generation systems and methods. For example, cells and polymer or gel precursors are mixed with an immiscible fluid (e.g., an oil), thereby generating a plurality of aqueous droplets, including a droplet comprising a cell. A droplet may comprise a charged species, as described herein. A droplet may be subjected to conditions sufficient for polymerization or gelation of the polymer or gel precursors to generate a cell bead comprising a cell. Gelation may comprise any of the gelation mechanisms and polymers described herein, including those utilizing a click chemistry reaction, as described elsewhere herein. In some instances, a cell bead is subjected to treatment conditions sufficient to lyse the cell (e.g., labelled B cell or plasma cell) , releasing components of the cell into the cell bead. In other embodiments, the cell is lysed in a droplet prior to polymerization or gelation of the polymer or gel precursors to generate a cell bead. In still other embodiments, a cell is permeabilized before or after polymerization or gelation of the polymer or gel precursors. Cell beads may be collected in an aqueous phase to generate a plurality of cell beads. Cell beads may be stored for further processing. In some cases, charged species may be attached to the cell beads subsequent to polymerization or gelation of the polymer or gel precursor. For instance, polymer or gel precursors may comprise one or more functional groups that facilitate the attachment of the charged species subsequent to polymerization or gelation of the polymer or gel precursors. In other embodiments, the polymer or gel precursors comprise functional groups comprising the charged species, which are incorporated into the cell bead during polymerization or gelation of the polymer or gel precursors.

In an aspect, the present disclosure provides methods for generating a cell bead comprising a charged species. First, a partition may be generated comprising a cell from a plurality of cells (e.g., a labelled B cell or plasma cell), a polymeric or gel precursor, and a charged species. Next, the partition may be subjected to conditions sufficient to react the polymeric or gel precursor to generate a polymer or gel network comprising the cell or a derivative thereof and the charged species, thereby generating a cell bead. The partition may be subjected to conditions sufficient to polymerize or gel the polymeric or gel precursors. Conditions sufficient to polymerize or gel polymeric or gel precursors are described elsewhere herein. In some embodiments, the cell is lysed to release components of the cell into the cell bead. The cell may be lysed prior to polymerization or gelling of the polymeric or gel precursors, concurrently with polymerization or gelling of the polymeric or gel precursors, or subsequent to polymerization or gelling of the polymeric or gel precursors. In other embodiments, the cell in the cell bead is not lysed, but is permeabilized to allow access to components within the nucleus.

In another aspect, the present disclosure provides methods for generating a cell bead comprising a charged species. First, a partition may be generated comprising a nucleus isolated from a cell, a polymeric or gel precursor, and a charged species. Next, the partition may be subjected to conditions sufficient to react the polymeric or gel precursors to generate a polymer or gel network comprising the nucleus and the charged species, thereby generating a cell bead. The partition may be subjected to conditions sufficient to polymerize or gel the polymeric or gel precursors. Conditions sufficient to polymerize or gel polymeric or gel precursors are described elsewhere herein. For example, a copper catalyst may be used to catalyze a click chemistry reaction, thereby generating a hydrogel. In some embodiments, the nucleus is lysed to release components of the nucleus into the cell bead. The nucleus may be lysed prior to polymerizing or gelling the polymeric or gel precursors, concurrently with polymerizing or gelling the polymeric or gel precursors, or subsequent to polymerizing or gelling the polymeric or gel precursors. In other embodiments, the nucleus in the cell bead is not lysed, but is permeabilized to allow access to nuclear components within the nucleus.

A charged species may be a positively charged species. A positively charged species may be a reagent comprising a positive charge. A positively charged species may comprise trimethylammonium. A positively charged species may be (3-Acrylamidopropyl)-trimethylammonium. A charged species may be a negatively charged species. A negatively charged species may comprise phosphate. A charged species may be attached to the polymer or gel network. A charged species may be incorporated into a polymer or gel network during polymerization. A cell bead may comprise one or more chemical cross-linkers. A chemical cross-linker may be a disulfide bond. A charged species may be attached to one or more chemical cross-linkers. A derivative of a cell may be a component from a cell (e.g., DNA, RNA, protein, etc.). A method of generating a cell bead may comprise lysing a cell within a partition (e.g., a droplet) to release a component from the cell. A component may be a nucleic acid. A nucleic acid may be DNA (e.g., genomic DNA) or RNA (e.g., mRNA, siRNA). A component may be a protein. A component may be a negatively charged component, for example, DNA, RNA, or miRNA. A component may be a positively charged component, for example, a protein. A component from a cell may interact with a charged species. A component from a cell may be non-covalently attached to a charged species.

In some embodiments, a negatively charged component from or derived from a cell (e.g., DNA) interacts with a positively charged species (e.g., ((3-Acrylamidopropyl)-trimethylammonium) of the cell bead (e.g., a positively charged functional group of the cell bead polymers) via ionic interactions. In other embodiments, a positively charged component from or derived from a cell (e.g., a protein) interacts with a negatively charged species (e.g., phosphate) of the cell bead (e.g., a negatively charged functional group of the cell bead polymers) via ionic interactions. In still other embodiments, a negatively charged component from or derived from a cell (e.g., DNA) interacts with a positively charged species (e.g., ((3-Acrylamidopropyl)-trimethylammonium) of the cell bead (e.g., a positively charged functional group of the cell bead polymers) and a positively charged component from or derived from a cell (e.g., a protein) interacts with a negatively charged species (e.g., phosphate) of the cell bead (e.g., a negatively charged functional group of the cell bead polymers). Thus, one or more components from a cell may be capable of being retained within the cell bead, for example, due to electrostatic interactions with a charged species of a cell bead. A component from a cell may be capable of being retained within the cell bead for about 1 hour, about 2 hours, about 3 hours, about 4 hours, about 5 hours, about 6 hours, about 12 hours, about 24 hours, about 48 hours, about 72 hours, or more. A component from a cell may be capable of being retained within the cell bead for at least 1 hour, at least 2 hours, at least 3 hours, at least 4 hours, at least 5 hours, at least 6 hours, at least 12 hours, at least 24 hours, at least 48 hours, at least 72 hours, or more. A component from a cell may be capable of being retained within the cell bead for at most 1 hour, at most 2 hours, at most 3 hours, at most 4 hours, at most 5 hours, at most 6 hours, at most 12 hours, at most 24 hours, at most 48 hours, or at most 72 hours.

In an aspect, the present disclosure provides methods for generating a cell bead comprising an electrically charged polymer or gel network (e.g., a cell bead comprising a charged species). First, a partition may be generated comprising a cell from a plurality of cells (e.g., labelled B cells or plasma cells) and a polymeric or gel precursor. Next, the partition may be subjected to conditions sufficient to react said electrically charged polymeric or gel precursor to generate an electrically charged polymer or gel network comprising the cell or a derivative thereof, thereby providing the cell bead comprising the charged species. The reaction may be such that the net charge on the polymer or gel precursor is changed, thereby generating an electrically charged polymer or gel network. The reaction may be such that the net charge on the polymer or gel network is changed, thereby generating an electrically charged polymer or gel network.

The polymer or gel precursor may be positively charged. The polymer or gel precursor may comprise chitosan. The polymer or gel precursor may comprise polyethyleneimine (PEI). The polymer or gel precursor may be negatively charged. The polymer or gel precursor may comprise alginate. A derivative of a cell may be a component from a cell (e.g., DNA, RNA, protein, secreted antibody or antigen binding fragment thereof, etc.). A method of generating a cell bead may comprise lysing a cell within a partition (e.g., a droplet) to release a component from the cell. A component may be a nucleic acid. A nucleic acid may be DNA (e.g., genomic DNA) or RNA (e.g., mRNA, siRNA). A component may be a protein. A component may be a negatively charged component, for example, DNA, RNA, or miRNA. A component may be a positively charged component, for example, a protein. A component from a cell may interact with the electrically charged polymer or gel network. A component from a cell may be non-covalently attached to the polymer or gel network of a cell bead comprising a charged species. In some embodiments, a negatively charged component from or derived from a cell (e.g., DNA) interacts with a positively charged species of the cell bead (e.g., a positive charged polymer or gel network) via ionic interactions. In other embodiments, a positively charged component from or derived from a cell (e.g., a protein) interacts with a negatively charged species of the cell bead (e.g., a negatively charged polymer or gel network) via ionic interactions.

In still other embodiments, a negatively charged component from or derived from a cell (e.g., DNA) interacts with a positively charged species of the cell bead (e.g., a positive charged polymer or gel network) and a positively charged component from or derived from a cell (e.g., a protein) interacts with a negatively charged species of the cell bead (e.g., a negatively charged polymer or gel network). Thus, one or more components from a cell may be capable of being retained within the cell bead, for example, due to electrostatic interactions with a charged species of a cell bead. A component from a cell may be capable of being retained within the cell bead, for example, due to interactions with an electrically charged polymer or gel network. A component from a cell may be capable of being retained within the cell bead for about 1 hour, about 2 hours, about 3 hours, about 4 hours, about 5 hours, about 6 hours, about 12 hours, about 24 hours, about 48 hours, about 72 hours, or more. A component from a cell may be capable of being retained within the cell bead for at least 1 hour, at least 2 hours, at least 3 hours, at least 4 hours, at least 5 hours, at least 6 hours, at least 12 hours, at least 24 hours, at least 48 hours, at least 72 hours, or more. A component from a cell may be capable of being retained within the cell bead for at most 1 hour, at most 2 hours, at most 3 hours, at most 4 hours, at most 5 hours, at most 6 hours, at most 12 hours, at most 24 hours, at most 48 hours, or at most 72 hours.

In an aspect, the present disclosure provides methods for generating a cell bead comprising a charged species. First, a partition may be generated comprising a cell (e.g., labelled B cell or plasma cell) from a plurality of cells and a polymeric or gel precursor. Next, the partition may be subjected to conditions sufficient to react the polymeric or gel precursor to generate a polymer or gel network comprising the cell or a derivative thereof. Next, a charged species may be coupled to the polymer or gel network, thereby providing the cell bead comprising the charged species. The partition may be subjected to conditions sufficient to polymerize or gel the polymeric or gel precursors. Conditions sufficient to polymerize or gel polymeric or gel precursors are described elsewhere herein. For example, a copper catalyst may be used to catalyze a click chemistry reaction, thereby generating a hydrogel. In some cases, the cell is lysed to release cellular components. The cell may be lysed prior to polymerizing or gelling the polymeric or gel precursors, concurrently with polymerizing or gelling the polymeric or gel precursors, or subsequent to polymerizing or gelling the polymeric or gel precursors.

A polymer or gel network can be a degradable polymer or gel network, as described herein, such that a cell bead is a degradable cell bead. Any number of cell beads may be generated by generating a plurality of partitions. In some cases, about 1, about 2, about 3, about 4, about 5, about 10, about 50, about 100, about 500, about 1000, about 5000, about 10000, about 20000, about 50000, about 100000, or more cell beads are generated, thereby generating a plurality of cell beads. A cell bead may be partitioned together with a barcode bead (e.g., a gel bead) for analysis of a cell or components thereof.

**E. Click Chemistry**

As used herein, the term "click chemistry," generally refers to reactions that are modular, wide in scope, give high yields, generate only inoffensive byproducts, such as those that can be removed by nonchromatographic methods, and are stereospecific (but not necessarily enantioselective). See, e.g., U.S. Pat. Pub. 2019/0100632 (now U.S. Pat. 10,590,244), U.S. Pat. Pub. 2019/0233878, and Angew. Chem. Int. Ed., 2001, 40(11):2004-2021.

In some cases, click chemistry can describe pairs of functional groups that can selectively react with each other in mild, aqueous conditions. An example of click chemistry reaction can be the Huisgen 1,3-dipolar cycloaddition of an azide and an alkynes, i.e., Copper-catalyzed reaction of an azide with an alkyne to form a 5-membered heteroatom ring called 1,2,3-triazole. The reaction can also be known as a Cu(I)-Catalyzed Azide-Alkyne Cycloaddition (CuAAC), a Cu(I) click chemistry or a $Cu^+$ click chemistry. Catalyst for the click chemistry can be Cu(I) salts, or Cu(I) salts made in situ by reducing Cu(II) reagent to Cu(I) reagent with a reducing reagent (Pharm Res. 2008, 25(10): 2216-2230). Known Cu(II) reagents for the click chemistry can include, but are not limited to, Cu(II)-(TBTA) complex and Cu(II) (THPTA) complex. TBTA, which is tris-[(1-benzyl-1H-1,2,3-triazol-4-yl)methyl]amine, also known as tris-(benzyltriazolylmethyl)amine, can be a stabilizing ligand for Cu(I) salts. THPTA, which is tris-(hydroxypropyltriazolylmethyl)amine, can be another example of stabilizing agent for Cu(I). Other conditions can also be accomplished to construct the 1,2,3-triazole ring from an azide and an alkyne using copper-free click chemistry, such as by the Strain-promoted Azide-Alkyne Click chemistry reaction (SPAAC, see, e.g., Chem. Commun., 2011, 47:6257-6259 and Nature, 2015, 519(7544)486-90).

In some cases, the present disclosure also contemplates the use of click chemistry reactions resulting in chemical linkages that are not a 1,2,3-triazole. See, e.g., U.S. Pat. Pub. 2019/023/878. A range of such click chemistry reactions useful for preparing biocompatible gels are well-known in the art. See e.g., Madl and Heilshom, "Bioorthogonal Strategies for Engineering Extracellular Matrices," Adv. Funct. Mater. 2018, 28:1706046.

An example of a click chemistry reaction useful in the compositions and methods of the present disclosure that is copper-free and does not result in a 1,2,3-triazole linkage is an Inverse-electron demand Diels-Alder (IED-DA) reaction. (See e.g., Madl and Heilshom 2018.) As described elsewhere herein, in the IED-DA click chemistry reaction, the pair of click chemistry functional groups comprises a tetrazine group and a trans-cyclooctene (TCO) group, or a tetrazine group and a norbonene group. This reaction is copper free and results in a linkage comprising a dihydropyridazine group rather than a 1,2,3-triazole.

Other specific biorthogonal click chemistry reactions that are useful in the compositions and methods of the present disclosure, but which result in a chemical linkage other than a 1,2,3-triazole include a Diels-Alder reaction between a pair of furan and maleimide functional groups, a Staudinger ligation, and nitrile oxide cycloaddition. These click chemistry reactions and others are well-known in the art and described in e.g., Madl and Heilshom 2018. Accordingly, in some embodiments the copper-free click chemistry useful in forming crosslinked polymers of the present disclosure can be selected from: (a) strain-promoted azide/dibenzocyclooctyne-amine (DBCO) click chemistry; (b) inverse electron demand Diels-Alder (IED-DA) tetrazine/trans-cyclooctene (TCO) click chemistry; (c) inverse electron demand Diels-Alder (IED-DA) tetrazine/norbonene click chemistry; (d) Diels-Alder maleimide/furan click-chemistry; (e) Staudinger ligation; and (f) nitrile-oxide/norbonene cycloaddition click chemistry.

**IV. REPORTER AGENTS**

An analyte (e.g., a secreted antibody disclosed herein) may be labeled by a reporter agent as described herein and at FIG. 8 (e.g., an antigen labeled by a barcode). The reporter agent may comprise a barcode, e.g., reporter barcode sequence (FIG. 8 at **816**) and/or a UMI. The barcode sequence can include from 6 to about 20 or more nucleotides within the sequence of the oligonucleotides. In some cases, the length of a barcode sequence may be 6, 7, 8, 9, 10, 11, 12, 13, 14, 15, 16, 17, 18, 19, 20 nucleotides or longer. In some cases, the length of a barcode sequence may be at least 6, 7, 8, 9, 10, 11, 12, 13, 14, 15, 16, 17, 18, 19, 20 nucleotides or longer. In some cases, the length of a barcode sequence may be at most 6, 7, 8, 9, 10, 11, 12, 13, 14, 15, 16, 17, 18, 19, 20 nucleotides or shorter. These nucleotides may be completely contiguous, i.e., in a single stretch of adjacent nucleotides, or they may be separated into two or more separate subsequences that are separated by 1 or more nucleotides. In some cases, separated barcode subsequences can be from about 4 to about 16 nucleotides in length. In some cases, the barcode subsequence may be 4, 5, 6, 7, 8, 9, 10, 11, 12, 13, 14, 15, 16 nucleotides or longer. In some cases, the barcode subsequence may be at least 4, 5, 6, 7, 8, 9, 10, 11, 12, 13, 14, 15, 16 nucleotides or longer. In some cases, the barcode subsequence may be at most 4, 5, 6, 7, 8, 9, 10, 11, 12, 13, 14, 15, 16 nucleotides or shorter. The reporter agent can also comprise other functional sequences useful in the processing of the nucleic acids from the co-partitioned cells. These sequences include, e.g., targeted or random/universal amplification primer sequences for amplifying the genomic DNA from the individual cells within the partitions while attaching the associated barcode sequences, sequencing primers or primer recognition sites, hybridization or probing sequences, e.g., for identification of presence of the sequences or for pulling down barcoded nucleic acids, or any of a number of other potential functional sequences.

The methods and systems described herein may comprise use of one or more barcoded components or molecules (e.g., capture agents or portions thereof) to analyze a biological sample (e.g., a population of immune cells, e.g., a population of B cells or plasma cells) on a single cell basis. In some embodiments, the methods described herein comprise using a reporter agent that is capable of coupling to an analyte secreted from a cell (e.g., an antibody). A reporter agent, (see e.g., **810**) may be added to the cell or cell bead, wherein the reporter agent may comprise (i) a second polypeptide (e.g., an antibody binding molecule, e.g., an antigen **814**) capable of binding a secreted analyte molecule (e.g., a secreted antibody) that is coupled to the capture agent; and (ii) a nucleic acid molecule comprising a first, analyte specific barcode sequence (e.g., **816),** thereby tagging the analyte molecules (e.g., an antibody) coupled to the capture agent. The reporter agent may couple to the analyte while the analyte is coupled to the capture agent that is coupled to the cell surface. The reporter agent may be an antibody binding agent (e.g., and antigen, antibody, or fragment, e.g., an antigen binding fragment) or derivative thereof. The reporter agent may include an antigen capable of coupling to the analyte coupled to the capture agent. In some cases, any reporter agent molecules that are not coupled to an analyte (e.g., antigens) may be removed from the cell, or cell bead containing a cell (e.g., by performing a washing step to remove unbound reporter agent molecules). The reporter agent may comprise a barcode (i.e., reporter barcode sequence), e.g., an oligonucleotide sequence comprising a barcode sequence, and may barcode the analyte. Thus, an analyte (e.g., a secreted molecule, e.g., a secreted antibody) may be barcoded while coupled to the reporter agent that in turn may be coupled to the cell that secreted said analyte (e.g., coupled through a capture agent). Barcodes as described herein may be used to associate one or more analytes (e.g., secreted molecules or cellular nucleotide sequences such as mRNAs) with a cell and/or a partition (e.g., droplet or well) upon analyzing the barcodes using e.g., sequencing reads generated using an Illumina sequencer.

Referring to operations **800** in **FIG. 8,** after secreted antibodies or other analytes bind to the first portion of a capture agent to form a first complex, the cells (e.g., B cells or plasma cells) or other types of cells are further incubated with a solution comprising a plurality of reporter agents **810.** Each reporter agent **810** is coupled to a nucleic acid molecule **816** comprising a barcode sequence (i.e., reporter barcode sequence). In certain embodiments, the reporter agent **810** comprises a protein, such as an antigen **814.** In certain embodiments, antigen **814** selectively binds to at least one antibody, e.g., a secreted antibody. In other embodiments, antigen **814** selectively binds to other secreted analytes of interest. Further, in some embodiments, the reporter agent **810** comprises a fluorophore, chromophore, heavy metal, or any combinations thereof (e.g., as an enrichment target **812)** in addition to the barcode **816.** Examples of fluorophore that can be utilized here are fluorescein isothiocyanate (FITC), phycoerythrin (PE), allophycocyanin (APC), ALexa Four, DyLight, and etc. The reporter agent **810** along with the reporter barcode **816** binds to secreted antibodies that are captured by the capture agent **804** to form a second complex. In an exemplary embodiment, the barcodes, including barcodes **816,** are DNA oligonucleotides. Accordingly, in some instances, attaching a cell-specific barcode (e.g., in a partition, such as a droplet-see **FIG. 3)** to molecules containing the antigen-barcode (e.g., reporter barcode **816)** and, where present, the barcode allows the association of both the secreted antibody molecule and the antigen with the same single cell. Optionally, a selection/enrichment agent may be used to select and/or enrich a cell bead comprising a cell that has secreted an antibody that is bound to a capture agent on the cell and to the reporter agent **810.** The selection/enrichment agent comprises an antibody conjugated to a magnetic bead, and the antibody may comprise a tag such as a fluorescent tag.

Referring to operations **1000** in **FIG. 10A,** after secreted antibodies or other analytes bind to the first portion of a capture agent of the matrix of the cell bead **1002,** the cells (e.g., B cells or plasma cells) or other types of cells are further incubated with a solution comprising a plurality of reporter agents **810.** Each reporter agent **810** is coupled to a nucleic acid molecule **816** comprising a barcode sequence (i.e., reporter barcode sequence). In certain embodiments, the reporter agent **810** comprises a protein, such as an antigen **814.** In certain embodiments, antigen **814** selectively binds to at least one antibody, e.g., a secreted antibody. In other embodiments, antigen **814** selectively binds to other secreted analytes of interest. Further, in some embodiments, the reporter agent **810** comprises a fluorophore, chromophore, heavy metal, or any combinations thereof (e.g., **812)** in addition to the barcode **816.** Examples of fluorophore that can be utilized here are fluorescein isothiocyanate (FITC), phycoerythrin (PE), allophycocyanin (APC), ALexa Four, DyLight, and etc. The reporter agent **810** along with the, e.g., reporter, barcode **816** binds to secreted antibodies that are captured by the capture agent **804** to form a second complex. In an exemplary embodiment, the barcodes, including barcodes **816,** are DNA oligonucleotides. Accordingly, in some instances, attaching a cell-specific barcode (e.g., in a partition, such as a droplet-see **FIG. 3)** to molecules containing the antigen-barcode (e.g., reporter barcode **816)** and, where present, the barcode allows the association of both the secreted antibody molecule and the antigen with the same single cell. Optionally, a selection/enrichment agent **1004** may be used to select and/or enrich a cell bead comprising a cell that has secreted an antibody **1012** that is bound, e.g., coupled, to a capture agent (e.g., **1014** in **FIG. 10B;** not shown in **FIG. 10A)** on cell bead **1002** and to reporter agent **810.** The selection/enrichment agent comprises an antibody **1006** conjugated to a magnetic bead **1010,** and antibody **1006** may comprises a tag **1008** such as a fluorescent tag. In some embodiments, the capture agent comprises an anti-Fc antibody or antigen binding fragment thereof. In some embodiments, the capture agent comprises an anti-ID antibody or antigen binding fragment thereof. In some embodiments, the capture agent comprises an antigen or epitope recognized by the secreted antibody analyte.

**FIG. 10B** shows binding or coupling of an analyte (e.g., a secreted antibody **1012)** to the capture agent **1014** coupled to cell bead **1002,** forming a complex **1016.** A reporter agent comprising an analyte specific antigen and a nucleic acid molecule comprising a barcode sequence (i.e., reporter barcode sequence) is contacted with the complex, to form a labelled cell bead.

In some embodiments, secretion of an analyte or molecule of interest (e.g., an antibody) from a cell and coupling (e.g., binding such as covalent or non-covalent binding) of the analyte to one or more binding agents (e.g., a capture agent and a reporter agent), is followed by co-partitioning the cell into a partition. In other cases, co-partitioning occurs prior to secretion of an analyte from the cell and coupling of the analyte to the one or more binding agents. In some embodiments, at least some of the partitions may comprise at most one cell. In some cases, a partition comprises at least one cell. A partition as described herein may include a droplet or a well (e.g., in a microwell array). A droplet may be an emulsion droplet. A droplet (e.g., an emulsion droplet) may be formed by bringing a first phase in contact with a second phase that is immiscible with the first phase. In other cases, the partition may be a well as part of a plurality of wells. In yet other cases, the partition may be a chamber as part of a plurality of chambers. Partitions may be fluidically isolated from one another or physically isolated from one another. In some instances, a partition (e.g., a droplet or a well) may comprise a cell, a capture agent and a reporter agent, wherein the capture agent and the reporter agent may be coupled to an analyte, and coupled directly and/or indirectly to the cell (see e.g., **FIG. 8** and **FIG. 10A).** In some cases, the reporter agent comprises (e.g., is coupled or linked to) a nucleic acid molecule comprising an analyte-specific barcode (i.e., reporter barcode sequence), thereby labelling a cell or a cell bead, for subsequent partitioning.

## V. LABELLED CELLS OR CELL BEADS

The secreted analyte of interest (e.g., secreted antibody), or antigen fragment thereof, can be isolated using a complex that coats the cell that can "catch" (e.g., capture ) said analyte. The analyte-coated cells can then be detected (e.g., using reporter agents, comprising antibody binding agents, e.g., antigens) and/or separated by mechanisms that allow the differentiation of the analyte-coated cells from non-coated cells. Exemplary methods include, but are not limited to, fluorescent-activated cell sorting (FACS), magnetic-activated cell sorting (MACS) or buoyancy-activated cell sorting (BACS).

In some embodiments, the antibody-coated cells can be detected and/or selected by using fluorescent-activated cell sorting (FACS). In some embodiments, antibody-coated cells can be detected by a fluorochrome-labeled antibody recognizing the cell-bound antibodies. In some embodiments, antibody-coated cells can be detected by a fluorochrome-labeled antibody recognizing the Fc region of the cell-bound antibodies, and subsequently analyzed using FACS. In some embodiments, antibody-coated cells can be detected by an antibody (e.g., a fluorochrome-labeled antibody, a magnetic bead-labeled antibody, a microbubble-labeled antibody, or a combination thereof) recognizing the reporter agent (e.g., recognizing tag **812** of reporter agent **1010)** coupled to the cell-bound antibodies, and subsequently analyzed.

In some embodiments, the antibody-coated cells can be detected and/or selected by using magnetic-activated cell sorting (MACS). In some embodiments, antibody-coated cells can be detected by an antibody bound to a magnetic bead recognizing the cell-bound antibodies. In some embodiments, antibody-coated cells can be detected by a magnetic bead-labeled antibody recognizing the Fc region of the cell-bound antibodies, and subsequently analyzed using MACS. In some embodiments, antibody-coated cells can be detected by an antibody (e.g., a fluorochrome-labeled antibody, a magnetic bead-labeled antibody, a microbubble-labeled antibody, or a combination thereof) recognizing the reporter agent (e.g., recognizing tag **812** of reporter agent **1010)** coupled to the cell-bound antibodies, and subsequently analyzed.

In some embodiments, the antibody-coated cells can be detected and/or selected by using buoyancy-activated cell sorting (BACS). In some embodiments, antibody-coated cells can be detected by a microbubble-labeled antibody recognizing the cell-bound antibodies. In some embodiments, antibody-coated cells can be bound by a microbubble-labeled antibody, resulting in lower density in a buoyancy separation. In some embodiments, antibody-coated cells can be detected by a microbubble-labeled antibody recognizing the Fc region of the cell-bound antibodies, and subsequently analyzed using BACS. In some embodiments, antibody-coated cells can be detected by an antibody (e.g., a fluorochrome-labeled antibody, a magnetic bead-labeled antibody, a microbubble-labeled antibody, or a combination thereof) recognizing the reporter agent (e.g., recognizing tag **812** of reporter agent **810)** coupled to the cell-bound antibodies, and subsequently analyzed.

In some embodiments, provided herein is a labelled cell comprising a complex coupled to a surface of the cell. In some embodiments, the complex comprises (i) a capture agent, (ii) a secreted antigen binding molecule (e.g., a secreted antigen receptor, such as a secreted antibody or antigen binding fragment thereof), and (iii) a reporter agent comprising a reporter nucleic acid molecule, wherein the reporter nucleic acid molecule comprises a reporter sequence, e.g., reporter barcode sequence, corresponding to the reporter agent. See depiction in, description of, FIG. 8, e.g., paragraph 166.

In some embodiments, provided herein are a plurality of labelled cells, wherein each cell comprises a complex coupled to a surface of the cell. In some embodiments, each complex comprises (i) a capture agent, (ii) a secreted antigen binding molecule (e.g., a secreted antigen receptor, such as a secreted antibody or antigen binding fragment thereof), and (iii) a reporter agent comprising a reporter nucleic acid molecule, wherein the reporter nucleic acid molecule comprises a reporter sequence, e.g., reporter barcode sequence, corresponding to the reporter agent. The capture agents, the secreted antigen binding molecules, the reporter agents, the reporter nucleic acid molecules, and/or the reporter sequences, e.g., reporter barcode sequences, can be the same or different among the plurality of labelled cells.

In some embodiments, provided herein is a labelled cell bead comprising a cell in a matrix and a complex in or on the cell bead. In some embodiments, the complex comprises (i) a capture agent, (ii) a secreted antigen binding molecule (e.g., a secreted antigen receptor, such as a secreted antibody or antigen binding fragment thereof), and (iii) a reporter agent comprising a reporter nucleic acid molecule, wherein the reporter nucleic acid molecule comprises a reporter sequence, e.g., reporter barcode sequence, corresponding to the reporter agent. See depiction in, descriptions of, FIG. 10A, e.g., paragraph 167.

In some embodiments, provided herein are a plurality of labelled cell beads, wherein each cell bead comprises a cell in a matrix and a complex in or on the cell bead. In some embodiments, each complex comprises (i) a capture agent, (ii) a secreted antigen binding molecule (e.g., a secreted antigen receptor, such as a secreted antibody or antigen binding fragment thereof), and (iii) a reporter agent comprising a reporter nucleic acid molecule, wherein the reporter nucleic acid molecule comprises a reporter sequence, e.g., reporter barcode sequence, corresponding to the reporter agent. The matrices, capture agents, the secreted antigen binding molecules, the reporter agents, the reporter nucleic acid molecules, and/or the reporter sequences, e.g., reporter barcode sequences, can be the same or different among the plurality of labelled cell beads.

In some embodiments, the labelled cell, the labelled cell bead, the plurality of labelled cells, or the plurality of labelled cell beads are partitioned. In some embodiments, a partition comprises a plurality of barcode nucleic acid molecules which comprise a plurality of partition barcode sequences. As described for **FIG. 4,** e.g., paragraph 320, the partition barcode sequences may be a partition-specific sequence common to the partition **(410).**

In some embodiments, in a partition, a barcoded nucleic acid molecule is generated from a barcode nucleic acid molecule of the plurality of barcode nucleic acid molecules and the reporter nucleic acid molecule, wherein the barcoded nucleic acid molecule comprises the reporter sequence, e.g., reporter barcode sequence, or a complement thereof and a partition barcode sequence or a complement thereof.

## VI. CELL PROCESSING AND PARTITIONING

In one aspect, the methods and system described herein provide for the compartmentalization, depositing or partitioning of individual cells (e.g., a cell of B cell lineage, e.g. a plasma cell) from a sample material containing cells, into discrete compartments or partitions (referred to interchangeably herein as partitions), where each partition maintains separation of its own contents from the contents of other partitions. In one aspect, the methods described herein comprise the compartmentalization, depositing or partitioning of nucleic acid molecules (e.g., genomic DNA and/or nucleic acid barcodes directly or indirectly attached to the cell surface) of one or more individual cells from a sample material containing cells, into discrete partitions, where each partition maintains separation of its own contents from the contents of other partitions.

In another aspect, the methods and system described herein provide for the compartmentalization, depositing or partitioning of individual cells from a sample material containing cells after at least one labelling agent or reporter agent has been bound to a cell surface feature of a cell, into discrete partitions, where each partition maintains separation of its own contents from the contents of other partitions. Identifiers including unique identifiers and common or universal tags, e.g., barcodes, may be previously, subsequently or concurrently delivered to the partitions that hold the compartmentalized or partitioned cells, in order to allow for the later attribution of the characteristics of the individual cells to one or more particular compartments. Further, identifiers including unique identifiers and common or universal tags, e.g., barcodes, may be coupled to labelling agents and previously, subsequently or concurrently delivered to the partitions that hold the compartmentalized or partitioned cells, in order to allow for the later attribution of the characteristics of the individual cells to one or more particular compartments. Identifiers including unique identifiers and common or universal tags, e.g., barcodes, may be delivered, for example on an oligonucleotide, to a partition via any suitable mechanism.

In some embodiments, a partition herein includes a space or volume that may be suitable to contain one or more species or conduct one or more reactions. A partition may be a physical compartment, such as a droplet or well. The partition may isolate space or volume from another space or volume. The droplet may be a first phase (e.g., aqueous phase) in a second phase (e.g., oil) immiscible with the first phase. The droplet may be a first phase in a second phase that does not phase separate from the first phase, such as, for example, a capsule or liposome in an aqueous phase. A partition may comprise one or more other (inner) partitions. In some cases, a partition may be a virtual compartment that can be defined and identified by an index (e.g., indexed libraries) across multiple and/or remote physical compartments. For example, a physical compartment may comprise a plurality of virtual compartments.

## A. Systems and Methods for Compartmentalization

In an aspect, the systems and methods described herein provide for the compartmentalization, depositing, or partitioning of one or more particles (e.g., biological particles, macromolecular constituents of biological particles, beads, reagents, etc.) into discrete compartments or partitions (referred to interchangeably herein as partitions), where each partition maintains separation of its own contents from the contents of other partitions. In some examples, the partitioned particle is a labelled cell of B-cell lineage, e.g. a plasma cell, which secretes an antibody. In other examples, the partitioned particle may be a labelled cell engineered to secrete antibodies. The partition can be a droplet in an emulsion or well. A partition may comprise one or more other partitions.

A partition may include one or more particles. A partition may include one or more types of particles. For example, a partition of the present disclosure may comprise one or more biological particles, e.g., labelled B cells or plasma cells, and/or macromolecular constituents thereof. A partition may comprise one or more beads. A partition may comprise one or more gel beads. A partition may comprise one or more cell beads. A partition may include a single gel bead, a single cell bead, or both a single cell bead and single gel bead. A partition may include one or more reagents. Alternatively, a partition may be unoccupied. For example, a partition may not comprise a bead. A cell bead can be a biological particle, e.g., labelled B cell or plasma cell, and/or one or more of its macromolecular constituents, e.g., a secreted antibody or antigen binding fragment thereof, encased inside of a gel or polymer matrix (e.g., via a capture agent coupled to both the matrix and said secreted antibody or antigen binding fragment thereof), such as via polymerization of a droplet containing the biological particle and precursors capable of being polymerized or gelled. Unique identifiers, such as barcodes, may be injected into the droplets previous to, subsequent to, or concurrently with droplet generation, such as via a microcapsule (e.g., bead), as described elsewhere herein. Microfluidic channel networks (e.g., on a chip) can be utilized to generate partitions as described herein. Alternative mechanisms may also be employed in the partitioning of individual biological particles, including porous membranes through which aqueous mixtures of cells are extruded into non-aqueous fluids.

The methods and systems of the present disclosure may comprise methods and systems for generating one or more partitions such as droplets. The droplets may comprise a plurality of droplets in an emulsion. In some examples, the droplets may comprise droplets in a colloid. In some cases, the emulsion may comprise a microemulsion or a nanoemulsion. In some examples, the droplets may be generated with aid of a microfluidic device and/or by subjecting a mixture of immiscible phases to agitation (e.g., in a container). In some cases, a combination of the mentioned methods may be used for droplet and/or emulsion formation.

Droplets can be formed by creating an emulsion by mixing and/or agitating immiscible phases. Mixing or agitation may comprise various agitation techniques, such as vortexing, pipetting, tube flicking, or other agitation techniques. In some cases, mixing or agitation may be performed without using a microfluidic device. In some examples, the droplets may be formed by exposing a mixture to ultrasound or sonication. Systems and methods for droplet and/or emulsion generation by agitation are described in International Application Nos. WO2015157567, WO2020167862 and WO2020176882.

Microfluidic devices or platforms comprising microfluidic channel networks (e.g., on a chip) can be utilized to generate partitions such as droplets and/or emulsions as described herein. Methods and systems for generating partitions such as droplets, methods of encapsulating biological particles and/or biological particles in partitions (or analyte carriers and/or analyte carriers in partitions), methods of increasing the throughput of droplet generation, and various geometries, architectures, and configurations of microfluidic devices and channels are described in U.S. Patent Publication Nos. 2019/0367997 and 2019/0064173.

In some examples, individual particles can be partitioned to discrete partitions by introducing a flowing stream of particles in an aqueous fluid into a flowing stream or reservoir of a non-aqueous fluid, such that droplets may be generated at the junction of the two streams/reservoir, such as at the junction of a microfluidic device provided elsewhere herein.

The methods of the present disclosure may comprise generating partitions and/or encapsulating particles, such as biological particles, in some cases, individual biological particles such as single cells. In some examples, reagents may be encapsulated and/or partitioned (e.g., co-partitioned with biological particles) in the partitions. Various mechanisms may be employed in the partitioning of individual particles. An example may comprise porous membranes through which aqueous mixtures of cells may be extruded into fluids (e.g., non-aqueous fluids).

The partitions can be flowable within fluid streams. The partitions may comprise, for example, micro-vesicles that have an outer barrier surrounding an inner fluid center or core. In some cases, the partitions may comprise a porous matrix that is capable of entraining and/or retaining materials (e.g., secreted analytes, i.e., secreted antibodies or antigen binding fragments thereof) within its matrix (e.g., via a capture agent configured to couple to both the matrix and said secreted antibody or antigen binding fragment thereof). The partitions can be droplets of a first phase within a second phase, wherein the first and second phases are immiscible. For example, the partitions can be droplets of aqueous fluid within a non-aqueous continuous phase (e.g., oil phase). In another example, the partitions can be droplets of a non-aqueous fluid within an aqueous phase. In some examples, the partitions may be provided in a water-in-oil emulsion or oil-in-water emulsion. A variety of different vessels are described in, for example, U.S. Patent Application Publication No. 2014/0155295. Emulsion systems for creating stable droplets in non-aqueous or oil continuous phases are described in, for example, U.S. Patent Application Publication No. 2010/0105112.

In the case of droplets in an emulsion, allocating individual particles (e.g., labelled B cells or plasma cells) to discrete partitions may, in one non-limiting example, be accomplished by introducing a flowing stream of particles in an aqueous fluid into a flowing stream of a non-aqueous fluid, such that droplets are generated at the junction of the two streams. Fluid properties (e.g., fluid flow rates, fluid viscosities, etc.), particle properties (e.g., volume fraction, particle size, particle concentration, etc.), microfluidic architectures (e.g., channel geometry, etc.), and other parameters may be adjusted to control the occupancy of the resulting partitions (e.g., number of biological particles per partition, number of beads per partition, etc.). For example, partition occupancy can be controlled by providing the aqueous stream at a certain concentration and/or flow rate of particles. To generate single biological particle partitions, the relative flow rates of the immiscible fluids can be selected such that, on average, the partitions may contain less than one biological particle per partition in order to ensure that those partitions that are occupied are primarily singly occupied. In some cases, partitions among a plurality of partitions may contain at most one biological particle (e.g., bead, DNA, cell, such as a labelled B cell or plasma cell, or cellular material). In some embodiments, the various parameters (e.g., fluid properties, particle properties, microfluidic architectures, etc.) may be selected or adjusted such that a majority of partitions are occupied, for example,

ple, allowing for only a small percentage of unoccupied partitions. The flows and channel architectures can be controlled as to ensure a given number of singly occupied partitions, less than a certain level of unoccupied partitions and/or less than a certain level of multiply occupied partitions.

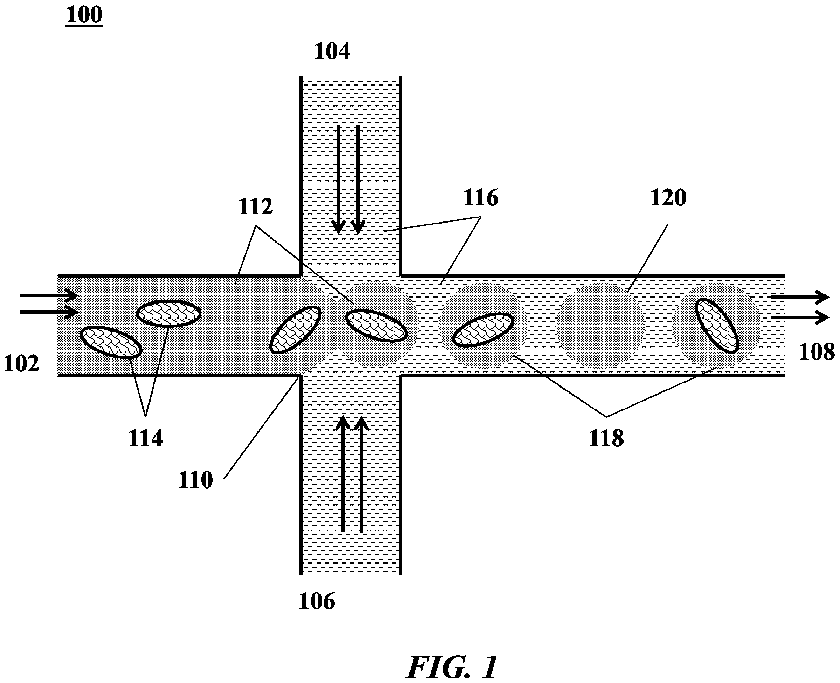

FIG. 1 shows an example of a microfluidic channel structure **100** for partitioning individual biological particles. The channel structure **100** can include channel segments **102, 104, 106** and **108** communicating at a channel junction **110**. In operation, a first aqueous fluid **112** that includes suspended biological particles (e.g., cells, i.e., labelled B cells or plasma cells) **114** may be transported along channel segment **102** into junction **110,** while a second fluid **116** that is immiscible with the aqueous fluid **112** is delivered to the junction **110** from each of channel segments **104** and **106** to create discrete droplets **118, 120** of the first aqueous fluid **112** flowing into channel segment **108,** and flowing away from junction **110.** The channel segment **108** may be fluidically coupled to an outlet reservoir where the discrete droplets can be stored and/or harvested. A discrete droplet generated may include an individual biological particle **114** (such as droplets **118**). A discrete droplet generated may include more than one individual biological particle (e.g., labelled B cell or plasma cell) **114** (not shown in **FIG. 1**). A discrete droplet may contain no biological particle **114** (such as droplet **120**). Each discrete partition may maintain separation of its own contents (e.g., individual biological particle **114**) from the contents of other partitions.

The second fluid **116** can comprise an oil, such as a fluorinated oil, that includes a fluorosurfactant for stabilizing the resulting droplets, for example, inhibiting subsequent coalescence of the resulting droplets **118, 120.** Examples of particularly useful partitioning fluids and fluorosurfactants are described, for example, in U.S. Patent Application Publication No. 2010/0105112.

As will be appreciated, the channel segments of the microfluidic devices described herein may be coupled to any of a variety of different fluid sources or receiving components, including reservoirs, tubing, manifolds, or fluidic components of other systems. As will be appreciated, the microfluidic channel structure **100** may have other geometries and/or configurations. For example, a microfluidic channel structure can have more than one channel junction. For example, a microfluidic channel structure can have 2, 3, 4, or 5 channel segments each carrying particles (e.g., biological particles, cell beads, and/or gel beads) that meet at a channel junction. Fluid may be directed to flow along one or more channels or reservoirs via one or more fluid flow units. A fluid flow unit can comprise compressors (e.g., providing positive pressure), pumps (e.g., providing negative pressure), actuators, and the like to control flow of the fluid. Fluid may also or otherwise be controlled via applied pressure differentials, centrifugal force, electrokinetic pumping, vacuum, capillary or gravity flow, or the like.

The generated droplets may comprise two subsets of droplets: (1) occupied droplets **118,** containing one or more biological particles **114,** e.g., labelled B cells or plasma cells, and (2) unoccupied droplets **120,** not containing any biological particles **114.** Occupied droplets **118** may comprise singly occupied droplets (having one biological particle, such as one B cell or plasma cell) and multiply occupied droplets (having more than one biological particle, such as multiple B cells or plasma cells). As described elsewhere herein, in some cases, the majority of occupied partitions can include no more than one biological particle, e.g., labelled B cell or plasma cell, per occupied partition and some of the generated partitions can be unoccupied (of any biological particle, or labelled B cell or plasma cell). In some cases, though, some of the occupied partitions may include more than one biological particle, e.g., labelled B cell or plasma cell. In some cases, the partitioning process may be controlled such that fewer than about 25% of the occupied partitions contain more than one biological particle, and in many cases, fewer than about 20% of the occupied partitions have more than one biological particle, while in some cases, fewer than about 10% or even fewer than about 5% of the occupied partitions include more than one biological particle per partition.

In some cases, it may be desirable to minimize the creation of excessive numbers of empty partitions, such as to reduce costs and/or increase efficiency. While this minimization may be achieved by providing a sufficient number of biological particles (e.g., biological particles, such as labelled B cells or plasma cells **114)** at the partitioning junction **110,** such as to ensure that at least one biological particle is encapsulated in a partition, the Poissonian distribution may expectedly increase the number of partitions that include multiple biological particles. As such, where singly occupied partitions are to be obtained, at most about 95%, 90%, 85%, 80%, 75%, 70%, 65%, 60%, 55%, 50%, 45%, 40%, 35%, 30%, 25%, 20%, 15%, 10%, 5% or less of the generated partitions can be unoccupied.

In some cases, the flow of one or more of the biological particles, such as B cells or plasma cells, (e.g., in channel segment **102),** or other fluids directed into the partitioning junction (e.g., in channel segments **104, 106)** can be controlled such that, in many cases, no more than about 50% of the generated partitions, no more than about 25% of the generated partitions, or no more than about 10% of the generated partitions are unoccupied. These flows can be controlled so as to present a non-Poissonian distribution of single-occupied partitions while providing lower levels of unoccupied partitions. The above noted ranges of unoccupied partitions can be achieved while still providing any of the single occupancy rates described above. For example, in many cases, the use of the systems and methods described herein can create resulting partitions that have multiple occupancy rates of less than about 25%, less than about 20%, less than about 15%, less than about 10%, and in many cases, less than about 5%, while having unoccupied partitions of less than about 50%, less than about 40%, less than about 30%, less than about 20%, less than about 10%, less than about 5%, or less.

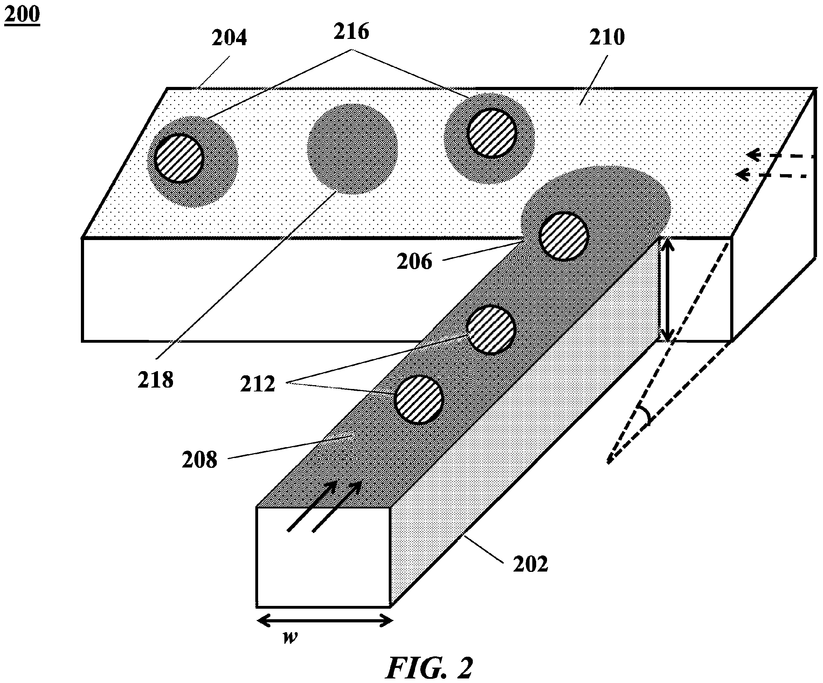

As will be appreciated, the above-described occupancy rates are also applicable to partitions that include both biological particles (e.g., labelled B cells or plasma cells) and additional reagents, including, but not limited to, microcapsules or beads (e.g., gel beads) carrying barcode nucleic acid molecules (e.g., oligonucleotides) (described in relation to **FIGS. 1 and 2).** The occupied partitions (e.g., at least about 10%, 20%, 30%, 40%, 50%, 60%, 70%, 80%, 90%, 95%, or 99% of the occupied partitions) can include both a microcapsule (e.g., bead) comprising barcode nucleic acid molecules and a biological particle.

In another aspect, in addition to or as an alternative to droplet based partitioning, biological particles may be encapsulated within a microcapsule that comprises an outer shell, layer or porous matrix in which is entrained one or more individual biological particles or small groups of biological particles. The microcapsule may include other reagents. Encapsulation of biological particles, e.g., labelled B cells or plasma cells, may be performed by a variety of processes. Such processes may combine an aqueous fluid containing the biological particles with a polymeric precursor material that may be capable of being formed into a gel or other solid or semi-solid matrix upon application of a particular stimulus to the polymer precursor. Such stimuli can include, for example, thermal stimuli (e.g., either heating or cooling), photo-stimuli (e.g., through photo-curing), chemical stimuli (e.g., through crosslinking, polymerization initiation of the precursor (e.g., through added initiators)), mechanical stimuli, or a combination thereof.

Preparation of microcapsules comprising biological particles, e.g., labelled B cells or plasma cells, may be performed by a variety of methods. For example, air knife droplet or aerosol generators may be used to dispense droplets of precursor fluids into gelling solutions in order to form microcapsules that include individual biological particles or small groups of biological particles (e.g., labelled B cells or plasma cells). Likewise, membrane based encapsulation systems may be used to generate microcapsules comprising encapsulated biological particles (e.g., B cells or plasma cells) as described herein. Microfluidic systems of the present disclosure, such as that shown in **FIG. 1,** may be readily used in encapsulating cells as described herein. In particular, and with reference to **FIG. 1,** the aqueous fluid **112** comprising (i) the biological particles (e.g., labelled B cells or plasma cells) **114** and (ii) the polymer precursor material (not shown) is flowed into channel junction **110,** where it is partitioned into droplets **118, 120** through the flow of non-aqueous fluid **116.** In the case of encapsulation methods, non-aqueous fluid **116** may also include an initiator (not shown) to cause polymerization and/or crosslinking of the polymer precursor to form the microcapsule that includes the entrained biological particles. Examples of polymer precursor/initiator pairs include those described in U.S. Patent Application Publication No. 2014/0378345.

For example, in the case where the polymer precursor material comprises a linear polymer material, such as a linear polyacrylamide, PEG, or other linear polymeric material, the activation agent may comprise a cross-linking agent, or a chemical that activates a cross-linking agent within the formed droplets. Likewise, for polymer precursors that comprise polymerizable monomers, the activation agent may comprise a polymerization initiator. For example, in certain cases, where the polymer precursor comprises a mixture of acrylamide monomer with a N,N'-bis-(acryloyl)cystamine (BAC) comonomer, an agent such as tetraethylmethylenediamine (TEMED) may be provided within the second fluid streams 116 in channel segments 104 and 106, which can initiate the copolymerization of the acrylamide and BAC into a cross-linked polymer network, or hydrogel.

Upon contact of the second fluid stream 116 with the first fluid stream 112 at junction 110, during formation of droplets, the TEMED may diffuse from the second fluid 116 into the aqueous fluid 112 comprising the linear polyacrylamide, which will activate the crosslinking of the polyacrylamide within the droplets 118, 120, resulting in the formation of gel (e.g., hydrogel) microcapsules, as solid or semi-solid beads or particles entraining the cells (e.g., labelled B cells or plasma cells) 114. Although described in terms of polyacrylamide encapsulation, other 'activatable' encapsulation compositions may also be employed in the context of the methods and compositions described herein. For example, formation of alginate droplets followed by exposure to divalent metal ions (e.g., $Ca^{2+}$ ions), can be used as an encapsulation process using the described processes. Likewise, agarose droplets may also be transformed into capsules through temperature based gelling (e.g., upon cooling, etc.).

In some cases, encapsulated biological particles can be selectively releasable from the microcapsule, such as through passage of time or upon application of a particular stimulus, that degrades the microcapsule sufficiently to allow the biological particles (e.g., labelled B cells or plasma cells), or its other contents to be released from the microcapsule, such as into a partition (e.g., droplet). For example, in the case of the polyacrylamide polymer described above, degradation of the microcapsule may be accomplished through the introduction of an appropriate reducing agent, such as DTT or the like, to cleave disulfide bonds that cross-link the polymer matrix. See, for example, U.S. Patent Application Publication No. 2014/0378345.

The biological particle (e.g., labelled B cell or plasma cell), can be subjected to other conditions sufficient to polymerize or gel the precursors. The conditions sufficient to polymerize or gel the precursors may comprise exposure to heating, cooling, electromagnetic radiation, and/or light. The conditions sufficient to polymerize or gel the precursors may comprise any conditions sufficient to polymerize or gel the precursors. Following polymerization or gelling, a polymer or gel may be formed around the biological particle (e.g., labelled B cell or plasma cell). The polymer or gel may be diffusively permeable to chemical or biochemical reagents. The polymer or gel may be diffusively impermeable to macromolecular constituents (e.g., secreted antibodies or antigen binding fragments thereof) of the biological particle (e.g., labelled B cell or plasma cell). In this manner, the polymer or gel may act to allow the biological particle (e.g., labelled B cell or plasma cell) to be subjected to chemical or biochemical operations while spatially confining the macromolecular constituents to a region of the droplet defined by the polymer or gel. The polymer or gel may include one or more of disulfide cross-linked polyacrylamide, agarose, alginate, polyvinyl alcohol, polyethylene glycol (PEG)-diacrylate, PEG-acrylate, PEG-thiol, PEG-azide, PEG-alkyne, other acrylates, chitosan, hyaluronic acid, collagen, fibrin, gelatin, or elastin. The polymer or gel may comprise any other polymer or gel.

The polymer or gel may be functionalized (e.g., coupled to a capture agent) to bind to targeted analytes (e.g., secreted antibodies or antigen binding fragment thereof), such as nucleic acids, proteins, carbohydrates, lipids or other analytes. The polymer or gel may be polymerized or gelled via a passive mechanism. The polymer or gel may be stable in alkaline conditions or at elevated temperature. The polymer or gel may have mechanical properties similar to the mechanical properties of the bead. For instance, the polymer or gel may be of a similar size to the bead. The polymer or gel may have a mechanical strength (e.g. tensile strength) similar to that of the bead. The polymer or gel may be of a lower density than an oil. The polymer or gel may be of a density that is roughly similar to that of a buffer. The polymer or gel may have a tunable pore size. The pore size may be chosen to, for instance, retain denatured nucleic acids. The pore size may be chosen to maintain diffusive permeability to exogenous chemicals such as sodium hydroxide (NaOH) and/or endogenous chemicals such as inhibitors. The polymer or gel may be biocompatible. The polymer or gel may maintain or enhance cell viability. The polymer or gel may be biochemically compatible. The polymer or gel may be polymerized and/or depolymerized thermally, chemically, enzymatically, and/or optically.

The polymer may comprise poly(acrylamide-co-acrylic acid) crosslinked with disulfide linkages. The preparation of the polymer may comprise a two-step reaction. In the first activation step, poly(acrylamide-co-acrylic acid) may be exposed to an acylating agent to convert carboxylic acids to esters. For instance, the poly(acrylamide-co-acrylic acid) may be exposed to 4-(4,6-dimethoxy-1,3,5-triazin-2-yl)-4-methylmorpholinium chloride (DMTMM). The polyacrylamide-co-acrylic acid may be exposed to other salts of 4-(4,6-dimethoxy-1,3,5-triazin-2-yl)-4-methylmorpholinium. In the second cross-linking step, the ester formed in the first step may be exposed to a disulfide crosslinking agent. For instance, the ester may be exposed to cystamine (2,2'-dithiobis(ethylamine)). Following the two steps, the biological particle may be surrounded by polyacrylamide strands linked together by disulfide bridges. In this manner, the biological particle may be encased inside of or comprise a gel or matrix (e.g., polymer matrix) to form a "cell bead." A cell bead can contain biological particles (e.g., labelled B cell or plasma cell) or macromolecular constituents (e.g., RNA, DNA, proteins, secreted antibodies or antigen binding fragments thereof etc.) of biological particles. A cell bead may include a single cell or multiple cells, or a derivative of the single cell or multiple cells. For example after lysing and washing the cells, inhibitory components from cell lysates can be washed away and the macromolecular constituents can be bound as cell beads. Systems and methods disclosed herein can be applicable to both cell beads (and/or droplets or other partitions) containing biological particles and cell beads (and/or droplets or other partitions) containing macromolecular constituents of biological particles.

Encapsulated biological particles (e.g., labelled B cells or plasma cells) can provide certain potential advantages of being more storable and more portable than droplet-based partitioned biological particles. Furthermore, in some cases, it may be desirable to allow biological particles (e.g., labelled B cell or plasma cell) to incubate for a select period of time before analysis, such as in order to characterize changes in such biological particles over time, either in the presence or absence of different stimuli (e.g., cytokines, antigens, etc.). In such cases, encapsulation may allow for longer incubation than partitioning in emulsion droplets, although in some cases, droplet partitioned biological particles may also be incubated for different periods of time, e.g., at least 10 seconds, at least 30 seconds, at least 1 minute, at least 5 minutes, at least 10 minutes, at least 30 minutes, at least 1 hour, at least 2 hours, at least 5 hours, or at least 10 hours or more. The encapsulation of biological particles (e.g., labelled B cells or plasma cells) may constitute the partitioning of the biological particles into which other reagents are co-partitioned. Alternatively or in addition, encapsulated biological particles may be readily deposited into other partitions (e.g., droplets) as described above.

## B. Samples and Cell Processing

A sample, e.g., samples comprising analytes such as nucleic acids, proteins, and/or cells, may be processed prior to, during, and/or after partitioning.

A sample may be derived from any useful source including any subject, such as a human subject. A sample may comprise material (e.g., one or more cells) from one or more different sources, such as one or more different subjects. Multiple samples, such as multiple samples from a single subject (e.g., multiple samples obtained in the same or different manners from the same or different bodily locations, and/or obtained at the same or different times (e.g., seconds, minutes, hours, days, weeks, months, or years apart)), or multiple samples from different subjects, may be obtained for analysis as described herein. For example, a first sample may be obtained from a subject at a first time and a second sample may be obtained from the subject at a second time later than the first time. The first time may be before a subject undergoes a treatment regimen or procedure (e.g., to address a disease or condition), and the second time may be during or after the subject undergoes the treatment regimen or procedure. In another example, a first sample may be obtained from a first bodily location or system of a subject (e.g., using a first collection technique) and a second sample may be obtained from a second bodily location or system of the subject (e.g., using a second collection technique), which second bodily location or system may be different than the first bodily location or system. In another example, multiple samples may be obtained from a subject at a same time from the same or different bodily locations. Different samples, such as different samples collected from different bodily locations of a same subject, at different times, from multiple different subjects, and/or using different collection techniques, may undergo the same or different processing (e.g., as described herein). For example, a first sample may undergo a first processing protocol and a second sample may undergo a second processing protocol.

A sample may be a biological sample, such as a cell sample (e.g., as described herein). A sample may include one or more biological particles (or analyte carriers), such as one or more cell beads, cells and/or cellular constituents, such as one or more cell nuclei. For example, a sample may comprise a plurality of cells and/or cellular constituents. Components (e.g., cells or cellular constituents, such as cell nuclei) of a sample may be of a single type or a plurality of different types. For example, cells of a sample may include one or more different types of blood cells.

A biological sample may include a plurality of cells having different dimensions and features. In some cases, processing of the biological sample, such as cell separation and sorting (e.g., as described herein), may affect the distribution of dimensions and cellular features included in the sample by depleting cells having certain features and dimensions and/or isolating cells having certain features and dimensions.

A sample may undergo one or more processes in preparation for analysis (e.g., as described herein), including, but not limited to, filtration, selective precipitation, purification, centrifugation, permeabilization, isolation, agitation, heating, and/or other processes. For example, a sample may be filtered to remove a contaminant or other materials. In an example, a filtration process may comprise the use of microfluidics (e.g., to separate biological particles or analyte carriers of different sizes, types, charges, or other features).

In an example, a sample comprising one or more cells may be processed to separate the one or more cells from other materials in the sample (e.g., using centrifugation and/or another process). In some cases, cells and/or cellular constituents of a sample may be processed to separate and/or sort groups of cells and/or cellular constituents, such as to separate and/or sort cells and/or cellular constituents of different types. Examples of cell separation include, but are not limited to, separation of white blood cells or immune cells from other blood cells and components, separation of circulating tumor cells from blood, and separation of bacteria from bodily cells and/or environmental materials. A separation process may comprise a positive selection process (e.g., targeting of a cell type of interest for retention for subsequent downstream analysis, such as by use of a monoclonal antibody that targets a surface marker of the cell type of interest), a negative selection process (e.g., removal of one or more cell types and retention of one or more other cell types of interest), and/or a depletion process (e.g., removal of a single cell type from a sample, such as removal of red blood cells from peripheral blood mononuclear cells).

Separation of one or more different types of cells may comprise, for example, centrifugation, filtration, microfluidic-based sorting, flow cytometry, fluorescence-activated cell sorting (FACS), magnetic-activated cell sorting (MACS), buoyancy-activated cell sorting (BACS), or any other useful method. For example, a flow cytometry method may be used to detect cells and/or cellular constituents based on a parameter such as a size, morphology, or protein expression. Flow cytometry-based cell sorting may comprise injecting a sample into a sheath fluid that conveys the cells and/or cellular constituents of the sample into a measurement region one at a time. In the measurement region, a light source such as a laser may interrogate the cells and/or cellular constituents and scattered light and/or fluorescence may be detected and converted into digital signals. A nozzle system (e.g., a vibrating nozzle system) may be used to generate droplets (e.g., aqueous droplets) comprising individual cells and/or cellular constituents. Droplets including cells and/or cellular constituents of interest (e.g., as determined via optical detection) may be labeled with an electric charge (e.g., using an electrical charging ring), which charge may be used to separate such droplets from droplets including other cells and/or cellular constituents. For example, FACS may comprise labeling cells and/or cellular constituents with fluorescent markers (e.g., using internal and/or external biomarkers). Cells and/or cellular constituents may then be measured and identified one by one and sorted based on the emitted fluorescence of the marker or absence thereof. MACS may use micro- or nano-scale magnetic particles to bind to cells and/or cellular constituents (e.g., via an antibody interaction with cell surface markers) to facilitate magnetic isolation of cells and/or cellular constituents of interest from other components of a sample (e.g., using a column-based analysis). BACS may use microbubbles (e.g., glass microbubbles) labeled with antibodies to target cells of interest. Cells and/or cellular components coupled to microbubbles may float to a surface of a solution, thereby separating target cells and/or cellular components from other components of a sample. Cell separation techniques may be used to enrich for populations of cells of interest (e.g., prior to partitioning, as described herein). For example, a sample comprising a plurality of cells including a plurality of cells of a given type may be subjected to a positive separation process. The plurality of cells of the given type may be labeled with a fluorescent marker (e.g., based on an expressed cell surface marker or another marker) and subjected to a FACS process to separate these cells from other cells of the plurality of cells. The selected cells may then be subjected to subsequent partition-based analysis (e.g., as described herein) or other downstream analysis. The fluorescent marker may be removed prior to such analysis or may be retained. The fluorescent marker may comprise an identifying feature, such as a nucleic acid barcode sequence and/or unique molecular identifier.

In another example, a first sample comprising a first plurality of cells including a first plurality of cells of a given type (e.g., immune cells expressing a particular marker or combination of markers) and a second sample comprising a second plurality of cells including a second plurality of cells of the given type may be subjected to a positive separation process. The first and second samples may be collected from the same or different subjects, at the same or different types, from the same or different bodily locations or systems, using the same or different collection techniques. For example, the first sample may be from a first subject and the second sample may be from a second subject different than the first subject. The first plurality of cells of the first sample may be provided a first plurality of fluorescent markers configured to label the first plurality of cells of the given type. The second plurality of cells of the second sample may be provided a second plurality of fluorescent markers configured to label the second plurality of cells of the given type. The first plurality of fluorescent markers may include a first identifying feature, such as a first barcode, while the second plurality of fluorescent markers may include a second identifying feature, such as a second barcode, that is different than the first identifying feature. The first plurality of fluorescent markers and the second plurality of fluorescent markers may fluoresce at the same intensities and over the same range of wavelengths upon excitation with a same excitation source (e.g., light source, such as a laser). The first and second samples may then be combined and subjected to a FACS process to separate cells of the given type from other cells based on the first plurality of fluorescent markers labeling the first plurality of cells of the given type and the second plurality of fluorescent markers labeling the second plurality of cells of the given type. Alternatively, the first and second samples may undergo separate FACS processes and the positively selected cells of the given type from the first sample and the positively selected cells of the given type from the second sample may then be combined for subsequent analysis. The encoded identifying features of the different fluorescent markers may be used to identify cells originating from the first sample and cells originating from the second sample. For example, the first and second identifying features may be configured to interact (e.g., in partitions, as described herein) with nucleic acid barcode molecules (e.g., as described herein) to generate barcoded nucleic acid products detectable using, e.g., nucleic acid sequencing.

## C. Beads

A partition may comprise one or more unique identifiers, such as barcodes (e.g., a plurality of barcode nucleic acid molecules which comprise a plurality of partition barcode sequences, which may include a common, e.g., partition-specific, sequence as described for **FIG. 4,** e.g., paragraph 321 in reference to **(410)** and **FIG. 3** at, e.g., paragraph 318 in reference to element **310).** Barcodes may be previously, subsequently or concurrently delivered to the partitions that hold the compartmentalized or partitioned biological particle (e.g., labelled B cell or plasma cell). For example, barcodes may be injected into droplets previous to, subsequent to, or concurrently with droplet generation. The delivery of the barcodes to a particular partition allows for the later attribution of the characteristics of the individual biological particle (e.g., labelled B cell or plasma cell) to the particular partition. Barcodes may be delivered, for example on a nucleic acid molecule (e.g., an oligonucleotide), to a partition via any suitable mechanism. Barcode nucleic acid molecules can be delivered to a partition via a microcapsule. A microcapsule, in some instances, can comprise a bead. Beads are described in further detail below.

In some cases, barcode nucleic acid molecules can be initially associated with the microcapsule and then released from the microcapsule. Release of the barcode nucleic acid molecules can be passive (e.g., by diffusion out of the microcapsule). In addition or alternatively, release from the microcapsule can be upon application of a stimulus which allows the barcode nucleic acid nucleic acid molecules to dissociate or to be released from the microcapsule. Such stimulus may disrupt the microcapsule, an interaction that couples the barcode nucleic acid molecules to or within the microcapsule, or both. Such stimulus can include, for example, a thermal stimulus, photo-stimulus, chemical stimulus (e.g., change in pH or use of a reducing agent(s)), a mechanical stimulus, a radiation stimulus; a biological stimulus (e.g., enzyme), or any combination thereof. Methods and systems for partitioning barcode carrying beads into droplets are provided in US. Patent Publication Nos. 2019/0367997 and 2019/0064173, and International Application Nos. WO2015157567, WO2020167862 and WO2020176882.

In some examples, beads, biological particles, and droplets may flow along channels (e.g., the channels of a microfluidic device). In some examples, beads, biological particles (e.g., labelled B cells or plasma cells) and droplets may flow along channels (e.g., the channels of a microfluidic device), in some cases at substantially regular flow profiles (e.g., at regular flow rates). Such regular flow profiles may permit a droplet to include a single bead and a single biological particle. Such regular flow profiles may permit the droplets to have an occupancy (e.g., droplets having beads and biological particles) greater than 5%, 10%, 20%, 30%, 40%, 50%, 60%, 70%, 80%, 90%, or 95%. Such regular flow profiles and devices that may be used to provide such regular flow profiles are provided in, for example, U.S. Patent Publication No. 2015/0292988.

A bead may be porous, non-porous, solid, semi-solid, semi-fluidic, fluidic, and/or a combination thereof. In some instances, a bead may be dissolvable, disruptable, and/or degradable. In some cases, a bead may not be degradable. In some cases, the bead may be a gel bead. A gel bead may be a hydrogel bead. A gel bead may be formed from molecular precursors, such as a polymeric or monomeric species. A semi-solid bead may be a liposomal bead. Solid beads may comprise metals including iron oxide, gold, and silver. In some cases, the bead may be a silica bead. In some cases, the bead can be rigid. In other cases, the bead may be flexible and/or compressible.

A bead may be of any suitable shape. Examples of bead shapes include, but are not limited to, spherical, non-spherical, oval, oblong, amorphous, circular, cylindrical, and variations thereof.

Beads may be of uniform size or heterogeneous size. In some cases, the diameter of a bead may be at least about 10 nanometers (nm), 100 nm, 500 nm, 1 micrometer ($\mu$m), 5$\mu$m, 10$\mu$m, 20$\mu$m, 30$\mu$m, 40$\mu$m, 50$\mu$m, 60$\mu$m, 70$\mu$m, 80$\mu$m, 90$\mu$m, 100$\mu$m, 250$\mu$m, 500$\mu$m, 1mm, or greater. In some cases, a bead may have a diameter of less than about 10 nm, 100 nm, 500 nm, 1$\mu$m, 5$\mu$m, 10$\mu$m, 20$\mu$m, 30$\mu$m, 40$\mu$m, 50$\mu$m, 60$\mu$m, 70$\mu$m, 80$\mu$m, 90$\mu$m, 100$\mu$m, 250$\mu$m, 500$\mu$m, 1mm, or less. In some cases, a bead may have a diameter in the range of about 40-75$\mu$m, 30-75$\mu$m, 20-75$\mu$m, 40-85$\mu$m, 40-95$\mu$m, 20-100$\mu$m, 10-100$\mu$m, 1-100$\mu$m, 20-250$\mu$m, or 20-500$\mu$m.

In certain aspects, beads can be provided as a population or plurality of beads having a relatively monodisperse size distribution. Where it may be desirable to provide relatively consistent amounts of reagents within partitions, maintaining relatively consistent bead characteristics, such as size, can contribute to the overall consistency. In particular, the beads described herein may have size distributions that have a coefficient of variation in their cross-sectional dimensions of less than 50%, less than 40%, less than 30%, less than 20%, and in some cases less than 15%, less than 10%, less than 5%, or less.

A bead may comprise natural and/or synthetic materials. For example, a bead can comprise a natural polymer, a synthetic polymer or both natural and synthetic polymers. Examples of natural polymers include proteins and sugars such as deoxyribonucleic acid, rubber, cellulose, starch (e.g., amylose, amylopectin), proteins, enzymes, polysaccharides, silks, polyhydroxyalkanoates, chitosan, dextran, collagen, carrageenan, ispaghula, acacia, agar, gelatin, shellac, sterculia gum, xanthan gum, Corn sugar gum, guar gum, gum karaya, agarose, alginic acid, alginate, or natural polymers thereof. Examples of synthetic polymers include acrylics, nylons, silicones, spandex, viscose rayon, polycarboxylic acids, polyvinyl acetate, polyacrylamide, polyacrylate, polyethylene glycol, polyurethanes, polylactic acid, silica, polystyrene, polyacrylonitrile, polybutadiene, polycarbonate, polyethylene, polyethylene terephthalate, poly(chlorotrifluoroethylene), poly(ethylene oxide), poly(ethylene terephthalate), polyethylene, polyisobutylene, poly(methyl methacrylate), poly(oxymethylene), polyformaldehyde, polypropylene, polystyrene, poly(tetrafluoroethylene), poly(vinyl acetate), poly(vinyl alcohol), poly(vinyl chloride), poly(vinylidene dichloride), poly(vinylidene difluoride), poly(vinyl fluoride) and/or combinations (e.g., co-polymers) thereof. Beads may also be formed from materials other than polymers, including lipids, micelles, ceramics, glass-ceramics, material composites, metals, other inorganic materials, and others.

In some instances, the bead may contain molecular precursors (e.g., monomers or polymers), which may form a polymer network via polymerization of the molecular precursors. In some cases, a precursor may be an already polymerized species capable of undergoing further polymerization via, for example, a chemical cross-linkage. In some cases, a precursor can comprise one or more of an acrylamide or a methacrylamide monomer, oligomer, or polymer. In some cases, the bead may comprise prepolymers, which are oligomers capable of further polymerization. For example, polyurethane beads may be prepared using prepolymers. In some cases, the bead may contain individual polymers that may be further polymerized together. In some cases, beads may be generated via polymerization of different precursors, such that they comprise mixed polymers, co-polymers, and/or block co-polymers. In some cases, the bead may comprise covalent or ionic bonds between polymeric precursors (e.g., monomers, oligomers, linear polymers), nucleic acid molecules (e.g., oligonucleotides), primers, and other entities. In some cases, the covalent bonds can be carbon-carbon bonds, thioether bonds, or carbon-heteroatom bonds.

Cross-linking may be permanent or reversible, depending upon the particular cross-linker used. Reversible cross-linking may allow for the polymer to linearize or dissociate under appropriate conditions. In some cases, reversible cross-linking may also allow for reversible attachment of a material bound to the surface of a bead.

In some cases, a cross-linker may form disulfide linkages. In some cases, the chemical cross-linker forming disulfide linkages may be cystamine or a modified cystamine.

In some cases, disulfide linkages can be formed between molecular precursor units (e.g., monomers, oligomers, or linear polymers) or precursors incorporated into a bead and nucleic acid molecules (e.g., oligonucleotides). Cystamine (including modified cystamines), for example, is an organic agent comprising a disulfide bond that may be used as a crosslinker agent between individual monomeric or polymeric precursors of a bead. Polyacrylamide may be polymerized in the presence of cystamine or a species comprising cystamine (e.g., a modified cystamine) to generate polyacrylamide gel beads comprising disulfide linkages (e.g., chemically degradable beads comprising chemically-reducible cross-linkers). The disulfide linkages may permit the bead to be degraded (or dissolved) upon exposure of the bead to a reducing agent.

In some cases, chitosan, a linear polysaccharide polymer, may be crosslinked with glutaraldehyde via hydrophilic chains to form a bead. Crosslinking of chitosan polymers may be achieved by chemical reactions that are initiated by heat, pressure, change in pH, and/or radiation.

In some cases, a bead may comprise an acrydite moiety, which in certain aspects may be used to attach one or more nucleic acid molecules (e.g., barcode sequence, barcoded nucleic acid molecule, barcoded oligonucleotide, primer, or other oligonucleotide) to the bead. In some cases, an acrydite moiety can refer to an acrydite analogue generated from the reaction of acrydite with one or more species, such as, the reaction of acrydite with other monomers and cross-linkers during a polymerization reaction. Acrydite moieties may be modified to form chemical bonds with a species to be attached, such as a nucleic acid molecule (e.g., barcode sequence, barcoded nucleic acid molecule, barcoded oligonucleotide, primer, or other oligonucleotide). Acrydite moieties may be modified with thiol groups capable of forming a disulfide bond or may be modified with groups already comprising a disulfide bond. The thiol or disulfide (via disulfide exchange) may be used as an anchor point for a species to be attached or another part of the acrydite moiety may be used for attachment. In some cases, attachment can be reversible, such that when the disulfide bond is broken (e.g., in the presence of a reducing agent), the attached species is released from the bead. In other cases, an acrydite moiety can comprise a reactive hydroxyl group that may be used for attachment.

Functionalization of beads for attachment of nucleic acid molecules (e.g., oligonucleotides) may be achieved through a wide range of different approaches, including activation of chemical groups within a polymer, incorporation of active or activatable functional groups in the polymer structure, or attachment at the pre-polymer or monomer stage in bead production.

For example, precursors (e.g., monomers, cross-linkers) that are polymerized to form a bead may comprise acrydite moieties, such that when a bead is generated, the bead also comprises acrydite moieties. The acrydite moieties can be attached to a nucleic acid molecule (e.g., oligonucleotide), which may include a priming sequence (e.g., a primer for amplifying target nucleic acids, random primer, primer sequence for messenger RNA) and/or one or more barcode sequences. The one or more barcode sequences may include sequences that are the same for all nucleic acid molecules coupled to a given bead and/or sequences that are different across all nucleic acid molecules coupled to the given bead. The nucleic acid molecule may be incorporated into the bead.

In some cases, the nucleic acid molecule can comprise a functional sequence, for example, for attachment to a sequencing flow cell, such as, for example, a P5 sequence for Illumina® sequencing. In some cases, the nucleic acid molecule or derivative thereof (e.g., oligonucleotide or polynucleotide generated from the nucleic acid molecule) can comprise another functional sequence, such as, for example, a P7 sequence for attachment to a sequencing flow cell for Illumina sequencing. In some cases, the nucleic acid molecule can comprise a barcode sequence. In some cases, the primer can further comprise a unique molecular identifier (UMI). In some cases, the primer can comprise an R1 primer sequence for Illumina sequencing. In some cases, the primer can comprise an R2 primer sequence for Illumina sequencing. Examples of such nucleic acid molecules (e.g., oligonucleotides, polynucleotides, etc.) and uses thereof, as may be used with compositions, devices, methods and systems of the present disclosure, are provided in U.S. Patent Pub. Nos. 2014/0378345 and 2015/0376609. The generation of a barcoded sequence, see, e.g., **FIG. 3,** is described herein.

In some cases, precursors comprising a functional group that is reactive or capable of being activated such that it becomes reactive can be polymerized with other precursors to generate gel beads comprising the activated or activatable functional group. The functional group may then be used to attach additional species (e.g., disulfide linkers, primers, other oligonucleotides, etc.) to the gel beads. For example, some precursors comprising a carboxylic acid (COOH) group can co-polymerize with other precursors to form a gel bead that also comprises a COOH functional group. In some cases, acrylic acid (a species comprising free COOH groups), acrylamide, and bis(acryloyl)cystamine can be co-polymerized together to generate a gel bead comprising free COOH groups. The COOH groups of the gel bead can be activated (e.g., via 1-Ethyl-3-(3-dimethylaminopropyl)carbodiimide (EDC) and N-Hydroxysuccinimide (NHS) or 4-(4,6-Dimethoxy-1,3,5-triazin-2-yl)-4-methylmorpholinium chloride (DMTMM)) such that they are reactive (e.g., reactive to amine functional groups where EDC/NHS or DMTMM are used for activation). The activated COOH groups can then react with an appropriate species (e.g., a species comprising an amine functional group where the carboxylic acid groups are activated to be reactive with an amine functional group) comprising a moiety to be linked to the bead.

Beads comprising disulfide linkages in their polymeric network may be functionalized with additional species via reduction of some of the disulfide linkages to free thiols. The disulfide linkages may be reduced via, for example, the action of a reducing agent (e.g., DTT, TCEP, etc.) to generate free thiol groups, without dissolution of the bead. Free thiols of the beads can then react with free thiols of a species or a species comprising another disulfide bond (e.g., via thiol-disulfide exchange) such that the species can be linked to the beads (e.g., via a generated disulfide bond). In some cases, free thiols of the beads may react with any other suitable group. For example, free thiols of the beads may react with species comprising an acrydite moiety. The free thiol groups of the beads can react with the acrydite via Michael addition chemistry, such that the species comprising the acrydite is linked to the bead. In some cases, uncontrolled reactions can be prevented by inclusion of a thiol capping agent such as N-ethylmalieamide or iodoacetate.

Activation of disulfide linkages within a bead can be controlled such that only a small number of disulfide linkages are activated. Control may be exerted, for example, by controlling the concentration of a reducing agent used to generate free thiol groups and/or concentration of reagents used to form disulfide bonds in bead polymerization. In some cases, a low concentration (e.g., molecules of reducing agent:gel bead ratios of less than or equal to about 1:100,000,000,000, less than or equal to about 1:10,000,000,000, less than or equal to about 1:1,000,000,000, less than or equal to about 1:100,000,000, less than or equal to about 1:10,000,000, less than or equal to about 1:1,000,000, less than or equal to about 1:100,000, less than or equal to about 1: 10,000) of reducing agent may be used for reduction. Controlling the number of disulfide linkages that are reduced to free thiols may be useful in ensuring bead structural integrity during functionalization. In some cases, optically-active agents, such as fluorescent dyes may be coupled to beads via free thiol groups of the beads and used to quantify the number of free thiols present in a bead and/or track a bead.

In some cases, addition of moieties to a gel bead after gel bead formation may be advantageous. For example, addition of an oligonucleotide (e.g., barcoded oligonucleotide, such as a barcoded nucleic acid molecule) after gel bead formation may avoid loss of the species during chain transfer termination that can occur during polymerization. Moreover, smaller precursors (e.g., monomers or cross linkers that do not comprise side chain groups and linked moieties) may be used for polymerization and can be minimally hindered from growing chain ends due to viscous effects. In some cases, functionalization after gel bead synthesis can minimize exposure of species (e.g., oligonucleotides) to be loaded with potentially damaging agents (e.g., free radicals) and/or chemical environments. In some cases, the generated gel may possess an upper critical solution temperature (UCST) that can permit temperature driven swelling and collapse of a bead. Such functionality may aid in oligonucleotide (e.g., a primer) infiltration into the bead during subsequent functionalization of the bead with the oligonucleotide. Post-production functionalization may also be useful in controlling loading ratios of species in beads, such that, for example, the variability in loading ratio is minimized. Species loading may also be performed in a batch process such that a plurality of beads can be functionalized with the species in a single batch.

A bead injected or otherwise introduced into a partition may comprise releasably, cleavably, or reversibly attached barcodes (e.g., partition barcode sequences). A bead injected or otherwise introduced into a partition may comprise activatable barcodes. A bead injected or otherwise introduced into a partition may be degradable, disruptable, or dissolvable beads.

Barcodes can be releasably, cleavably or reversibly attached to the beads such that barcodes can be released or be releasable through cleavage of a linkage between the barcode molecule and the bead, or released through degradation of the underlying bead itself, allowing the barcodes to be accessed or be accessible by other reagents, or both. In non-limiting examples, cleavage may be achieved through reduction of di-sulfide bonds, use of restriction enzymes, photo-activated cleavage, or cleavage via other types of stimuli (e.g., chemical, thermal, pH, enzymatic, etc.) and/or reactions, such as described elsewhere herein. Releasable barcodes may sometimes be referred to as being activatable, in that they are available for reaction once released. Thus, for example, an activatable barcode may be activated by releasing the barcode from a bead (or other suitable type of partition described herein). Other activatable configurations are also envisioned in the context of the described methods and systems.

In addition to, or as an alternative to the cleavable linkages between the beads and the associated molecules, such as barcode containing nucleic acid molecules (e.g., barcode oligonucleotides), the beads may be degradable, disruptable, or dissolvable spontaneously or upon exposure to one or more stimuli (e.g., temperature changes, pH changes, exposure to particular chemical species or phase, exposure to light, reducing agent, etc.). In some cases, a bead may be dissolvable, such that material components of the beads are solubilized when exposed to a particular chemical species or an environmental change, such as a change temperature or a change in pH. In some cases, a gel bead can be degraded or dissolved at elevated temperature and/or in basic conditions. In some cases, a bead may be thermally degradable such that when the bead is exposed to an appropriate change in temperature (e.g., heat), the bead degrades. Degradation or dissolution of a bead bound to a species (e.g., a nucleic acid molecule, e.g., barcode oligonucleotide) may result in release of the species from the bead.

As will be appreciated from the above disclosure, the degradation of a bead may refer to the disassociation of a bound (e.g., capture agent configured to couple to a secreted antibody or antigen binding fragment thereof) or entrained species (e.g., labelled B cell or plasma cell, or secreted antibody or antigen binding fragment thereof) from a bead, both with and without structurally degrading the physical bead itself. For example, the degradation of the bead may involve cleavage of a cleavable linkage via one or more species and/or methods described elsewhere herein. In another example, entrained species may be released from beads through osmotic pressure differences due to, for example, changing chemical environments. By way of example, alteration of bead pore sizes due to osmotic pressure differences can generally occur without structural degradation of the bead itself. In some cases, an increase in pore size due to osmotic swelling of a bead can permit the release of entrained species within the bead. In other cases, osmotic shrinking of a bead may cause a bead to better retain an entrained species due to pore size contraction.

A degradable bead may be introduced into a partition, such as a droplet of an emulsion or a well, such that the bead degrades within the partition and any associated species (e.g., oligonucleotides) are released within the droplet when the appropriate stimulus is applied. The free species (e.g., oligonucleotides, nucleic acid molecules) may interact with other reagents contained in the partition. For example, a polyacrylamide bead comprising cystamine and linked, via a disulfide bond, to a barcode sequence, may be combined with a reducing agent within a droplet of a water-in-oil emulsion. Within the droplet, the reducing agent can break the various disulfide bonds, resulting in bead degradation and release of the species (e.g., oligonucleotides, nucleic acid molecules, barcode sequence) into the aqueous, inner environment of the droplet. In another example, heating of a droplet comprising a bead-bound barcode sequence in basic solution may also result in bead degradation and release of the attached species (e.g., oligonucleotides, nucleic acid molecules barcode sequence) into the aqueous, inner environment of the droplet.

Any suitable number of molecular tag molecules (e.g., primer, barcode oligonucleotide) can be associated with a bead such that, upon release from the bead, the molecular tag molecules (e.g., primer, e.g., barcode oligonucleotide) are present in the partition at a pre-defined concentration. Such pre-defined concentration may be selected to facilitate certain reactions for generating a sequencing library, e.g., amplification, within the partition. In some cases, the pre-defined concentration of the primer can be limited by the process of producing nucleic acid molecule (e.g., oligonucleotide) bearing beads.

In some cases, beads can be non-covalently loaded with one or more reagents. The beads can be non-covalently loaded by, for instance, subjecting the beads to conditions sufficient to swell the beads, allowing sufficient time for the reagents to diffuse into the interiors of the beads, and subjecting the beads to conditions sufficient to de-swell the beads. The swelling of the beads may be accomplished, for instance, by placing the beads in a thermodynamically favorable solvent, subjecting the beads to a higher or lower temperature, subjecting the beads to a higher or lower ion concentration, and/or subjecting the beads to an electric field. The swelling of the beads may be accomplished by various swelling methods. The de-swelling of the beads may be accomplished, for instance, by transferring the beads in a thermodynamically unfavorable solvent, subjecting the beads to lower or high temperatures, subjecting the beads to a lower or higher ion concentration, and/or removing an electric field. The de-swelling of the beads may be accomplished by various de-swelling methods. Transferring the beads may cause pores in the bead to shrink. The shrinking may then hinder reagents within the beads from diffusing out of the interiors of the beads. The hindrance may be due to steric interactions between the reagents and the interiors of the beads. The transfer may be accomplished microfluidically. For instance, the transfer may be achieved by moving the beads from one co-flowing solvent stream to a different co-flowing solvent stream. The swellability and/or pore size of the beads may be adjusted by changing the polymer composition of the bead.

In some cases, an acrydite moiety linked to a precursor, another species linked to a precursor, or a precursor itself can comprise a labile bond, such as chemically, thermally, or photosensitive bond e.g., disulfide bond, UV sensitive bond, or the like. Once acrydite moieties or other moieties comprising a labile bond are incorporated into a bead, the bead may also comprise the labile bond. The labile bond may be, for example, useful in reversibly linking (e.g., covalently linking) species (e.g., barcodes, primers, etc.) to a bead. In some cases, a thermally labile bond may include a nucleic acid hybridization based attachment, e.g., where an oligonucleotide is hybridized to a complementary sequence that is attached to the bead, such that thermal melting of the hybrid releases the oligonucleotide, e.g., a barcode containing sequence, from the bead or microcapsule.

The addition of multiple types of labile bonds to a gel bead may result in the generation of a bead capable of responding to varied stimuli. Each type of labile bond may be sensitive to an associated stimulus (e.g., chemical stimulus, light, temperature, enzymatic, etc.) such that release of species attached to a bead via each labile bond may be controlled by the application of the appropriate stimulus. Such functionality may be useful in controlled release of species from a gel bead. In some cases, another species comprising a labile bond may be linked to a gel bead after gel bead formation via, for example, an activated functional group of the gel bead as described above. As will be appreciated, barcodes that are releasably, cleavably or reversibly attached to the beads described herein include barcodes that are released or releasable through cleavage of a linkage between the barcode molecule and the bead, or that are released through degradation of the underlying bead itself, allowing the barcodes to be accessed or accessible by other reagents, or both.

The barcodes that are releasable as described herein may sometimes be referred to as being activatable, in that they are available for reaction once released. Thus, for example, an activatable barcode may be activated by releasing the barcode from a bead (or other suitable type of partition described herein). Other activatable configurations are also envisioned in the context of the described methods and systems.

In addition to thermally cleavable bonds, disulfide bonds and UV sensitive bonds, other non-limiting examples of labile bonds that may be coupled to a precursor or bead include an ester linkage (e.g., cleavable with an acid, a base, or hydroxylamine), a vicinal diol linkage (e.g., cleavable via sodium periodate), a Diels-Alder linkage (e.g., cleavable via heat), a sulfone linkage (e.g., cleavable via a base), a silyl ether linkage (e.g., cleavable via an acid), a glycosidic linkage (e.g., cleavable via an amylase), a peptide linkage (e.g., cleavable via a protease), or a phosphodiester linkage (e.g., cleavable via a nuclease (e.g., DNAase)). A bond may be cleavable via other nucleic acid molecule targeting enzymes, such as restriction enzymes (e.g., restriction endonucleases), as described further below.

Species may be encapsulated in beads (e.g., capture agent) during bead generation (e.g., during polymerization of precursors). Such species may or may not participate in polymerization. Such species may be entered into polymerization reaction mixtures such that generated beads comprise the species upon bead formation. In some cases, such species may be added to the gel beads after formation. Such species may include, for example, nucleic acid molecules (e.g., oligonucleotides), reagents for a nucleic acid amplification reaction (e.g., primers, polymerases, dNTPs, co-factors (e.g., ionic co-factors), buffers) including those described herein, reagents for enzymatic reactions (e.g., enzymes, co-factors, substrates, buffers), reagents for nucleic acid modification reactions such as polymerization, ligation, or digestion, and/or reagents for template preparation (e.g., tagmentation) for one or more sequencing platforms (e.g., Nextera® for Illumina®). Such species may include one or more enzymes described herein, including without limitation, polymerase, reverse transcriptase, restriction enzymes (e.g., endonuclease), transposase, ligase, proteinase K, DNAse, etc. Such species may include one or more reagents described elsewhere herein (e.g., lysis agents, inhibitors, inactivating agents, chelating agents, stimulus). Trapping of such species may be controlled by the polymer network density generated during polymerization of precursors, control of ionic charge within the gel bead (e.g., via ionic species linked to polymerized species), or by the release of other species. Encapsulated species may be released from a bead upon bead degradation and/or by application of a stimulus capable of releasing the species from the bead. Alternatively or in addition, species may be partitioned in a partition (e.g., droplet) during or subsequent to partition formation. Such species may include, without limitation, the abovementioned species that may also be encapsulated in a bead.

A degradable bead may comprise one or more species with a labile bond such that, when the bead/species is exposed to the appropriate stimuli, the bond is broken and the bead degrades. The labile bond may be a chemical bond (e.g., covalent bond, ionic bond) or may be another type of physical interaction (e.g., van der Waals interactions, dipole-dipole interactions, etc.). In some cases, a crosslinker used to generate a bead may comprise a labile bond. Upon exposure to the appropriate conditions, the labile bond can be broken and the bead degraded. For example, upon exposure of a polyacrylamide gel bead comprising cystamine crosslinkers to a reducing agent, the disulfide bonds of the cystamine can be broken and the bead degraded.

A degradable bead may be useful in more quickly releasing an attached species (e.g., a nucleic acid molecule, a barcode sequence, a primer, etc) from the bead when the appropriate stimulus is applied to the bead as compared to a bead that does not degrade. For example, for a species bound to an inner surface of a porous bead or in the case of an encapsulated species, the species may have greater mobility and accessibility to other species in solution upon degradation of the bead. In some cases, a species may also be attached to a degradable bead via a degradable linker (e.g., disulfide linker). The degradable linker may respond to the same stimuli as the degradable bead or the two degradable species may respond to different stimuli. For example, a species (e.g., a nucleic acid molecule, a barcode sequence, a primer, etc) may be attached, via a disulfide bond, to a polyacrylamide bead comprising cystamine. Upon exposure of the barcoded-bead to a reducing agent, the bead degrades and the species (e.g., nucleic acid molecule, barcode sequence, primer) is released upon breakage of both the disulfide linkage between the barcode sequence and the bead and the disulfide linkages of the cystamine in the bead.

As will be appreciated from the above disclosure, while referred to as degradation of a bead, in many instances as noted above, that degradation may refer to the disassociation of a bound or entrained species from a bead, both with and without structurally degrading the physical bead itself. For example, entrained species may be released from beads through osmotic pressure differences due to, for example, changing chemical environments. By way of example, alteration of bead pore sizes due to osmotic pressure differences can generally occur without structural degradation of the bead itself. In some cases, an increase in pore size due to osmotic swelling of a bead can permit the release of entrained species within the bead. In other cases, osmotic shrinking of a bead may cause a bead to better retain an entrained species due to pore size contraction.

Where degradable beads are provided, it may be beneficial to avoid exposing such beads to the stimulus or stimuli that cause such degradation prior to a given time, in order to, for example, avoid premature bead degradation and issues that arise from such degradation, including for example poor flow characteristics and aggregation. By way of example, where beads comprise reducible cross-linking groups, such as disulfide groups, it will be desirable to avoid contacting such beads with reducing agents, e.g., DTT or other disulfide cleaving reagents. In such cases, treatment to the beads described herein will, in some cases be provided free of reducing agents, such as DTT. Because reducing agents are often provided in commercial enzyme preparations, it may be desirable to provide reducing agent free (or DTT free) enzyme preparations in treating the beads described herein. Examples of such enzymes include, e.g., polymerase enzyme preparations, reverse transcriptase enzyme preparations, ligase enzyme preparations, as well as many other enzyme preparations that may be used to treat the beads described herein. The terms "reducing agent free" or "DTT free" preparations can refer to a preparation having less than about 1/10th, less than about 1/50th, or even less than about 1/100th of the lower ranges for such materials used in degrading the beads. For example, for DTT, the reducing agent free preparation can have less than about 0.01 millimolar (mM), 0.005 mM, 0.001 mM DTT, 0.0005 mM DTT, or even less than about 0.0001 mM DTT. In many cases, the amount of DTT can be undetectable.

Numerous chemical triggers may be used to trigger the degradation of beads. Examples of these chemical changes may include, but are not limited to pH-mediated changes to the integrity of a component within the bead, degradation of a component of a bead via cleavage of cross-linked bonds, and depolymerization of a component of a bead.

In some embodiments, a bead may be formed from materials that comprise degradable chemical crosslinkers, such as BAC or cystamine. Degradation of such degradable crosslinkers may be accomplished through a number of mechanisms. In some examples, a bead may be contacted with a chemical degrading agent that may induce oxidation, reduction or other chemical changes. For example, a chemical degrading agent may be a reducing agent, such as dithiothreitol (DTT). Additional examples of reducing agents may include β-mercaptoethanol, (2S)-2-amino-1,4-dimercaptobutane (dithiobutylamine or DTBA), tris(2-carboxyethyl) phosphine (TCEP), or combinations thereof. A reducing agent may degrade the disulfide bonds formed between gel precursors forming the bead, and thus, degrade the bead. In other cases, a change in pH of a solution, such as an increase in pH, may trigger degradation of a bead. In other cases, exposure to an aqueous solution, such as water, may trigger hydrolytic degradation, and thus degradation of the bead. In some cases, any combination of stimuli may trigger degradation of a bead. For example, a change in pH may enable a chemical agent (e.g., DTT) to become an effective reducing agent.

Beads may also be induced to release their contents upon the application of a thermal stimulus. A change in temperature can cause a variety of changes to a bead. For example, heat can cause a solid bead to liquefy. A change in heat may cause melting of a bead such that a portion of the bead degrades. In other cases, heat may increase the internal pressure of the bead components such that the bead ruptures or explodes. Heat may also act upon heat-sensitive polymers used as materials to construct beads.

Any suitable agent may degrade beads. In some embodiments, changes in temperature or pH may be used to degrade thermo-sensitive or pH-sensitive bonds within beads. In some embodiments, chemical degrading agents may be used to degrade chemical bonds within beads by oxidation, reduction or other chemical changes. For example, a chemical degrading agent may be a reducing agent, such as DTT, wherein DTT may degrade the disulfide bonds formed between a crosslinker and gel precursors, thus degrading the bead. In some embodiments, a reducing agent may be added to degrade the bead, which may or may not cause the bead to release its contents. Examples of reducing agents may include dithiothreitol (DTT), β-mercaptoethanol, (2S)-2-amino-1,4-dimercaptobutane (dithiobutylamine or DTBA), tris(2-carboxyethyl) phosphine (TCEP), or combinations thereof. The reducing agent may be present at a concentration of about 0.1mM, 0.5mM, 1mM, 5mM, 10mM. The reducing agent may be present at a concentration of at least about 0.1mM, 0.5mM, 1mM, 5mM, 10mM, or greater than 10 mM. The reducing agent may be present at concentration of at most about 10mM, 5mM, 1mM, 0.5mM, 0.1mM, or less.

Any suitable number of molecular tag molecules (e.g., primer, barcode oligonucleotide) can be associated with a bead such that, upon release from the bead, the molecular tag molecules (e.g., primer, e.g., barcode oligonucleotide) are present in the partition at a predefined concentration. Such pre-defined concentration may be selected to facilitate certain reactions for generating a sequencing library, e.g., amplification, within the partition. In some cases, the pre-defined concentration of the primer can be limited by the process of producing oligonucleotide bearing beads.

Although **FIG. 1** and **FIG. 2** have been described in terms of providing substantially singly occupied partitions, above, in certain cases, it may be desirable to provide multiply occupied partitions, e.g., containing two, three, four or more cells and/or microcapsules (e.g., beads) comprising barcode nucleic acid molecules (e.g., oligonucleotides) within a single partition (e.g., multi-omic method described elsewhere, herein). Accordingly, as noted above, the flow characteristics of the biological particle and/or bead containing fluids and partitioning fluids may be controlled to provide for such multiply occupied partitions. In particular, the flow parameters may be controlled to provide a given occupancy rate at greater than about 50% of the partitions, greater than about 75%, and in some cases greater than about 80%, 90%, 95%, or higher.

In some cases, additional microcapsules can be used to deliver additional reagents to a partition. In such cases, it may be advantageous to introduce different beads into a common channel or droplet generation junction, from different bead sources (e.g., containing different associated reagents) through different channel inlets into such common channel or droplet generation junction. In such cases, the flow and frequency of the different beads into the channel or junction may be controlled to provide for a certain ratio of microcapsules from each source, while ensuring a given pairing or combination of such beads into a partition with a given number of biological particles (e.g., one biological particle and one bead per partition).

The partitions described herein may comprise small volumes, for example, less than about 10 microliters (μL), 5μL, 1μL, 900 picoliters (pL), 800 pL, 700 pL, 600 pL, 500 pL, 400pL, 300 pL, 200 pL, 100pL, 50 pL, 20 pL, 10 pL, 1 pL, 500 nanoliters (nL), 100 nL, 50 nL, or less.

For example, in the case of droplet based partitions, the droplets may have overall volumes that are less than about 1000 pL, 900 pL, 800 pL, 700 pL, 600 pL, 500 pL, 400pL, 300 pL, 200 pL, 100pL, 50 pL, 20 pL, 10 pL, 1 pL, or less. Where co-partitioned with microcapsules, it will be appreciated that the sample fluid volume, e.g., including co-partitioned biological particles and/or beads, within the partitions may be less than about 90% of the above described volumes, less than about 80%, less than about 70%, less than about 60%, less than about 50%, less than about 40%, less than about 30%, less than about 20%, or less than about 10% of the above described volumes.

As is described elsewhere herein, partitioning species may generate a population or plurality of partitions. In such cases, any suitable number of partitions can be generated or otherwise provided. For example, at least about 1,000 partitions, at least about 5,000 partitions, at least about 10,000 partitions, at least about 50,000 partitions, at least about 100,000 partitions, at least about 500,000 partitions, at least about 1,000,000 partitions, at least about 5,000,000 partitions at least about 10,000,000 partitions, at least about 50,000,000 partitions, at least about 100,000,000 partitions, at least about 500,000,000 partitions, at least about 1,000,000,000 partitions, or more partitions can be generated or otherwise provided. Moreover, the plurality of partitions may comprise both unoccupied partitions (e.g., empty partitions) and occupied partitions.

## D. Reagents

In accordance with certain aspects, biological particles may be partitioned along with lysis reagents in order to release the contents of the biological particles within the partition. See, e.g., U.S. Pat. Pub. 2018/0216162 (now U.S. Pat. 10,428,326), U.S. Pat. Pub. 2019/0100632 (now U.S. Pat. 10,590,244), and U.S. Pat. Pub. 2019/0233878. Cell beads may be partitioned together with nucleic acid barcode molecules and the nucleic acid molecules of or derived from the cell bead (e.g., mRNA, cDNA, gDNA, secreted antibodies or antigen binding fragments thereof, etc.) can be barcoded as described elsewhere herein. In some embodiments, cell beads are co-partitioned with barcode carrying beads (e.g., gel beads) and the nucleic acid molecules of or derived from the cell bead are barcoded as described elsewhere herein. In such cases, the lysis agents can be contacted with the biological particle (e.g., labelled B cell or plasma cell) suspension concurrently with, or immediately prior to, the introduction of the biological particles into the partitioning junction/droplet generation zone, such as through an additional channel or channels upstream of the channel junction. In accordance with other aspects, additionally or alternatively, biological particles may be partitioned along with other reagents, as will be described further below.

Beneficially, when lysis reagents and biological particles are co-partitioned, the lysis reagents can facilitate the release of the contents of the biological particles within the partition. The contents released in a partition may remain discrete from the contents of other partitions.

As will be appreciated, the channel segments described herein may be coupled to any of a variety of different fluid sources or receiving components, including reservoirs, tubing, manifolds, or fluidic components of other systems. As will be appreciated, the microfluidic channel structures may have other geometries and/or configurations. For example, a microfluidic channel structure can have more than two channel junctions. For example, a microfluidic channel structure can have 2, 3, 4, 5 channel segments or more each carrying the same or different types of beads, reagents, and/or biological particles that meet at a channel junction. Fluid flow in each channel segment may be controlled to control the partitioning of the different elements into droplets. Fluid may be directed flow along one or more channels or reservoirs via one or more fluid flow units. A fluid flow unit can comprise compressors (e.g., providing positive pressure), pumps (e.g., providing negative pressure), actuators, and the like to control flow of the fluid. Fluid may also or otherwise be controlled via applied pressure differentials, centrifugal force, electrokinetic pumping, vacuum, capillary or gravity flow, or the like.

Examples of lysis agents include bioactive reagents, such as lysis enzymes that are used for lysis of different cell types, e.g., gram positive or negative bacteria, plants, yeast, mammalian, etc., such as lysozymes, achromopeptidase, lysostaphin, labiase, kitalase, lyticase, and a variety of other lysis enzymes available from, e.g., Sigma-Aldrich, Inc. (St Louis, MO), as well as other commercially available lysis enzymes. Other lysis agents may additionally or alternatively be co-partitioned with the biological particles to cause the release of the biological particle's contents into the partitions. For example, in some cases, surfactant-based lysis solutions may be used to lyse cells (e.g., labelled B cell or plasma cell), although these may be less desirable for emulsion based systems where the surfactants can interfere with stable emulsions. In some cases, lysis solutions may include non-ionic surfactants such as, for example, TritonX-100 and Tween 20. In some cases, lysis solutions may include ionic surfactants such as, for example, sarcosyl and sodium dodecyl sulfate (SDS). Electroporation, thermal, acoustic or mechanical cellular disruption may also be used in certain cases, e.g., non-emulsion based partitioning such as encapsulation of biological particles that may be in addition to or in place of droplet partitioning, where any pore size of the encapsulate is sufficiently small to retain nucleic acid fragments of a given size, following cellular disruption.

Alternatively or in addition to the lysis agents co-partitioned with the biological particles (e.g., labelled B cells or plasma cells) described above, other reagents can also be co-partitioned with the biological particles, including, for example, DNase and RNase inactivating agents or inhibitors, such as proteinase K, chelating agents, such as EDTA, and other reagents employed in removing or otherwise reducing negative activity or impact of different cell lysate components on subsequent processing of nucleic acids. In addition, in the case of encapsulated biological particles (e.g., labelled B cells or plasma cells), the biological particles may be exposed to an appropriate stimulus to release the biological particles or their contents from a co-partitioned microcapsule. For example, in some cases, a chemical stimulus may be co-partitioned along with an encapsulated biological particle to allow for the degradation of the microcapsule and release of the cell or its contents into the larger partition. In some cases, this stimulus may be the same as the stimulus described elsewhere herein for release of nucleic acid molecules (e.g., oligonucleotides) from their respective microcapsule (e.g., bead). In alternative aspects, this may be a different and non-overlapping stimulus, in order to allow an encapsulated biological particle to be released into a partition at a different time from the release of nucleic acid molecules into the same partition.

Additional reagents may also be co-partitioned with the biological particles (e.g., labelled B cell or plasma cell), such as endonucleases to fragment a biological particle's DNA, DNA polymerase enzymes and dNTPs used to amplify the biological particle's nucleic acid fragments and to attach the barcode molecular tags to the amplified fragments. Other enzymes may be co-partitioned, including without limitation, polymerase, transposase, ligase, proteinase K, DNAse, etc. Additional reagents may also include reverse transcriptase enzymes, including enzymes with terminal transferase activity, primers and oligonucleotides, and switch oligonucleotides (also referred to herein as "switch oligos" or "template switching oligonucleotides") which can be used for template switching. In some cases, template switching can be used to increase the length of a cDNA. In some cases, template switching can be used to append a predefined nucleic acid sequence to the cDNA. In an example of template switching, cDNA can be generated from reverse transcription of a template, e.g., cellular mRNA, where a reverse transcriptase with terminal transferase activity can add additional nucleotides, e.g., polyC, to the cDNA in a template independent manner. Switch oligos can include sequences complementary to the additional nucleotides, e.g., polyG. The additional nucleotides (e.g., polyC) on the cDNA can hybridize to the additional nucleotides (e.g., polyG) on the switch oligo, whereby the switch oligo can be used by the reverse transcriptase as template to further extend the cDNA. Template switching oligonucleotides may comprise a hybridization region and a template region. The hybridization region can comprise any sequence capable of hybridizing to the target. In some cases, as previously described, the hybridization region comprises a series of G bases to complement the overhanging C bases at the 3' end of a cDNA molecule. The series of G bases may comprise 1 G base, 2 G bases, 3 G bases, 4 G bases, 5 G bases or more than 5 G bases. The template sequence can comprise any sequence to be incorporated into the cDNA. In some cases, the template region comprises at least 1 (e.g., at least 2, 3, 4, 5 or more) tag sequences and/or functional sequences. Switch oligos may comprise deoxyribonucleic acids; ribonucleic acids; modified nucleic acids including 2-Aminopurine, 2,6-Diaminopurine (2-Amino-dA), inverted dT, 5-Methyl dC, 2'-deoxyInosine, Super T (5-hydroxybutynl-2'-deoxyuridine), Super G (8-aza-7-deazaguanosine), locked nucleic acids (LNAs), unlocked nucleic acids (UNAs, e.g., UNA-A, UNA-U, UNA-C, UNA-G), Iso-dG, Iso-dC, 2' Fluoro bases (e.g., Fluoro C, Fluoro U, Fluoro A, and Fluoro G), or any combination.

In some cases, the length of a switch oligo may be at least about 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197 , 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249 or 250 nucleotides or longer.

In some cases, the length of a switch oligo may be at most about 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197 , 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249 or 250 nucleotides.

Once the contents of the cells (e.g., labelled B cells or plasma cells) are released into their respective partitions, the macromolecular components (e.g., macromolecular constituents of biological particles, such as RNA, DNA, proteins, or secreted antibodies or antigen binding fragments thereof) contained therein may be further processed within the partitions. In accordance with the methods and systems described herein, the macromolecular component contents of individual biological particles (e.g., labelled B cells or plasma cells) can be provided with unique identifiers such that, upon characterization of those macromolecular components they may be attributed as having been derived from the same biological particle or particles. The ability to attribute characteristics to individual biological particles or groups of biological particles is provided by the assignment of unique identifiers specifically to an individual biological particle or groups of biological particles. Unique identifiers, e.g., in the form of nucleic acid barcodes can be assigned or associated with individual biological particles or populations of biological particles, in order to tag or label the biological particle's macromolecular components (and as a result, its characteristics) with the unique identifiers. These unique identifiers can then be used to attribute the biological particle's components and characteristics to an individual biological particle or group of biological particles.

In some aspects, this is performed by co-partitioning the individual biological particle (e.g., labelled B cell or plasma cell) or groups of biological particles (e.g.,

labelled B cells or plasma cells) with the unique identifiers, such as described above (with reference to **FIGS. 1 and 2**). In some aspects, the unique identifiers are provided in the form of nucleic acid molecules (e.g., oligonucleotides) that comprise nucleic acid barcode sequences that may be attached to or otherwise associated with the nucleic acid contents of individual biological particle, or to other components of the biological particle, and particularly to fragments of those nucleic acids. The nucleic acid molecules are partitioned such that as between nucleic acid molecules in a given partition, the nucleic acid barcode sequences contained therein are the same, but as between different partitions, the nucleic acid molecule can, and do have differing barcode sequences, or at least represent a large number of different barcode sequences across all of the partitions in a given analysis. In some aspects, only one nucleic acid barcode sequence can be associated with a given partition, although in some cases, two or more different barcode sequences may be present.

The nucleic acid barcode sequences can include from about 6 to about 20 or more nucleotides within the sequence of the nucleic acid molecules (e.g., oligonucleotides). The nucleic acid barcode sequences can include from about 6 to about 20, 30, 40, 50, 60, 70, 80, 90, 100 or more nucleotides. In some cases, the length of a barcode sequence may be about 6, 7, 8, 9, 10, 11, 12, 13, 14, 15, 16, 17, 18, 19, 20 nucleotides or longer. In some cases, the length of a barcode sequence may be at least about 6, 7, 8, 9, 10, 11, 12, 13, 14, 15, 16, 17, 18, 19, 20 nucleotides or longer. In some cases, the length of a barcode sequence may be at most about 6, 7, 8, 9, 10, 11, 12, 13, 14, 15, 16, 17, 18, 19, 20 nucleotides or shorter. These nucleotides may be completely contiguous, i.e., in a single stretch of adjacent nucleotides, or they may be separated into two or more separate subsequences that are separated by 1 or more nucleotides. In some cases, separated barcode subsequences can be from about 4 to about 16 nucleotides in length. In some cases, the barcode subsequence may be about 4, 5, 6, 7, 8, 9, 10, 11, 12, 13, 14, 15, 16 nucleotides or longer. In some cases, the barcode subsequence may be at least about 4, 5, 6, 7, 8, 9, 10, 11, 12, 13, 14, 15, 16 nucleotides or longer. In some cases, the barcode subsequence may be at most about 4, 5, 6, 7, 8, 9, 10, 11, 12, 13, 14, 15, 16 nucleotides or shorter.

The co-partitioned nucleic acid molecules can also comprise other functional sequences useful in the processing of the nucleic acids from the co-partitioned biological particles (e.g., labelled B cells or plasma cells). These sequences include, e.g., targeted or random/universal amplification primer sequences for amplifying the genomic DNA from the individual biological particles within the partitions while attaching the associated barcode sequences, sequencing primers or primer recognition sites, hybridization or probing sequences, e.g., for identification of presence of the sequences or for pulling down barcoded nucleic acids, or any of a number of other potential functional sequences. Other mechanisms of co-partitioning oligonucleotides may also be employed, including, e.g., coalescence of two or more droplets, where one droplet contains oligonucleotides, or microdispensing of oligonucleotides into partitions, e.g., droplets within microfluidic systems.

In an example, microcapsules, such as beads, are provided that each include large numbers of the above described barcode nucleic acid molecules (e.g., barcode oligonucleotides) releasably attached to the beads, where all of the nucleic acid molecules attached to a particular bead will include the same nucleic acid barcode sequence, but where a large number of diverse barcode sequences are represented across the population of beads used. In some embodiments, hydrogel beads, e.g., comprising polyacrylamide polymer matrices, are used as a solid support and delivery vehicle for the nucleic acid molecules into the partitions, as they are capable of carrying large numbers of nucleic acid molecules, and may be configured to release those nucleic acid molecules upon exposure to a particular stimulus, as described elsewhere herein. In some cases, the population of beads provides a diverse barcode sequence library that includes at least about 1,000 different barcode sequences, at least about 5,000 different barcode sequences, at least about 10,000 different barcode sequences, at least about 50,000 different barcode sequences, at least about 100,000 different barcode sequences, at least about 1,000,000 different barcode sequences, at least about 5,000,000 different barcode sequences, or at least about 10,000,000 different barcode sequences, or more. Additionally, each bead can be provided with large numbers of nucleic acid (e.g., oligonucleotide) molecules attached. In particular, the number of molecules of nucleic acid molecules including the barcode sequence on an individual bead can be at least about 1,000 nucleic acid molecules, at least about 5,000 nucleic acid molecules, at least about 10,000 nucleic acid molecules, at least about 50,000 nucleic acid molecules, at least about 100,000 nucleic acid molecules, at least about 500,000 nucleic acids, at least about 1,000,000 nucleic acid molecules, at least about 5,000,000 nucleic acid molecules, at least about 10,000,000 nucleic acid molecules, at least about 50,000,000 nucleic acid molecules, at least about 100,000,000 nucleic acid molecules, at least about 250,000,000 nucleic acid molecules and in some cases at least about 1 billion nucleic acid molecules, or more. Nucleic acid molecules of a given bead can include identical (or common) barcode sequences, different barcode sequences, or a combination of both. Nucleic acid molecules of a given bead can include multiple sets of nucleic acid molecules. Nucleic acid molecules of a given set can include identical barcode sequences. The identical barcode sequences can be different from barcode sequences of nucleic acid molecules of another set.

Moreover, when the population of beads is partitioned, the resulting population of partitions can also include a diverse barcode library that includes at least about 1,000 different barcode sequences, at least about 5,000 different barcode sequences, at least about 10,000 different barcode sequences, at least at least about 50,000 different barcode sequences, at least about 100,000 different barcode sequences, at least about 1,000,000 different barcode sequences, at least about 5,000,000 different barcode sequences, or at least about 10,000,000 different barcode sequences. Additionally, each partition of the population can include at least about 1,000 nucleic acid molecules, at least about 5,000 nucleic acid molecules, at least about 10,000 nucleic acid molecules, at least about 50,000 nucleic acid molecules, at least about 100,000 nucleic acid molecules, at least about 500,000 nucleic acids, at least about 1,000,000 nucleic acid molecules, at least about 5,000,000 nucleic acid molecules, at least about 10,000,000 nucleic acid molecules, at least about 50,000,000 nucleic acid molecules, at least about 100,000,000 nucleic acid molecules, at least about 250,000,000 nucleic acid molecules and in some cases at least about 1 billion nucleic acid molecules.

In some cases, it may be desirable to incorporate multiple different barcodes within a given partition, either attached to a single or multiple beads within the partition. For example, in some cases, a mixed, but known set of barcode sequences may provide greater assurance of identification in the subsequent processing, e.g., by providing a stronger address or attribution of the barcodes to a given partition, as a duplicate or independent confirmation of the output from a given partition.

The nucleic acid molecules (e.g., oligonucleotides) are releasable from the beads upon the application of a particular stimulus to the beads. In some cases, the stimulus may be a photo-stimulus, e.g., through cleavage of a photo-labile linkage that releases the nucleic acid molecules. In other cases, a thermal stimulus may be used, where elevation of the temperature of the beads environment will result in cleavage of a linkage or other release of the nucleic acid molecules from the beads. In still other cases, a chemical stimulus can be used that cleaves a linkage of the nucleic acid molecules to the beads, or otherwise results in release of the nucleic acid molecules from the beads. In one case, such compositions include the polyacrylamide matrices described above for encapsulation of biological particles, and may be degraded for release of the attached nucleic acid molecules through exposure to a reducing agent, such as DTT.

In some aspects, provided are systems and methods for controlled partitioning. Droplet size may be controlled by adjusting certain geometric features in channel architecture (e.g., microfluidics channel architecture). For example, an expansion angle, width, and/or length of a channel may be adjusted to control droplet size.

**FIG. 2** shows an example of a microfluidic channel structure for the controlled partitioning of beads into discrete droplets. A channel structure **200** can include a channel segment **202** communicating at a channel junction **206** (or intersection) with a reservoir **204**. The reservoir **204** can be a chamber. Any reference to "reservoir," as used herein, can also refer to a "chamber." In operation, an aqueous fluid **208** that includes suspended beads **212** may be transported along the channel segment **202** into the junction **206** to meet a second fluid **210** that is immiscible with the aqueous fluid **208** in the reservoir **204** to create droplets **216, 218** of the aqueous fluid **208** flowing into the reservoir **204**. At the junction **206** where the aqueous fluid **208** and the second fluid **210** meet, droplets can form based on factors such as the hydrodynamic forces at the junction **206**, flow rates of the two fluids **208, 210**, fluid properties, and certain geometric parameters (e.g., $w$, $h_0$, $\alpha$, etc.) of the channel structure **200**. A plurality of droplets can be collected in the reservoir **204** by continuously injecting the aqueous fluid **208** from the channel segment **202** through the junction **206**.

A discrete droplet generated may include a bead (e.g., as in occupied droplets **216**). Alternatively, a discrete droplet generated may include more than one bead. Alternatively, a discrete droplet generated may not include any beads (e.g., as in unoccupied droplet **218**). In some instances, a discrete droplet generated may contain one or more biological particles, as described elsewhere herein. In some instances, a discrete droplet generated may comprise one or more reagents, as described elsewhere herein.

In some instances, the aqueous fluid **208** can have a substantially uniform concentration or frequency of beads **212**. The beads **212** can be introduced into the channel segment **202** from a separate channel (not shown in **FIG. 2**). The frequency of beads **212** in the channel segment **202** may be controlled by controlling the frequency in which the beads **212** are introduced into the channel segment **202** and/or the relative flow rates of the fluids in the channel segment 202 and the separate channel. In some instances, the beads can be introduced into the channel segment **202** from a plurality of different channels, and the frequency controlled accordingly.

In some instances, the aqueous fluid **208** in the channel segment **202** can comprise biological particles (e.g., described with reference to **FIG. 1**). In some instances, the aqueous fluid **208** can have a substantially uniform concentration or frequency of biological particles. As with the beads, the biological particles (e.g., labelled B cells or plasma cells) can be introduced into the channel segment **202** from a separate channel. The frequency or concentration of the biological particles in the aqueous fluid **208** in the channel segment **202** may be controlled by controlling the frequency in which the biological particles are introduced into the channel segment **202** and/or the relative flow rates of the fluids in the channel segment **202** and the separate channel. In some instances, the biological particles can be introduced into the channel segment **202** from a plurality of different channels, and the frequency controlled accordingly. In some instances, a first separate channel can introduce beads and a second separate channel can introduce biological particles into the channel segment **202**. The first separate channel introducing the beads may be upstream or downstream of the second separate channel introducing the biological particles.

The second fluid **210** can comprise an oil, such as a fluorinated oil, that includes a fluorosurfactant for stabilizing the resulting droplets, for example, inhibiting subsequent coalescence of the resulting droplets.

In some instances, the second fluid **210** may not be subjected to and/or directed to any flow in or out of the reservoir **204**. For example, the second fluid **210** may be substantially stationary in the reservoir **204**. In some instances, the second fluid **210** may be subjected to flow within the reservoir **204**, but not in or out of the reservoir **204**, such as via application of pressure to the reservoir **204** and/or as affected by the incoming flow of the aqueous fluid **208** at the junction **206**. Alternatively, the second fluid **210** may be subjected and/or directed to flow in or out of the reservoir **204**. For example, the reservoir **204** can be a channel directing the second fluid **210** from upstream to downstream, transporting the generated droplets.

The channel structure **200** at or near the junction **206** may have certain geometric features that at least partly determine the sizes of the droplets formed by the channel structure **200**. The channel segment **202** can have a height, $h_0$ and width, $w$, at or near the junction **206**. By way of example, the channel segment **202** can comprise a rectangular cross-section that leads to a reservoir **204** having a wider cross-section (such as in width or diameter). Alternatively, the cross-section of the channel segment **202** can be other shapes, such as a circular shape, trapezoidal shape, polygonal shape, or any other shapes. The top and bottom walls of the reservoir **204** at or near the junction **206** can be inclined at an expansion angle, $\alpha$. The expansion angle, $\alpha$, allows the tongue (portion of the aqueous fluid **208** leaving channel segment **202** at junction **206** and entering the reservoir **204** before droplet formation) to increase in depth and facilitate decrease in curvature of the intermediately formed droplet. Droplet size may decrease with increasing expansion angle. The resulting droplet radius, $R_d$, may be predicted by the following equation for the aforementioned geometric parameters of $h_0$, $w$, and $\alpha$:

$$R_d \approx 0.44 \left( 1 + 2.2\sqrt{\tan \alpha}\, \frac{w}{h_0} \right) \frac{h_0}{\sqrt{\tan \alpha}}$$

By way of example, for a channel structure with $w$ = 21 μm, $h$ = 21 μm, and $\alpha$ = 3°, the predicted droplet size is 121 μm. In another example, for a channel structure with $w$ = 25 μm, $h$ = 25 μm, and $\alpha$ = 5°, the predicted droplet size is 123 μm. In another example, for a channel structure with $w$ = 28 μm, $h$ = 28 μm, and $\alpha$ = 7°, the predicted droplet size is 124 μm.

In some instances, the expansion angle, $\alpha$, may be between a range of from about 0.5° to about 4°, from about 0.1° to about 10°, or from about 0° to about 90°. For example, the expansion angle can be at least about 0.01°, 0.1°, 0.2°, 0.3°, 0.4°, 0.5°, 0.6°, 0.7°, 0.8°, 0.9°, 1°, 2°, 3°, 4°, 5°, 6°, 7°, 8°, 9°, 10°, 15°, 20°, 25°, 30°, 35°, 40°, 45°, 50°, 55°, 60°, 65°, 70°, 75°, 80°, 85°, or higher. In some instances, the expansion angle can be at most about 89°, 88°, 87°, 86°, 85°, 84°, 83°, 82°, 81°, 80°, 75°, 70°, 65°, 60°, 55°, 50°, 45°, 40°, 35°, 30°, 25°, 20°, 15°, 10°, 9°, 8°, 7°, 6°, 5°, 4°, 3°, 2°, 1°, 0.1°, 0.01°, or less. In some instances, the width, $w$, can be between a range of from about 100 micrometers (μm) to about 500 μm. In some instances, the width, $w$, can be between a range of from about 10 μm to about 200 μm. Alternatively, the width can be less than about 10 μm. Alternatively, the width can be greater than about 500 μm. In some instances, the flow rate of the aqueous fluid **208** entering the junction **206** can be between about 0.04 microliters (μL)/minute (min) and about 40 μL/min. In some instances, the flow rate of the aqueous fluid **208** entering the junction **206** can be between about 0.01 microliters (μL)/minute (min) and about 100 μL/min. Alternatively, the flow rate of the aqueous fluid **208** entering the junction **206** can be less than about 0.01 μL/min. Alternatively, the flow rate of the aqueous fluid **208** entering the junction **206** can be greater than about 40 μL/min, such as 45 μL/min, 50 μL/min, 55 μL/min, 60 μL/min, 65 μL/min, 70 μL/min, 75 μL/min, 80 μL/min, 85 μL/min, 90 μL/min, 95 μL/min, 100 μL/min, 110 μL/min , 120 μL/min , 130 μL/min , 140 μL/min , 150 μL/min, or greater. At lower flow rates, such as flow rates of about less than or equal to 10 microliters/minute, the droplet radius may not be dependent on the flow rate of the aqueous fluid **208** entering the junction **206**.

In some instances, at least about 50% of the droplets generated can have uniform size. In some instances, at least about 55%, 60%, 65%, 70%, 75%, 80%, 85%, 90%, 95%, 96%, 97%, 98%, 99%, or greater of the droplets generated can have uniform size. Alternatively, less than about 50% of the droplets generated can have uniform size.

The throughput of droplet generation can be increased by increasing the points of generation, such as increasing the number of junctions (e.g., junction **206**) between aqueous fluid **208** channel segments (e.g., channel segment **202**) and the reservoir **204**. Alternatively or in addition, the throughput of droplet generation can be increased by increasing the flow rate of the aqueous fluid **208** in the channel segment **202**.

The methods and systems described herein may be used to greatly increase the efficiency of single cell applications and/or other applications receiving droplet-based input. For example, following the sorting of occupied cells and/or appropriately-sized cells, subsequent operations that can be performed can include generation of amplification products, purification (e.g., via solid phase reversible immobilization (SPRI)), further processing (e.g., shearing, ligation of functional sequences, and subsequent amplification (e.g., via PCR)). These operations may occur in bulk (e.g., outside the partition). In the case where a partition is a droplet in an emulsion, the emulsion can be broken and the contents of the droplet pooled for additional operations. Additional reagents that may be co-partitioned along with the barcode bearing bead may include oligonucleotides to block ribosomal RNA (rRNA) and nucleases to digest genomic DNA from cells. Alternatively, rRNA removal agents may be applied during additional processing operations. The configuration of the constructs generated by such a method can help minimize (or avoid) sequencing of the poly-T sequence during sequencing and/or sequence the 5' end of a polynucleotide sequence. The amplification products, for example, first amplification products and/or second amplification products, may be subject to sequencing for sequence analysis. In some cases, amplification may be performed using the Partial Hairpin Amplification for Sequencing (PHASE) method.

A variety of applications require the evaluation of the presence and quantification of different biological particle or organism types within a population of biological particles, including, for example, microbiome analysis and characterization, environmental testing, food safety testing, epidemiological analysis, e.g., in tracing contamination or the like.

Partitions comprising a barcode bead (e.g., a gel bead) associated with barcode molecules and a bead encapsulating cellular constituents (e.g., a cell bead) such as cellular nucleic acids can be useful in constituent analysis as is described in U.S. Patent Publication No. 2018/0216162.

**E. Microwells**

As described herein, one or more processes may be performed in a partition, which may be a well. The well may be a well of a plurality of wells of a substrate, such as a microwell of a microwell array or plate, or the well may be a microwell or microchamber of a device (e.g., microfluidic device) comprising a substrate. The well may be a well of a well array or plate, or the well may be a well or chamber of a device (e.g., fluidic device). Accordingly, the wells or microwells may assume an "open" configuration, in which the wells or microwells are exposed to the environment (e.g., contain an open surface) and are accessible on one planar face of the substrate, or the wells or microwells may assume a "closed" or "sealed" configuration, in which the microwells are not accessible on a planar face of the substrate. In some instances, the wells or microwells may be configured to toggle between "open" and "closed" configurations. For instance, an "open" microwell or set of microwells may be "closed" or "sealed" using a membrane (e.g., semipermeable membrane), an oil (e.g., fluorinated oil to cover an aqueous solution), or a lid, as described elsewhere herein. The wells or microwells may be initially provided in a "closed" or "sealed" configuration, wherein they are not accessible on a planar surface of the substrate without an external force. For instance, the "closed" or "sealed" configuration may comprise a substrate such as a sealing film or foil that is puncturable or pierceable by pipette tip(s). Suitable materials for the substrate include, without limitation, polyester, polypropylene, polyethylene, vinyl, and aluminum foil

The well may have a volume of less than 1 milliliter (mL). For instance, the well may be configured to hold a volume of at most 1000 microliters (μL), at most 100 μL, at most 10 μL, at most 1 μL, at most 100 nanoliters (nL), at most 10 nL, at most 1 nL, at most 100 picoliters (pL), at most 10 (pL), or less. The well may be configured to hold a volume of about 1000 μL, about 100 μL, about 10 μL, about 1 μL, about 100 nL, about 10 nL, about 1 nL, about 100 pL, about 10 pL, etc. The well may be configured to hold a volume of at least 10 pL, at least 100 pL, at least 1 nL, at least 10 nL, at least 100 nL, at least 1 μL, at least 10 μL, at least 100 μL, at least 1000 μL, or more. The well may be configured to hold a volume in a range of volumes listed herein, for example, from about 5 nL to about 20 nL, from about 1 nL to about 100 nL, from about 500 pL to about 100 μL, etc. The well may be of a plurality of wells that have varying volumes and may be configured to hold a volume appropriate to accommodate any of the partition volumes described herein.

In some instances, a microwell array or plate comprises a single variety of microwells. In some instances, a microwell array or plate comprises a variety of microwells. For instance, the microwell array or plate may comprise one or more types of microwells within a single microwell array or plate. The types of microwells may have different dimensions (e.g., length, width, diameter, depth, cross-sectional area, etc.), shapes (e.g., circular, triangular, square, rectangular, pentagonal, hexagonal, heptagonal, octagonal, nonagonal, decagonal, etc.), aspect ratios, or other physical characteristics. The microwell array or plate may comprise any number of different types of microwells. For example, the microwell array or plate may comprise 1, 2, 3, 4, 5, 6, 7, 8, 9, 10, 20, 30, 40, 50, 60, 70, 80, 90, 100, 200, 300, 400, 500, 600, 700, 800, 900, 1000 or more different types of microwells. A well may have any dimension (e.g., length, width, diameter, depth, cross-sectional area, volume, etc.), shape (e.g., circular, triangular, square, rectangular, pentagonal, hexagonal, heptagonal, octagonal, nonagonal, decagonal, other polygonal, etc.), aspect ratios, or other physical characteristics described herein with respect to any well.

In certain instances, the microwell array or plate comprises different types of microwells that are located adjacent to one another within the array or plate. For instance, a microwell with one set of dimensions may be located adjacent to and in contact with another microwell with a different set of dimensions. Similarly, microwells of different geometries may be placed adjacent to or in contact with one another. The adjacent microwells may be configured to hold different articles; for example, one microwell may be used to contain a cell, cell bead, or other sample (e.g., cellular components, nucleic acid molecules, etc.) while the adjacent microwell may be used to contain a microcapsule, droplet, bead, or other reagent. In some cases, the adjacent microwells may be configured to merge the contents held within, e.g., upon application of a stimulus, or spontaneously, upon contact of the articles in each microwell.

As is described elsewhere herein, a plurality of partitions may be used in the systems, compositions, and methods described herein. For example, any suitable number of partitions (e.g., wells or droplets) can be generated or otherwise provided. For example, in the case when wells are used, at least about 1,000 wells, at least about 5,000 wells, at least about 10,000 wells, at least about 50,000 wells, at least about 100,000 wells, at least about 500,000 wells, at least about 1,000,000 wells, at least about 5,000,000 wells at least about 10,000,000 wells, at least about 50,000,000 wells, at least about 100,000,000 wells, at least about 500,000,000 wells, at least about 1,000,000,000 wells, or more wells can be generated or otherwise provided. Moreover, the plurality of wells may comprise both unoccupied wells (e.g., empty wells) and occupied wells.

A well may comprise any of the reagents described herein, or combinations thereof. These reagents may include, for example, barcode molecules, enzymes, adapters, and combinations thereof. The reagents may be physically separated from a sample (e.g., a cell, cell bead, or cellular components, e.g., proteins, nucleic acid molecules, etc.) that is placed in the well. This physical separation may be accomplished by containing the reagents within, or coupling to, a microcapsule or bead that is placed within a well. The physical separation may also be accomplished by dispensing the reagents in the well and overlaying the reagents with a layer that is, for example, dissolvable, meltable, or permeable prior to introducing the polynucleotide sample into the well. This layer may be, for example, an oil, wax, membrane (e.g., semipermeable membrane), or the like. The well may be sealed at any point, for example, after addition of the microcapsule or bead, after addition of the reagents, or after addition of either of these components. The sealing of the well may be useful for a variety of purposes, including preventing escape of beads or loaded reagents from the well, permitting select delivery of certain reagents (e.g., via the use of a semi-permeable membrane), for storage of the well prior to or following further processing, etc.

A well may comprise free reagents and/or reagents encapsulated in, or otherwise coupled to or associated with, microcapsules, beads, or droplets. Any of the reagents described in this disclosure may be encapsulated in, or otherwise coupled to, a microcapsule, droplet, or bead, with any chemicals, particles, and elements suitable for sample processing reactions involving biomolecules, such as, but not limited to, nucleic acid molecules and proteins. For example, a bead or droplet used in a sample preparation reaction for DNA sequencing may comprise one or more of the following reagents: enzymes, restriction enzymes (e.g., multiple cutters), ligase, polymerase, fluorophores, reporter barcode sequences, adapters, buffers, nucleotides (e.g., dNTPs, ddNTPs) and the like.

Additional examples of reagents include, but are not limited to: buffers, acidic solution, basic solution, temperature-sensitive enzymes, pH-sensitive enzymes, light-sensitive enzymes, metals, metal ions, magnesium chloride, sodium chloride, manganese, aqueous buffer, mild buffer, ionic buffer, inhibitor, enzyme, protein, polynucleotide, antibodies, saccharides, lipid, oil, salt, ion, detergents, ionic detergents, non-ionic detergents, oligonucleotides, nucleotides, deoxyribonucleotide triphosphates (dNTPs), dideoxyribonucleotide triphosphates (ddNTPs), DNA, RNA, peptide polynucleotides, complementary DNA (cDNA), double stranded DNA (dsDNA), single stranded DNA (ssDNA), plasmid DNA, cosmid DNA, chromosomal DNA, genomic DNA, viral DNA, bacterial DNA, mtDNA (mitochondrial DNA), mRNA, rRNA, tRNA, nRNA, siRNA, snRNA, snoRNA, scaRNA, microRNA, dsRNA, ribozyme, riboswitch and viral RNA, polymerase, ligase, restriction enzymes, proteases, nucleases, protease inhibitors, nuclease inhibitors, chelating agents, reducing agents, oxidizing agents, fluorophores, probes, chromophores, dyes, organics, emulsifiers, surfactants, stabilizers, polymers, water, small molecules, pharmaceuticals, radioactive molecules, preservatives, antibiotics, aptamers, and pharmaceutical drug compounds. As described herein, one or more reagents in the well may be used to perform one or more reactions, including but not limited to: cell lysis, cell fixation, permeabilization, nucleic acid reactions, e.g., nucleic acid extension reactions, amplification, reverse transcription, transposase reactions (e.g., tagmentation), etc.

The wells may be provided as a part of a kit. For example, a kit may comprise instructions for use, a microwell array or device, and reagents (e.g., beads). The kit may comprise any useful reagents for performing the processes described herein, e.g., nucleic acid reactions, barcoding of nucleic acid molecules, sample processing (e.g., for cell lysis, fixation, and/or permeabilization).

In some cases, a well comprises a microcapsule, bead, or droplet that comprises a set of reagents that has a similar attribute (e.g., a set of enzymes, a set of minerals, a set of oligonucleotides, a mixture of different barcode molecules, a mixture of identical barcode molecules). In other cases, a microcapsule, bead, or droplet comprises a heterogeneous mixture of reagents. In some cases, the heterogeneous mixture of reagents can comprise all components necessary to perform a reaction. In some cases, such mixture can comprise all components necessary to perform a reaction, except for 1, 2, 3, 4, 5, or more components necessary to perform a reaction. In some cases, such additional components are contained within, or otherwise coupled to, a different microcapsule, droplet, or bead, or within a solution within a partition (e.g., microwell) of the system.

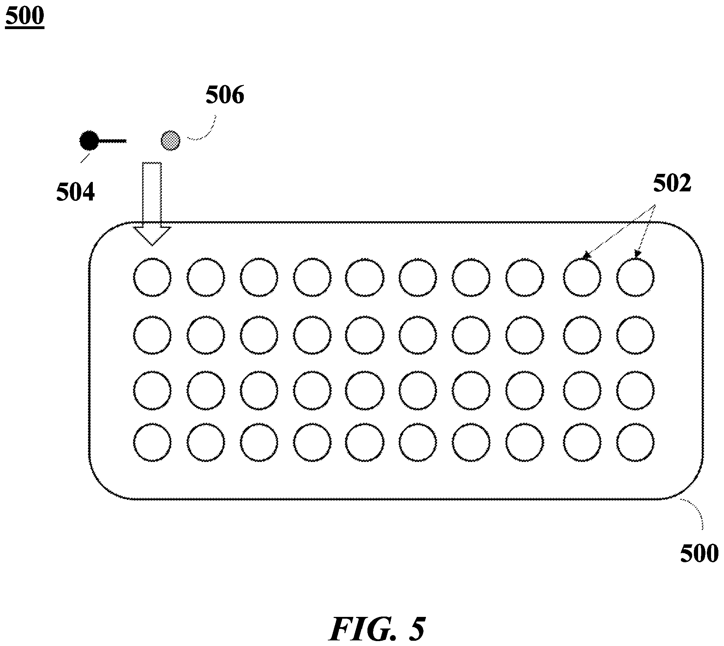

FIG. 5 schematically illustrates an example of a microwell array. The array can be contained within a substrate **500**. The substrate **500** comprises a plurality of wells **502**. The wells **1002** may be of any size or shape, and the spacing between the wells, the number of wells per substrate, as well as the density of the wells on the substrate **500** can be modified, depending on the particular application. In one such example application, a sample molecule **506,** which may comprise a cell or cellular components (e.g., nucleic acid molecules) is co-partitioned with a bead **504,** which may comprise a nucleic acid barcode molecule coupled thereto. The wells **502** may be loaded using gravity or other loading technique (e.g., centrifugation, liquid handler, acoustic loading, optoelectronic, etc.). In some instances, at least one of the wells **502** contains a single sample molecule **506** (e.g., cell) and a single bead **504.**

Reagents may be loaded into a well either sequentially or concurrently. In some cases, reagents are introduced to the device either before or after a particular operation. In some cases, reagents (which may be provided, in certain instances, in microcapsules, droplets, or beads) are introduced sequentially such that different reactions or operations occur at different steps. The reagents (or microcapsules, droplets, or beads) may also be loaded at operations interspersed with a reaction or operation step. For example, microcapsules (or droplets or beads) comprising reagents for fragmenting polynucleotides (e.g., restriction enzymes) and/or other enzymes (e.g., transposases, ligases, polymerases, etc.) may be loaded into the well or plurality of wells, followed by loading of microcapsules, droplets, or beads comprising reagents for attaching nucleic acid barcode molecules to a sample nucleic acid molecule. Reagents may be provided concurrently or sequentially with a sample, e.g., a cell or cellular components (e.g., organelles, proteins, nucleic acid molecules, carbohydrates, lipids, etc.). Accordingly, use of wells may be useful in performing multi-step operations or reactions.

As described elsewhere herein, the nucleic acid barcode molecules and other reagents may be contained within a microcapsule, bead, or droplet. These microcapsules, beads, or droplets may be loaded into a partition (e.g., a microwell) before, after, or concurrently with the loading of a cell, such that each cell is contacted with a different microcapsule, bead, or droplet. This technique may be used to attach a unique nucleic acid barcode molecule to nucleic acid molecules obtained from each cell. Alternatively or in addition to, the sample nucleic acid molecules may be attached to a support. For instance, the partition (e.g., microwell) may comprise a bead which has coupled thereto a plurality of nucleic acid barcode molecules. The sample nucleic acid molecules, or derivatives thereof, may couple or attach to the nucleic acid barcode molecules on the support. The resulting barcoded nucleic acid molecules may then be removed from the partition, and in some instances, pooled and sequenced. In such cases, the nucleic acid barcode sequences may be used to trace the origin of the sample nucleic acid molecule. For example, polynucleotides with identical barcodes may be determined to originate from the same cell or partition, while polynucleotides with different barcodes may be determined to originate from different cells or partitions.

The samples or reagents may be loaded in the wells or microwells using a variety of approaches. The samples (e.g., a cell, cell bead, or cellular component) or reagents (as described herein) may be loaded into the well or microwell using an external force, e.g., gravitational force, electrical force, magnetic force, or using mechanisms to drive the sample or reagents into the well, e.g., via pressure-driven flow, centrifugation, optoelectronics, acoustic loading, electrokinetic pumping, vacuum, capillary flow, etc. In certain cases, a fluid handling system may be used to load the samples or reagents into the well. The loading of the samples or reagents may follow a Poissonian distribution or a non-Poissonian distribution, e.g., super Poisson or sub-Poisson. The geometry, spacing between wells, density, and size of the microwells may be modified to accommodate a useful sample or reagent distribution; for instance, the size and spacing of the microwells may be adjusted such that the sample or reagents may be distributed in a super-Poissonian fashion.

In one particular non-limiting example, the microwell array or plate comprises pairs of microwells, in which each pair of microwells is configured to hold a droplet (e.g., comprising a single cell) and a single bead (such as those described herein, which may, in some instances, also be encapsulated in a droplet). The droplet and the bead (or droplet containing the bead) may be loaded simultaneously or sequentially, and the droplet and the bead may be merged, e.g., upon contact of the droplet and the bead, or upon application of a stimulus (e.g., external force, agitation, heat, light, magnetic or electric force, etc.). In some cases, the loading of the droplet and the bead is super-Poissonian. In other examples of pairs of microwells, the wells are configured to hold two droplets comprising different reagents and/or samples, which are merged upon contact or upon application of a stimulus. In such instances, the droplet of one microwell of the pair can comprise reagents that may react with an agent in the droplet of the other microwell of the pair. For instance, one droplet can comprise reagents that are configured to release the nucleic acid barcode molecules of a bead contained in another droplet, located in the adjacent microwell. Upon merging of the droplets, the nucleic acid barcode molecules may be released from the bead into the partition (e.g., the microwell or microwell pair that are in contact), and further processing may be performed (e.g., barcoding, nucleic acid reactions, etc.). In cases where intact or live cells are loaded in the microwells, one of the droplets may comprise lysis reagents for lysing the cell upon droplet merging.

A droplet or microcapsule may be partitioned into a well. The droplets may be selected or subjected to pre-processing prior to loading into a well. For instance, the droplets may comprise cells, and only certain droplets, such as those containing a single cell (or at least one cell), may be selected for use in loading of the wells. Such a pre-selection process may be useful in efficient loading of single cells, such as to obtain a non-Poissonian distribution, or to pre-filter cells for a selected characteristic prior to further partitioning in the wells. Additionally, the technique may be useful in obtaining or preventing cell doublet or multiplet formation prior to or during loading of the microwell.

In some instances, the wells can comprise nucleic acid barcode molecules attached thereto. The nucleic acid barcode molecules may be attached to a surface of the well (e.g., a wall of the well). The nucleic acid barcode molecule (e.g., a partition barcode sequence) of one well may differ from the nucleic acid barcode molecule of another well, which can permit identification of the contents contained with a single partition or well. In some cases, the nucleic acid barcode molecule can comprise a spatial barcode sequence that can identify a spatial coordinate of a well, such as within the well array or well plate. In some cases, the nucleic acid barcode molecule can comprise a unique molecular identifier for individual molecule identification. In some instances, the nucleic acid barcode molecules may be configured to attach to or capture a nucleic acid molecule within a sample or cell distributed in the well. For example, the nucleic acid barcode molecules may comprise a capture sequence that may be used to capture or hybridize to a nucleic acid molecule (e.g., RNA, DNA) within the sample. In some instances, the nucleic acid barcode molecules may be releasable from the microwell. For instance, the nucleic acid barcode molecules may comprise a chemical cross-linker which may be cleaved upon application of a stimulus (e.g., photo-, magnetic, chemical, biological, stimulus). The released nucleic acid barcode molecules, which may be hybridized or configured to hybridize to a sample nucleic acid molecule, may be collected and pooled for further processing, which can include nucleic acid processing (e.g., amplification, extension, reverse transcription, etc.) and/or characterization (e.g., sequencing). In such cases, the unique partition barcode sequences may be used to identify the cell or partition from which a nucleic acid molecule originated.

Characterization of samples within a well may be performed. Such characterization can include, in non-limiting examples, imaging of the sample (e.g., cell, cell bead, or cellular components) or derivatives thereof. Characterization techniques such as microscopy or imaging may be useful in measuring sample profiles in fixed spatial locations. For instance, when cells are partitioned, optionally with beads, imaging of each microwell and the contents contained therein may provide useful information on cell doublet formation (e.g., frequency, spatial locations, etc.), cell-bead pair efficiency, cell viability, cell size, cell morphology, expression level of a biomarker (e.g., a surface marker, a fluorescently labeled molecule therein, etc.), cell or bead loading rate, number of cell-bead pairs, etc. In some instances, imaging may be used to characterize live cells in the wells, including, but not limited to: dynamic live-cell tracking, cell-cell interactions (when two or more cells are co-partitioned), cell proliferation, etc. Alternatively or in addition to, imaging may be used to characterize a quantity of amplification products in the well.

In operation, a well may be loaded with a sample and reagents, simultaneously or sequentially. When cells or cell beads are loaded, the well may be subjected to washing, e.g., to remove excess cells from the well, microwell array, or plate. Similarly, washing may be performed to remove excess beads or other reagents from the well, microwell array, or plate. In the instances where live cells are used, the cells may be lysed in the individual partitions to release the intracellular components or cellular analytes. Alternatively, the cells may be fixed or permeabilized in the individual partitions. The intracellular components or cellular analytes may couple to a support, e.g., on a surface of the microwell, on a solid support (e.g., bead), or they may be collected for further downstream processing. For instance, after cell lysis, the intracellular components or cellular analytes may be transferred to individual droplets or other partitions for barcoding. Alternatively, or in addition to, the intracellular components or cellular analytes (e.g., nucleic acid molecules) may couple to a bead comprising a nucleic acid barcode molecule; subsequently, the bead may be collected and further processed, e.g., subjected to nucleic acid reaction such as reverse transcription, amplification, or extension, and the nucleic acid molecules thereon may be further characterized, e.g., via sequencing. Alternatively, or in addition to, the intracellular components or cellular analytes may be barcoded in the well (e.g., using a bead comprising nucleic acid barcode molecules that are releasable or on a surface of the microwell comprising nucleic acid barcode molecules). The barcoded nucleic acid molecules or analytes may be further processed in the well, or the barcoded nucleic acid molecules or analytes may be collected from the individual partitions and subjected to further processing outside the partition. Further processing can include nucleic acid processing (e.g., performing an amplification, extension) or characterization (e.g., fluorescence monitoring of amplified molecules, sequencing). At any convenient or useful step, the well (or microwell array or plate) may be sealed (e.g., using an oil, membrane, wax, etc.), which enables storage of the assay or selective introduction of additional reagents.

Once sealed, the well may be subjected to conditions for further processing of a biological particle (e.g., a cell, a cell bead or a nucleus) in the well. For instance, reagents in the well may allow further processing of the biological particle, e.g., lysis of the cell or nucleus, as further described herein. Alternatively, the well (or wells such as those of a well-based array) comprising the biological particle (e.g., cell, cell bead, or nucleus) may be subjected to freeze-thaw cycling to process the biological particle(s), e.g., lysis of a cell or nucleus. The well containing the biological particle (e.g., cell, cell bead, or nucleus) may be subjected to freezing temperatures (e.g., 0°C, below 0°C, -5°C, -10°C, -15°C, -20°C, -25°C, -30°C, -35°C, -40°C, -45°C, -50°C, -55°C, - 60°C, -65°C, -70°C, -80°C, or -85°C). Freezing may be performed in a suitable manner, e.g., sub-zero freezer or a dry ice/ethanol bath. Following an initial freezing, the well (or wells) comprising the biological particle(s) (e.g., cell(s), cell bead(s), nucleus or nuclei) may be subjected to freeze thaw cycles to lyse biological particle(s). In one embodiment, the initially frozen well (or wells) are thawed to a temperature above freezing (e.g., room temperature or 25°C). In another embodiment, the freezing is performed for less than 10 minutes (e.g., 5 minutes or 7 minutes) followed by thawing at room temperature for less than 10 minutes (e.g., 5 minutes or 7 minutes). This freeze-thaw cycle may be repeated a number of times, e.g., 2, 3, or 4 times, to obtain lysis of the biological particle(s) (e.g., cell(s), cell bead(s), nucleus, or nuclei) in the well (or wells). In one embodiment, the freezing, thawing and/or freeze/thaw cycling is performed in the absence of a lysis buffer. Additional disclosure related to freeze-thaw cycling is provided in WO2019165181A1.

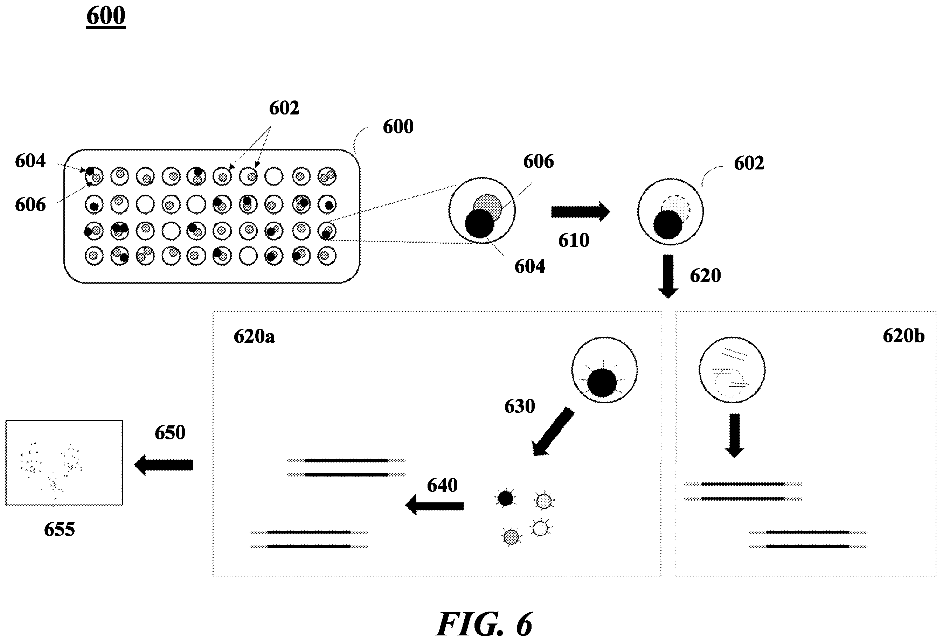

**FIG. 6** schematically shows an example workflow for processing nucleic acid molecules within a sample. A substrate **600** comprising a plurality of microwells **602** may be provided. A sample **606** which may comprise a cell, cell bead, cellular components or analytes (e.g., proteins and/or nucleic acid molecules) can be co-partitioned, in a plurality of microwells **602,** with a plurality of beads **604** comprising nucleic acid barcode molecules. During process **610,** the sample **606** may be processed within the partition. For instance, in the case of live cells, the cell may be subjected to conditions sufficient to lyse the cells and release the analytes contained therein. In process **620,** the bead **604** may be further processed. By way of example, processes **620a** and **620b** schematically illustrate different workflows, depending on the properties of the bead **604.**

In **620a,** the bead comprises nucleic acid barcode molecules that are attached thereto, and sample nucleic acid molecules (e.g., RNA, DNA) may attach, e.g., via hybridization of ligation, to the nucleic acid barcode molecules. Such attachment may occur on the bead. Such beads may be solid beads or magnetic beads to allow for removal from the microwell array. In process **630,** the beads **604** from multiple wells **602** may be collected and pooled. Further processing may be performed in process **640.** For example, one or more nucleic acid reactions may be performed, such as reverse transcription, nucleic acid extension, amplification, ligation, transposition, etc. In some embodiments, one or more reactions occur on the bead using the sample nucleic acid molecules captured on the bead via the nucleic acid barcode molecules on the bead, e.g., sample nucleic acid molecules hybridized to complementary sequences of the nucleic acid barcode molecules on the bead. In some instances, adapter sequences are ligated to the nucleic acid molecules, or derivatives thereof, as described elsewhere herein. For instance, sequencing primer sequences may be appended to each end of the nucleic acid molecule. In process **650,** further characterization, such as sequencing may be performed to generate sequencing reads. The sequencing reads may yield information on individual cells or populations of cells, which may be represented visually or graphically, e.g., in a plot **655.**

In **620b,** the bead comprises nucleic acid barcode molecules that are releasably attached thereto, as described below. The bead may degrade or otherwise release the nucleic acid barcode molecules into the well **602;** the nucleic acid barcode molecules may then be used to barcode nucleic acid molecules within the well **602.** Further processing may be performed either inside the partition or outside the partition. For example, one or more nucleic acid reactions may be performed, such as reverse transcription, nucleic acid extension, amplification, ligation, transposition, etc. In some instances, adapter sequences are ligated to the nucleic acid molecules, or derivatives thereof, as described elsewhere herein. For instance, sequencing primer sequences may be appended to each end of the nucleic acid molecule. In process **650,** further characterization, such as sequencing may be performed to generate sequencing reads. The sequencing reads may yield information on individual cells or populations of cells, which may be represented visually or graphically, e.g., in a plot **655.**

## VII. DETECTION AND ANALYSIS

### A. Generation of Barcoded Molecules

Barcodes as described herein may be used to associate one or more analytes (e.g., secreted molecules or cellular nucleotide sequences such as mRNAs) with a cell and/or a partition (e.g., droplet or well) upon analyzing the barcodes using e.g., sequencing reads generated using an Illumina sequencer.

The herein described methods may comprise use of multiple barcodes to analyze multiple analytes and cellular molecules such as antibodies and/or mRNA molecules. A barcode may be a nucleic acid sequence (barcode sequence). A first barcode may be different from a second barcode. For example, the nucleic acid sequence of a first barcode sequence may be different from that of a second barcode sequence. As described herein, nucleic acid molecules comprising a barcode sequence may be coupled to other molecules, polymer, or particles. For example, nucleic acid molecules comprising a barcode sequence may be coupled to MHC molecules (e.g.,

tetrameric MHC-peptide complexes comprising a barcode sequence), secondary binding agents (e.g., an antibody coupled to a barcode sequence), polymers (e.g., dextramers or polymers capable of forming hydrogels), and/or beads (e.g., beads in emulsion droplets or in wells of a microwell array). Use of one or more barcodes may allow measurement and analysis of one or more analytes of a cell and may allow associating the one or more analytes with the respective cell. This may be particularly advantageous when measuring and analyzing multiple analytes (e.g., secreted molecules and/or mRNAs) from a single cell or from a plurality of cells such as one or more cell populations (e.g., immune cells). In such cases, a first barcode may be used to measure a first analyte of a cell (e.g., a secreted molecule such as an antibody, e.g., reporter barcode as depicted in, described by FIG. 8 and FIG. 10A), and a second barcode, e.g., second reporter barcode, may be used to measure a second analyte of the cell (e.g., an mRNA molecule), and so forth. In these instances, a partition or cell specific barcode (e.g., attached to a bead, such as a gel bead) may be utilized to link the first barcode and the second barcode to attribute one or more analytes to a single cell. The analysis of an immune cell (e.g., a T cell, B cell, plasma cell, or dendritic cell), for example, may comprise measuring one or more signaling molecules (e.g., antibodies) that may be secreted upon stimulation of the cell (e.g., by using a stimulatory molecule), and one or more mRNA molecules that may be released from the cell upon cell lysis for analyzing, e.g., immune cell receptor gene segments (e.g., a V(D)J sequence of a T cell receptor (TCR)). See, e.g., U.S. Pat. Pub. 2018/0105808, for exemplary molecules and methods for analyzing V(D)J sequences of single cells using nucleic acid barcode molecules.

FIG. 3 illustrates an example of a barcode carrying bead. A nucleic acid molecule 302, such as an oligonucleotide, can be coupled to a bead 304 by a releasable linkage 306, such as, for example, a disulfide linker. The same bead 304 may be coupled (e.g., via releasable linkage) to one or more other nucleic acid molecules 318, 320. The nucleic acid molecule 302 may be or comprise a barcode. As noted elsewhere herein, the structure of the barcode may comprise a number of sequence elements. The nucleic acid molecule 302 may comprise a functional sequence 308 that may be used in subsequent processing. For example, the functional sequence 308 may include one or more of a sequencer specific flow cell attachment sequence (e.g., a P5 sequence for Illumina® sequencing systems) and a sequencing primer sequence (e.g., a R1 primer for Illumina® sequencing systems). The nucleic acid molecule 302 may comprise a barcode sequence 310 for use in barcoding the sample (e.g., DNA, RNA, protein, antibody, etc.). In some cases, the barcode sequence 310 can be bead-specific such that the barcode sequence 310 is common to all nucleic acid molecules (e.g., including nucleic acid molecule 302) coupled to the same bead 304. Alternatively or in addition, the barcode sequence 310 can be partition-specific such that the barcode sequence 310 is common to all nucleic acid molecules coupled to one or more beads that are partitioned into the same partition. The nucleic acid molecule 302 may comprise a specific priming sequence 312, such as an mRNA specific priming sequence (e.g., poly-T sequence), a targeted priming sequence, and/or a random priming sequence. The nucleic acid molecule 302 may comprise an anchoring sequence 314 to ensure that the specific priming sequence 312 hybridizes at the sequence end (e.g., of the mRNA). For example, the anchoring sequence 314 can include a random short sequence of nucleotides, such as a 1-mer, 2-mer, 3-mer or longer sequence, which can ensure that a poly-T segment is more likely to hybridize at the sequence end of the poly-A tail of the mRNA.

The nucleic acid molecule 302 may comprise a unique molecular identifying sequence 316 (e.g., unique molecular identifier (UMI)). In some cases, the unique molecular identifying sequence 316 may comprise from about 5 to about 8 nucleotides. Alternatively, the unique molecular identifying sequence 316 may compress less than about 5 or more than about 8 nucleotides. The unique molecular identifying sequence 316 may be a unique sequence that varies across individual nucleic acid molecules (e.g., 302, 318, 320, etc.) coupled to a single bead (e.g., bead 304). In some cases, the unique molecular identifying sequence 316 may be a random sequence (e.g., such as a random N-mer sequence). For example, the UMI may provide a unique identifier of the starting mRNA molecule that was captured, in order to allow quantitation of the number of original expressed RNA. As will be appreciated, although FIG. 3 shows three nucleic acid molecules 302, 318, 320 coupled to the surface of the bead 304, an individual bead may be coupled to any number of individual nucleic acid molecules, for example, from one to tens to hundreds of thousands or even millions of individual nucleic acid molecules. The respective barcodes for the individual nucleic acid molecules can comprise both common sequence segments or relatively common sequence segments (e.g., 308, 310, 312, etc.) and variable or unique sequence segments (e.g., 316) between different individual nucleic acid molecules coupled to the same bead.

A biological particle (e.g., cell, DNA, RNA, etc.) can be co-partitioned along with a barcode bearing bead 304. The barcode nucleic acid molecules 302, 318, 320 can be released from the bead 304 in the partition. By way of example, in the context of analyzing sample RNA, the poly-T segment (e.g., 312) of one of the released nucleic acid molecules (e.g., 302) can hybridize to the poly-A tail of a mRNA molecule. Reverse transcription may result in a cDNA transcript of the mRNA, but which transcript includes each of the sequence segments 308, 310, 316 of the nucleic acid molecule 302. Because the nucleic acid molecule 302 comprises an anchoring sequence 314, it will more likely hybridize to and prime reverse transcription at the sequence end of the poly-A tail of the mRNA. Within any given partition, all of the cDNA transcripts of the individual mRNA molecules may include a common barcode sequence segment **310**. However, the transcripts made from the different mRNA molecules within a given partition may vary at the unique molecular identifying sequence **312** segment (e.g., UMI segment). Beneficially, even following any subsequent amplification of the contents of a given partition, the number of different UMIs can be indicative of the quantity of mRNA originating from a given partition, and thus from the biological particle (e.g., cell). As noted above, the transcripts can be amplified, cleaned up and sequenced to identify the sequence of the cDNA transcript of the mRNA, as well as to sequence the barcode segment and the UMI segment. While a poly-T primer sequence is described, other targeted or random priming sequences may also be used in priming the reverse transcription reaction. Likewise, although described as releasing the barcode oligonucleotides into the partition, in some cases, the nucleic acid molecules bound to the bead (e.g., gel bead) may be used to hybridize and capture the mRNA on the solid phase of the bead, for example, in order to facilitate the separation of the RNA from other cell contents. In such cases, further processing may be performed, in the partitions or outside the partitions (e.g., in bulk). For instance, the RNA molecules on the beads may be subjected to reverse transcription or other nucleic acid processing, additional adapter sequences may be added to the barcode nucleic acid molecules, or other nucleic acid reactions (e.g., amplification, nucleic acid extension) may be performed. The beads or products thereof (e.g., barcoded nucleic acid molecules) may be collected from the partitions, and/or pooled together and subsequently subjected to clean up and further characterization (e.g., sequencing).

**FIG. 4** illustrates another example of a barcode carrying bead. A nucleic acid molecule **405,** such as an oligonucleotide, can be coupled to a bead **404** by a releasable linkage **406,** such as, for example, a disulfide linker. The nucleic acid molecule **405** may comprise a first capture sequence **460**. The same bead **404** may be coupled (e.g., via releasable linkage) to one or more other nucleic acid molecules **403, 407** comprising other capture sequences. The nucleic acid molecule **405** may be or comprise a barcode. As noted elsewhere herein, the structure of the barcode may comprise a number of sequence elements, such as a functional sequence **408** (e.g., flow cell attachment sequence, sequencing primer sequence, etc.), a barcode sequence **410** (e.g., bead-specific sequence common to bead, partition-specific sequence common to partition, etc.), and a unique molecular identifier **412** (e.g., unique sequence within different molecules attached to the bead), or partial sequences thereof. The capture sequence **460** may be configured to attach to a corresponding capture sequence **465**. In some instances, the corresponding capture sequence **465** may be coupled to another molecule that may be an analyte or an intermediary carrier. For example, as illustrated in **FIG. 4,** the corresponding capture sequence **465** is coupled to a guide RNA molecule **462** comprising a target sequence **464,** wherein the target sequence **464** is configured to attach to the analyte. Another oligonucleotide molecule **407** attached to the bead **404** comprises a second capture sequence **480** which is configured to attach to a second corresponding capture sequence **485**. As illustrated in **FIG. 4,** the second corresponding capture sequence **485** is coupled to an antibody **482**. In some cases, the antibody **482** may have binding specificity to an analyte (e.g., surface protein). Alternatively, the antibody **482** may not have binding specificity. Another oligonucleotide molecule **403** attached to the bead **404** comprises a third capture sequence **470** which is configured to attach to a second corresponding capture sequence **475**. As illustrated in **FIG. 4,** the third corresponding capture sequence **475** is coupled to a molecule **472**. The molecule **472** may or may not be configured to target an analyte. The other oligonucleotide molecules **403, 407** may comprise the other sequences (e.g., functional sequence, barcode sequence, UMI, etc.) described with respect to oligonucleotide molecule **405**. While a single oligonucleotide molecule comprising each capture sequence is illustrated in **FIG. 4,** it will be appreciated that, for each capture sequence, the bead may comprise a set of one or more oligonucleotide molecules each comprising the capture sequence. For example, the bead may comprise any number of sets of one or more different capture sequences. Alternatively or in addition, the bead **404** may comprise other capture sequences. Alternatively or in addition, the bead **404** may comprise fewer types of capture sequences (e.g., two capture sequences). Alternatively or in addition, the bead **404** may comprise oligonucleotide molecule(s) comprising a priming sequence, such as a specific priming sequence such as an mRNA specific priming sequence (e.g., poly-T sequence), a targeted priming sequence, and/or a random priming sequence, for example, to facilitate an assay for gene expression.

In one aspect, nucleic acid barcode molecules are provided as coupled to one or more beads as further described herein. In one embodiment, a bead may have coupled to it a plurality of nucleic acid barcode molecules (e.g., **302** in **FIG. 3** or 405 in **FIG. 4)** wherein a subset of the plurality of nucleic acid barcode molecules includes a barcode sequence (e.g., **310** in **FIG. 3** or 410 in **FIG. 4)**. In such a case, the barcode sequence in the subset of nucleic acid barcode molecules is a common barcode sequence.

The operations described herein may be performed at any useful or convenient step. For instance, the beads comprising nucleic acid barcode molecules may be introduced into a partition (e.g., well or droplet) prior to, during, or following introduction of a sample into the partition. The nucleic acid molecules of a sample may be subjected to barcoding, which may occur on the bead (in cases where the nucleic acid molecules remain coupled to the bead) or following release of the nucleic acid barcode molecules into the partition. In cases where the nucleic acid molecules from the sample remain attached to the bead, the beads from various partitions may be collected, pooled, and subjected to further processing (e.g., reverse transcription, adapter attachment, amplification, clean up, sequencing). In other instances, the processing may occur in the partition. For example, conditions sufficient for barcoding, adapter attachment, reverse transcription, or other nucleic acid processing operations may be provided in the partition and performed prior to clean up and sequencing.

An exemplary secreted analyte barcoding process utilizing capture agents or cell beads, is described in **FIGS. 8 and 10A,** respectively. In certain embodiments, a microfluidic channel structure (e.g., **FIGS. 1-2)** can be used to encapsulate a cell (e.g., a single cell) into a droplet. See, e.g., U.S. Pat. Pub. 2018/0216162 (now U.S. Pat. 10,428,326) and U.S. Pat. Pub. 2019/0100632 (now U.S. Pat. 10,590,244), for exemplary cell bead generation systems and methods. In certain embodiments, droplet comprises cell (e.g., a single cell, e.g., a single B cell or plasma cell) and an aqueous solution comprising polymer and/or crosslink precursors. To encapsulate a cell into a droplet, in some embodiments, a microfluidic channel structure comprises a first aqueous fluid that includes suspended biological particles (e.g., cells or nuclei) may be transported along a first channel segment and brought into contact with a second fluid that is immiscible with the first aqueous fluid to create discrete droplets of the first aqueous fluid. In some instances, the first aqueous fluid includes other reagents as described elsewhere herein, including polymer and/or crosslink precursors for cell bead generation. In other instances, a second aqueous fluid containing other reagents, including polymer and/or crosslink precursors, is brought into contact with the first aqueous fluid prior to or concurrent with droplet generation. In still other instances, two discrete droplets containing a first aqueous fluid (e.g., comprising a cell) and a second aqueous fluid (e.g., comprising reagents, such as polymer and/or crosslink precursors) can be merged into a coalesced droplet. In some instances, a discrete droplet generated includes at most, a single biological particle (such as a single cell or single nucleus).

Further, the droplet encapsulating, e.g., a cell, can be subjected to suitable conditions such that the polymer and/or crosslink precursors can be polymerized/crosslinked to generate extra-cellular matrix. Cell beads may be of uniform size or heterogeneous size. In some cases, the diameter of a cell bead may be at least about 10 nanometers (nm), 100 nm, 500 nm, 1 micrometer ($\mu$m), 5$\mu$m, 10$\mu$m, 20$\mu$m, 30$\mu$m, 40$\mu$m, 50$\mu$m, 60$\mu$m, 70$\mu$m, 80$\mu$m, 90$\mu$m, 100$\mu$m, 250$\mu$m, 500$\mu$m, 1mm, or greater. In some cases, a cell bead may have a diameter of less than about 10 nm, 100 nm, 500 nm, 1$\mu$m, 5$\mu$m, 10$\mu$m, 20$\mu$m, 30$\mu$m, 40$\mu$m, 50$\mu$m, 60$\mu$m, 70$\mu$m, 80$\mu$m, 90$\mu$m, 100$\mu$m, 250$\mu$m, 500$\mu$m, 1mm, or less. In some cases, a bead may have a diameter in the range of about 40-75$\mu$m, 30-75$\mu$m, 20-75$\mu$m, 40-85$\mu$m, 40-95$\mu$m, 20-100$\mu$m, 10-100$\mu$m, 1-100$\mu$m, 20-250$\mu$m, or 20-500$\mu$m.

In some embodiments, the bead may comprise covalent or ionic bonds between polymeric precursors (e.g., monomers, oligomers, linear polymers), oligonucleotides, primers, and other entities. In some cases, the covalent bonds comprise carbon-carbon bonds or thioether bonds. In some cases, a bead may comprise an acrydite moiety or click chemistry moeity, which in certain aspects may be used to attach one or more first capture agents (e.g., capture agents comprising antibodies). See, e.g., U.S. Pat. Pub. 2019/0100632 (now U.S. Pat. 10,590,244), for exemplary cell bead polymers and functionalization strategies. In some instances, a chemical conjugation method is utilized to attach molecules (e.g., capture agent) to the cell bead hydrogel matrix, as described herein.

In some embodiments, after the formation of cell beads comprising antibody-specific capture agents, stimulatory agents (such as antigens) are used to stimulate the cells (e.g., T cells) to secrete antibodies or other analytes of interest. In certain embodiments, the antigens are coupled to barcoded peptide-major histocompatibility complex (MHC) molecules (e.g., MHC multimers such as tetramers or dextramers). Any suitable concentrations of antigen can be used to allow sufficient secretion of antibodies from the cell beads. Moreover, sufficient incubation time is employed to ensure antibody secretion from the cell beads. After antibodies or other analytes of interest are secreted from the cells, a plurality of first capture agents may couple to (e.g., covalently or non-covalently bind to) the secreted antibodies or other analytes. As a result, the capture agent may form a complex with a "captured" or bound antibody or analyte.

Subsequently, a reporter agent that, in some instances, comprises an analyte (e.g., antibody) specific antigen comprising a nucleic acid molecule comprising a first barcode sequence (i.e., reporter barcode sequence) may couple to (e.g., covalently or non-covalently bind to) the secreted antibody or other analyte coupled to the capture agent. Further processing (e.g., coupling a cell and/or partition barcode and sequencing) of the reporter agent and/or nucleic acid molecule may allow for identification of the presence and/or amount of the secreted analyte.

Furthermore, the extra-cellular matrix (ECM) of the cell beads can be partially digested by enzymes. In some cases, the enzyme may be collagenase, which assists in breaking the peptide bonds in collagen. In other cases, the enzyme may be dispase, which is a protease which cleaves fibronectin, collagen IV, and to a lesser extent collagen I. In other cases, a suitable enzyme that can partially digest the ECM may be used here. Additionally, ECM may comprise collagen, laminin, fibronectin, etc. The cell beads may be partitioned into emulsion droplets as described herein.

The herein disclosed methods and systems may further comprise partitioning cell beads into a plurality of partitions with a plurality of nucleic acid molecule comprising a cell-specific barcode sequence (see, e.g., the barcode molecules described in **FIG. 3).** In some instances, the cell specific barcodes (or partition-specific barcodes, as described description of **310)** are attached to a bead, such a gel bead. The cellular barcodes may be releasably attached to the bead as described elsewhere herein. Cell beads and cellular barcodes (e.g., attached to a bead, such as a gel bead) may be partitioned in a droplet or a well. The droplet may be an emulsion droplet and may comprise a cell bead. The emulsion droplet may be formed or generated as described elsewhere herein, e.g., by contacting two phases (e.g., a first and a second phase) that are immiscible (e.g., an aqueous phase and an oil). The hydrogel matrix forming the cell bead may be dissolved, thus releasing the analyte conjugate comprising the analyte (e.g., a cytokine), the capture agent and the reporter agent comprising the first barcode. The cell bead may be dissolved using one or more stimuli such as change in pH, temperature, or ion concentration within the partition. In some instances, the cell is lysed, releasing cellular molecules such as nucleic acid molecules (e.g., mRNAs). The cell may be lysed prior to partitioning and barcoding of analyte or lysed in the partition. The cellular mRNA molecules may be reverse transcribed using nucleic acid molecules comprising a second barcode sequence, thereby attaching the second barcode to the reversed transcribed nucleic acid molecules (and e.g., associating the analytes with the cell).

Alternatively, cellular mRNA molecules may be first reverse transcribed into cDNA (e.g., using a poly-T containing primer) and the second, cell-specific barcode sequence attached (e.g., to the 5' end of an mRNA/cDNA molecule) using, e.g., a template switching reaction as described elsewhere herein. See, e.g., U.S. Pat. Pub. 2018/0105808, for exemplary molecules and methods for analyzing and barcoding mRNA of single cells using template switching reactions and template switching oligonucleotides. In some instances, cellular barcodes (e.g., cell-specific barcodes) are released from, e.g., a bead (such as a gel bead) into the partition as described elsewhere herein (e.g., using a stimulus, such as a reducing agent). Similarly, the nucleic acid molecules comprising the first, analyte-specific barcode sequence (e.g., **816)** can be utilized to generate a molecule comprising the first analyte specific barcode and the second, cell-specific barcode. The nucleic acid molecules (e.g., **816)** attached to an analyte specific binding agent, i.e. reporter agent (e.g., **814),** may comprise one or more functional sequences in addition to the analyte-specific barcode sequence. For example, the nucleic acid molecules (e.g., **816)** attached to an analyte specific binding agent (e.g., **814)** may comprise one or more of a unique molecular identifier (UMI), a primer sequence or primer binding sequence (e.g., a sequencing primer sequence (or partial sequencing primer sequence) such as an R1 and/or R2 sequence), a sequence configured to attach to the flow cell of a sequencer (e.g., P5 and/or P7), or sequence complementary to a sequence on a nucleic acid barcode molecule (e.g., attached to a bead, such as those described in **FIG. 3).** Accordingly, barcoded molecules (e.g., comprising an analyte specific and cell specific barcode), may also comprise these functional sequences.

## B. Multiplexing Methods

Once the contents of the cells are released into their respective partitions, the nucleic acids contained therein may be further processed within the partitions. In accordance with the methods and systems described herein, the nucleic acid contents of individual cells can be provided with unique identifiers such that, upon characterization of those nucleic acids they may be attributed as having been derived from the same cell or cells (or nucleus/nuclei or cell bead(s)). The ability to attribute characteristics to individual cells or groups of cells is provided by the assignment of unique identifiers specifically to an individual cell or groups of cells. Unique identifiers, e.g., in the form of nucleic acid barcodes can be assigned or associated with individual cells or populations of cells, in order to tag or label the cell's components (and as a result, its characteristics) with the unique identifiers. These unique identifiers can then be used to attribute the cell's components and characteristics to an individual cell or group of cells. In some aspects, this is carried out by co-partitioning the individual cells or groups of cells with the unique identifiers. In some aspects, the unique identifiers are provided in the form of oligonucleotides (also referred to herein as anchor oligonucleotides) that comprise nucleic acid barcode sequences that may be attached to or otherwise associated with the nucleic acid contents of individual cells, or to other components of the cells, and particularly to fragments of those nucleic acids. The oligonucleotides may be partitioned such that as between oligonucleotides in a given partition, the nucleic acid barcode sequences contained therein are the same, but as between different partitions, the oligonucleotides can, and do have differing barcode sequences, or at least represent a large number of different barcode sequences across all of the partitions in a given analysis. In some aspects, only one nucleic acid barcode sequence can be associated with a given partition, although in some cases, two or more different barcode sequences may be present.

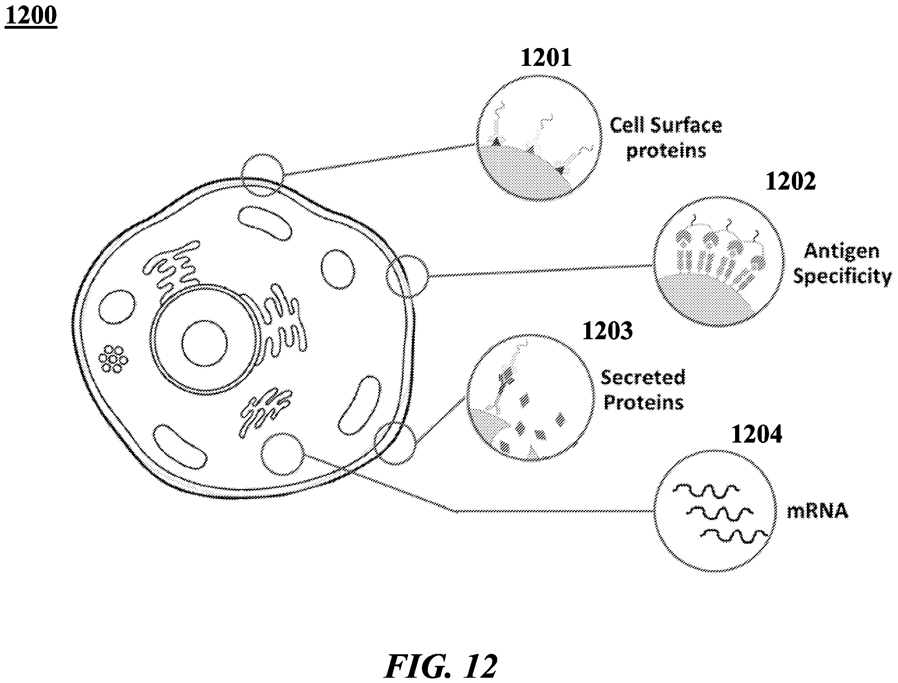

Upon completion of the one or more barcoding, reverse transcription, and/or nucleic acid processing steps (e.g., depending on how many different analytes of a cell are being barcoded), the contents of the partitions (e.g., droplets or wells) may be pooled and the nucleic acid molecules subjected to further bulk processing and sequencing. Thus, the presently described methods and systems allow the association of multiple analytes (e.g., secreted antibodies or antigen binding fragment thereof) to a single cell (e.g., labelled B cell or plasma cell), thereby enabling the measurement, analysis, and/or characterization of a plurality of cells at the single cell level. As described herein, the plurality of cells (e.g., one or more cell populations such as populations of immune cells, i.e., B cells or plasma cells) may be analyzed and characterized in an efficient and simultaneous manner. The methods disclosed herein not only allow analysis of cellular molecules after lysis of the cell, but also allow analysis of molecules that may be secreted by the cell (e.g., an immune cell such as a T cell, B cell, plasma cell, or dendritic cell) such as secreted proteins, antibodies, antigen binding fragments thereof, or cytokines, (see e.g., operation **1203**) in addition and/or simultaneous to the analysis of cellular nucleic acid molecules (e.g., mRNAs for analyzing BCR or antibody sequences, see e.g., operations **1204),** cell surface proteins (e.g., receptors or ligands, etc., see e.g., operation **1201),** antigen presentation (e.g., antigen presentation by B cells), antigen specificity (see e.g., operation **1202)** of a single cell, etc., e.g., as shown in **FIG. 12.** Exemplary methods and compositions for single cell analysis of multiple analytes are disclosed in WO2019/157529. In some embodiments, disclosed herein is a method comprising analyzing, e.g., simultaneously, in parallel, or sequentially, a secreted antibody (e.g., an antibody recognizing an antigen from a pathogen, or an anti-ID antibody such as anti-antibody drug antibody) or antigen binding fragment thereof from a cell of the B-lineage (e.g., a plasma cell) and one or more other analytes including cytokine molecules secreted by the B-lineage cell.

In embodiments where intracellular analytes (e.g., mRNA) are processed in parallel to secreted antibody or antigen binding fragment thereof, the cells contained in a partition (e.g., a droplet or a well) and cell beads contained in a partition (e.g., a droplet or a well), after cell beads are optionally dissolved (e.g., by dissolving the polymer matrix), are contacted with lysis reagents in order to release the contents of cells or viruses associated with the cell bead. In some cases, the lysis agents can be contacted with a cell bead suspension in bulk after cell bead formation. Examples of lysis agents include bioactive reagents, such as lysis enzymes that are used for lysis of different cell types, e.g., gram positive or negative bacteria, plants, yeast, mammalian, etc., such as lysozymes, achromopeptidase, lysostaphin, labiase, kitalase, lyticase, and a variety of other lysis enzymes available from, e.g., Sigma-Aldrich, Inc. (St Louis, MO), a surfactant based lysis solution (e.g., TritonX-100, Tween 20, sodium dodecyl sulfate (SDS)) for example, as well as other commercially available lysis enzymes. Electroporation, thermal, acoustic or mechanical cellular disruption may also be used in certain cases. In some cases, the cell bead matrix can be configured to give rise to a pore size that is sufficiently small to retain nucleic acid fragments of a particular size, following cellular disruption. In other instances, the cell bead matrix may be functionalized (e.g., covalently bound) with nucleic acid molecules (e.g., containing a poly-T sequence) configured to capture released analytes (e.g., mRNA, which optionally can be processed into cDNA prior to partitioning).

Other reagents can also be contacted with the cells, including, for example, DNase and RNase inactivating agents or inhibitors, such as proteinase K, chelating agents, such as EDTA, and other reagents employed in removing or otherwise reducing negative activity or impact of different cell lysate components on subsequent processing of nucleic acids. In addition, in the case of encapsulated cell beads, the cell beads may be exposed to an appropriate stimulus to release the cell beads or their contents into, e.g., a partition. For example, in some cases, a chemical stimulus may be co-partitioned along with an encapsulated cell bead to allow for the degradation of the microcapsule and release of the cell or its contents into the larger partition. In some cases, this stimulus may be the same as the stimulus described elsewhere herein for release of oligonucleotides from their respective microcapsule (e.g., bead). In alternative aspects, this may be a different and non-overlapping stimulus, in order to allow an encapsulated cell bead release its contents into a partition at a different time from the release of oligonucleotides into the same partition.

After the releasing of cellular macromolecular constituent, the cellular mRNA molecules are subject to a reverse transcription reaction with other types of reverse transcription primers such that cDNA is generated from the mRNAs. In some cases, simultaneously, a partition-specific (e.g., cell-specific, such as those described in **FIG. 3)** barcode molecule is attached during cDNA generation. In certain embodiments, the partition-specific (e.g., cell-specific) barcode sequence is a DNA oligonucleotide. The partition-specific (e.g., cell-specific) barcode sequence may be a second barcode sequence that is different from a first barcode sequence (e.g., analyte-specific barcode) attached to or coupled to a second binding reporter agent used to barcode an analyte that is secreted from a cell (e.g., an immune cell such as a B cell).

Alternatively, cellular mRNA molecules may be first reverse transcribed into cDNA (e.g., using a poly-T containing primer) and the second, cell-specific barcode sequence attached (e.g., to the 5' end of an mRNA/cDNA molecule) using, e.g., a template switching reaction as described elsewhere herein. See, e.g., U.S. Pat. Pub. 2018/0105808, for exemplary molecules and methods for analyzing and barcoding mRNA of single cells using template switching reactions and template switching oligonucleotides.

In certain embodiments, nucleic acid molecules, e.g., the partition-specific (e.g., cell-specific) barcode molecules (e.g., oligonucleotides) are releasable from the beads upon the application of a particular stimulus to the beads, as described elsewhere herein. In some cases, the stimulus may be a photo-stimulus, e.g., through cleavage of a photo-labile linkage that releases the nucleic acid molecules. In other cases, a thermal stimulus may be used, where elevation of the temperature of the beads environment will result in cleavage of a linkage or other release of the nucleic acid molecules from the beads. In still other cases, a chemical stimulus can be used that cleaves a linkage of the nucleic acid molecules to the beads, or otherwise results in release of the nucleic acid molecules from the beads. In one case, such compositions include the polyacrylamide matrices described above for encapsulation of biological particles, and may be degraded for release of the attached nucleic acid molecules through exposure to a reducing agent, such as DTT.

In some embodiments, barcode molecules attached to reporter agent (e.g., an analyte specific polypeptide, such as an antigen) bound to an analyte may be released (e.g., through a releasable linkage/labile bound as described elsewhere herein). Similarly, in some embodiments, barcode molecules attached to MHC multimers, attached to a protein or polypeptide (e.g., an antigen or prospective antigen), and/or attached to cell surface protein specific antibodies may also be released (e.g., through a releasable linkage/labile bound as described elsewhere herein). Partition-specific (e.g., droplet-specific or secondary) barcode sequences may be attached to any or all of these released barcode molecules (or derivatives thereof). For example, a barcoded bead comprising a partition specific barcode sequence may be used in a partition such as a droplet to couple to a barcode (e.g., a partition-specific barcode) to one or more analytes (e.g., secreted cytokines, secreted antibodies, mRNAs, etc.) of a single cell, thereby associating said one or more analytes with the single cell. These barcodes are used as cell and/or partition-specific identifiers for RNA, DNA, proteins, secreted antibodies or antigen binding fragments thereof, and/or antigens that used to stimulate the cells. The assignment of unique barcodes specifically to an individual biological particle or groups of biological particles can attribute characteristics to individual biological particles or groups of biological particles. Unique identifiers, i.e., in the form of nucleic acid barcodes, are assigned or associated with individual biological particles or populations of biological particles, in order to tag or label the biological particle's macromolecular components (and as a result, its characteristics) with the unique identifiers. These unique identifiers, i.e., barcodes, can then be used to attribute the biological particle's components and characteristics to an individual biological particle or group of biological particles. Furthermore, as described elsewhere herein, in addition to cell and/or partition specific barcodes, unique molecular identifiers (UMIs) can also be added to cellular analytes (e.g., mRNA molecules) and reporter nucleic acid molecules (e.g., attached to binding agents, or MHC molecules/multimers and/or antibodies, such as cell surface antibodies) to provide a unique identifier for quantitation of individual molecules.

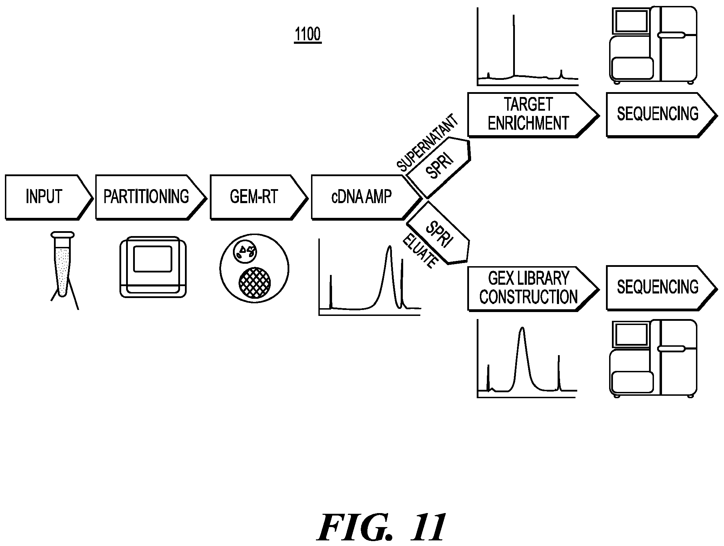

Barcoded partition contents are pooled into a bulk solution and further processed as described elsewhere herein to generate a sequencing library (see, e.g. **FIG. 11)**. For example, following partitioning into Gel Bead-in-Emulsion (GEM), the cell in each droplet may be lysed and reverse transcription (GEM-RT) may be performed. After cDNA amplification, the supernatant and eluate are subjected to Solid Phase Reversible Immobilization (SPRI) followed by enrichment and library construction, respectively. Referring to **FIG. 12,** information regarding secreted antibodies, cytokines, as well as other analytes, such as mRNAs, cell surface proteins, and antigen binding specificity can all be analyzed and attributed to the same cell using the cell-specific barcode (see, e.g., **FIG. 3)**.

The present disclosure provides methods and systems for multiplexing, and otherwise increasing throughput of samples for analysis. For example, a single or integrated process workflow may permit the processing, identification, and/or analysis of more or multiple analytes, more or multiple types of analytes, and/or more or multiple types of analyte characterizations. For example, in the methods and systems described herein, one or more labelling agents capable of binding to or otherwise coupling to one or more cells or cell features may be used to characterize cells and/or cell features. In some instances, cell features include cell surface features. Cell surface features may include, but are not limited to, a receptor, an antigen, a surface protein, a transmembrane protein, a cluster of differentiation protein, a protein channel, a protein pump, a carrier protein, a phospholipid, a glycoprotein, a glycolipid, a cell-cell interaction protein complex, an antigen-presenting complex, a major histocompatibility complex, an engineered T-cell receptor, a T-cell receptor, a B-cell receptor, a chimeric antigen receptor, a gap junction, an adherens junction, or any combination thereof. In some instances, cell features may include intracellular analytes, such as proteins, protein modifications (e.g., phosphorylation status or other post-translational modifications), nuclear proteins, nuclear membrane proteins, or any combination thereof. A labelling agent may include, but is not limited to, a protein, a peptide, an antibody (or an epitope binding fragment thereof), a lipophilic moiety (such as cholesterol), a cell surface receptor binding molecule, a receptor ligand, a small molecule, a bi-specific antibody, a bi-specific T-cell engager, a T-cell receptor engager, a B-cell receptor engager, a pro-body, an aptamer, a monobody, an affimer, a darpin, and a protein scaffold, or any combination thereof. The labelling agents can include (e.g., are attached to) a reporter oligonucleotide that is indicative of the cell surface feature to which the binding group binds. For example, the reporter oligonucleotide may comprise a barcode sequence that permits identification of the labelling agent. For example, a labelling agent that is specific to one type of cell feature (e.g., a first cell surface feature) may have a first reporter oligonucleotide coupled thereto, while a labelling agent that is specific to a different cell feature (e.g., a second cell surface feature) may have a different reporter oligonucleotide coupled thereto. For a description of exemplary labelling agents, reporter oligonucleotides, and methods of use, see, e.g., U.S. Pat. 10,550,429; U.S. Pat. Pub. 20190177800; and U.S. Pat.

Pub. 20190367969.

In a particular example, a library of potential cell feature labelling agents may be provided, where the respective cell feature labelling agents are associated with nucleic acid reporter molecules, such that a different reporter oligonucleotide sequence is associated with each labelling agent capable of binding to a specific cell feature. In other aspects, different members of the library may be characterized by the presence of a different oligonucleotide sequence label. For example, an antibody capable of binding to a first protein may have associated with it a first reporter oligonucleotide sequence, while an antibody capable of binding to a second protein may have a different reporter oligonucleotide sequence associated with it. The presence of the particular oligonucleotide sequence may be indicative of the presence of a particular antibody or cell feature which may be recognized or bound by the particular antibody.

Labelling agents capable of binding to or otherwise coupling to one or more cells may be used to characterize a cell as belonging to a particular set of cells. For example, labeling agents may be used to label a sample of cells or a group of cells. In this way, a group of cells may be labeled as different from another group of cells. In an example, a first group of cells may originate from a first sample and a second group of cells may originate from a second sample. Labelling agents may allow the first group and second group to have a different labeling agent (or reporter oligonucleotide associated with the labeling agent). This may, for example, facilitate multiplexing, where cells of the first group and cells of the second group may be labeled separately and then pooled together for downstream analysis. The downstream detection of a label may indicate analytes as belonging to a particular group.

For example, a reporter oligonucleotide may be linked to an antibody or an epitope binding fragment thereof, and labeling a cell may comprise subjecting the antibody-linked to the reporter oligonucleotide, e.g., antibody-linked barcode molecule or the epitope binding fragment-linked to the reporter oligonucleotide, e.g., epitope binding fragment-barcode molecule to conditions suitable for binding the antibody to a molecule present on a surface of the cell. The binding affinity between the antibody or the epitope binding fragment thereof and the molecule present on the surface may be within a desired range to ensure that the antibody or the epitope binding fragment thereof remains bound to the molecule. For example, the binding affinity may be within a desired range to ensure that the antibody or the epitope binding fragment thereof remains bound to the molecule during various sample processing steps, such as partitioning and/or nucleic acid amplification or extension. A dissociation constant (Kd) between the antibody or an epitope binding fragment thereof and the molecule to which it binds may be less than about 100 $\mu$M, 90 $\mu$M, 80 $\mu$M, 70 $\mu$M, 60 $\mu$M, 50 $\mu$M, 40 $\mu$M, 30 $\mu$M, 20 $\mu$M, 10 $\mu$M, 9 $\mu$M, 8 $\mu$M, 7 $\mu$M, 6 $\mu$M, 5 $\mu$M, 4 $\mu$M, 3 $\mu$M, 2 $\mu$M, 1 $\mu$M, 900 nM, 800 nM, 700 nM, 600 nM, 500 nM, 400 nM, 300 nM, 200 nM, 100 nM, 90 nM, 80 nM, 70 nM, 60 nM, 50 nM, 40 nM, 30 nM, 20 nM, 10 nM, 9 nM, 8 nM, 7 nM, 6 nM, 5 nM, 4 nM, 3 nM, 2 nM, 1 nM, 900 pM, 800 pM, 700 pM, 600 pM, 500 pM, 400 pM, 300 pM, 200 pM, 100 pM, 90 pM, 80 pM, 70 pM, 60 pM, 50 pM, 40 pM, 30 pM, 20 pM, 10 pM, 9 pM, 8 pM, 7 pM, 6 pM, 5 pM, 4 pM, 3 pM, 2 pM, or 1 pM. For example, the dissociation constant may be less than about 10 $\mu$M.

In another example, a reporter oligonucleotide may be coupled to a cell-penetrating peptide (CPP), and labeling cells may comprise delivering the CPP coupled reporter oligonucleotide into a biological particle (or an analyte carrier), such as a cell, nucleus, or cell bead. Labeling biological particles (or analyte carriers) may comprise delivering the CPP conjugated oligonucleotide into a cell and/or cell bead by the cell-penetrating peptide. A CPP that can be used in the methods provided herein can comprise at least one non-functional cysteine residue, which may be either free or derivatized to form a disulfide link with an oligonucleotide that has been modified for such linkage. Non-limiting examples of CPPs that can be used in embodiments herein include penetratin, transportan, plsl, TAT(48-60), pVEC, MTS, and MAP. Cell-penetrating peptides useful in the methods provided herein can have the capability of inducing cell penetration for at least about 30%, 40%, 50%, 60%, 70%, 80%, 90%, 95%, 96%, 97%, 98%, 99%, or 100% of cells of a cell population. The CPP may be an arginine-rich peptide transporter. The CPP may be Penetratin or the Tat peptide. In another example, a reporter oligonucleotide may be coupled to a fluorophore or dye, and labeling cells may comprise subjecting the fluorophore coupled to the reporter oligonucleotide, e.g., fluorophore-linked barcode molecule to conditions suitable for binding the fluorophore to the surface of the cell.

In some instances, fluorophores can interact strongly with lipid bilayers and labeling cells may comprise subjecting the fluorophore coupled to the reporter oligonucleotide, e.g., fluorophore linked barcode molecule to conditions such that the fluorophore binds to or is inserted into a membrane of the cell. In some cases, the fluorophore is a water-soluble, organic fluorophore. In some instances, the fluorophore is Alexa 532 maleimide, tetramethylrhodamine-5-maleimide (TMR maleimide), BODIPY-TMR maleimide, Sulfo-Cy3 maleimide, Alexa 546 carboxylic acid/succinimidyl ester, Atto 550 maleimide, Cy3 carboxylic acid/succinimidyl ester, Cy3B carboxylic acid/succinimidyl ester, Atto 565 biotin, Sulforhodamine B, Alexa 594 maleimide, Texas Red maleimide, Alexa 633 maleimide, Abberior STAR 635P azide, Atto 647N maleimide, Atto 647 SE, or Sulfo-Cy5 maleimide. See, e.g., Hughes L D, et al. PLoS One. 2014 Feb. 4; 9(2):e87649, for a description of organic fluorophores.

A reporter oligonucleotide may be coupled to a lipophilic molecule, and labeling cells may comprise delivering the reporter oligonucleotide coupled to the lipophilic molecule to a membrane of a cell or a nuclear membrane by the lipophilic molecule. Lipophilic molecules can associate with and/or insert into lipid membranes such as cell membranes and nuclear membranes. In some cases, the insertion can be reversible. In some cases, the association between the lipophilic molecule and the cell or nuclear membrane may be such that the membrane retains the lipophilic molecule (e.g., and associated components, such as nucleic acid barcode molecules, thereof) during subsequent processing (e.g., partitioning, cell permeabilization, amplification, pooling, etc.). The reporter oligonucleotide may enter into the intracellular space and/or a cell nucleus. In one embodiment, a reporter oligonucleotide coupled to a lipophilic molecule will remain associated with and/or inserted into lipid membrane (as described herein) via the lipophilic molecule until lysis of the cell occurs, e.g., inside a partition.

A reporter oligonucleotide may be part of a nucleic acid molecule comprising any number of functional sequences, as described elsewhere herein, such as a target capture sequence, a random primer sequence, and the like, and coupled to another nucleic acid molecule that is, or is derived from, the analyte.

Prior to partitioning, the cells may be incubated with the library of labelling agents, that may be labelling agents to a broad panel of different cell features, e.g., receptors, proteins, etc., and which include their associated reporter oligonucleotides. Unbound labelling agents may be washed from the cells, and the cells may then be co-partitioned (e.g., into droplets or wells) along with partition-specific barcode oligonucleotides (e.g., attached to a support, such as a bead or gel bead) as described elsewhere herein. As a result, the partitions may include the cell or cells, as well as the bound labelling agents and their known, associated reporter oligonucleotides.

In other instances, e.g., to facilitate sample multiplexing, a labelling agent that is specific to a particular cell feature may have a first plurality of the labelling agent (e.g., an antibody or lipophilic moiety) coupled to a first reporter oligonucleotide and a second plurality of the labelling agent coupled to a second reporter oligonucleotide. For example, the first plurality of the labeling agent and second plurality of the labeling agent may interact with different cells, cell populations or samples, allowing a particular report oligonucleotide to indicate a particular cell population (or cell or sample) and cell feature. In this way, different samples or groups can be independently processed and subsequently combined together for pooled analysis (e.g., partition-based barcoding as described elsewhere herein). See, e.g., U.S. Pat. Pub. 20190323088.

As described elsewhere herein, libraries of labelling agents may be associated with a particular cell feature as well as be used to identify analytes as originating from a particular cell population, or sample. Cell populations may be incubated with a plurality of libraries such that a cell or cells comprise multiple labelling agents.

For example, a cell may comprise coupled thereto a lipophilic labeling agent and an antibody. The lipophilic labeling agent may indicate that the cell is a member of a particular cell sample, whereas the antibody may indicate that the cell comprises a particular analyte. In this manner, the reporter oligonucleotides and labelling agents may allow multi-analyte, multiplexed analyses to be performed.

In some instances, these reporter oligonucleotides may comprise nucleic acid barcode sequences that permit identification of the labelling agent which the reporter oligonucleotide is coupled to. The use of oligonucleotides as the reporter may provide advantages of being able to generate significant diversity in terms of sequence, while also being readily attachable to most biomolecules, e.g., antibodies, etc., as well as being readily detected, e.g., using sequencing or array technologies.

Attachment (coupling) of the reporter oligonucleotides to the labelling agents may be achieved through any of a variety of direct or indirect, covalent or non-covalent associations or attachments. For example, oligonucleotides may be covalently attached to a portion of a labelling agent (such a protein, e.g., an antibody or antibody fragment) using chemical conjugation techniques (e.g., Lightning-Link® antibody labelling kits available from Innova Biosciences), as well as other non-covalent attachment mechanisms, e.g., using biotinylated antibodies and oligonucleotides (or beads that include one or more biotinylated linker, coupled to oligonucleotides) with an avidin or streptavidin linker. Antibody and oligonucleotide biotinylation techniques are available. See, e.g., Fang, et al., "Fluoride-Cleavable Biotinylation Phosphoramidite for 5'-end-Labelling and Affinity Purification of Synthetic Oligonucleotides," Nucleic Acids Res. Jan. 15, 2003; 3 1(2):708-7] 5. Likewise, protein and peptide biotinylation techniques have been developed and are readily available. See, e.g., U.S. Pat. No. 6,265,552. Furthermore, click reaction chemistry such as a Methyltetrazine-PEG5-NHS Ester reaction, a TCO-PEG4-NHS Ester reaction, or the like, may be used to couple reporter oligonucleotides to labelling agents. Commercially available kits, such as those from Thunderlink and Abeam, and techniques common in the art may be used to couple reporter oligonucleotides to labelling agents as appropriate. In another example, a labelling agent is indirectly (e.g., via hybridization) coupled to a reporter oligonucleotide comprising a barcode sequence that identifies the labelling agent. For instance, the labelling agent may be directly coupled (e.g., covalently bound) to a hybridization oligonucleotide that comprises a sequence that hybridizes with a sequence of the reporter oligonucleotide. Hybridization of the hybridization oligonucleotide to the reporter oligonucleotide couples the labelling agent to the reporter oligonucleotide. In some embodiments, the reporter oligonucleotides are releasable from the labelling agent, such as upon application of a stimulus. For example, the reporter oligonucleotide may be attached to the labeling agent through a labile bond (e.g., chemically labile, photolabile, thermally labile, etc.) as generally described for releasing molecules from supports elsewhere herein. In some instances, the reporter oligonucleotides described herein may include one or more functional sequences that can be used in subsequent processing, such as an adapter sequence, a unique molecular identifier (UMI) sequence, a sequencer specific flow cell attachment sequence (such as an P5, P7, or partial P5 or P7 sequence), a primer or primer binding sequence, a sequencing primer or primer biding sequence (such as an R1, R2, or partial R1 or R2 sequence).

In some cases, the labelling agent can comprise a reporter oligonucleotide and a label. A label can be fluorophore, a radioisotope, a molecule capable of a colorimetric reaction, a magnetic particle, or any other suitable molecule or compound capable of detection. The label can be conjugated to a labelling agent (or reporter oligonucleotide) either directly or indirectly (e.g., the label can be conjugated to a molecule that can bind to the labelling agent or reporter oligonucleotide). In some cases, a label is conjugated to an oligonucleotide that is complementary to a sequence of the reporter oligonucleotide, and the oligonucleotide may be allowed to hybridize to the reporter oligonucleotide.

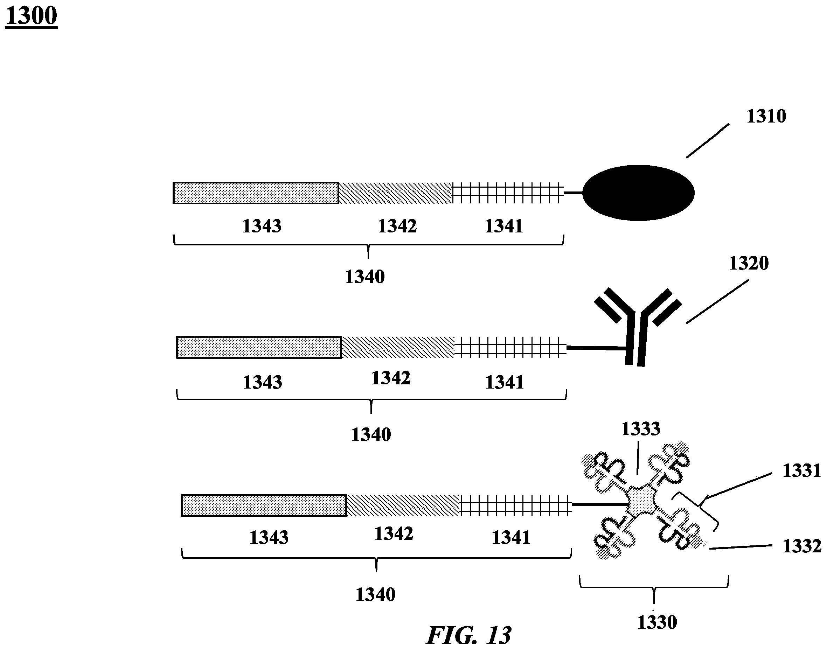

**FIG. 13** describes exemplary labelling agents **(1310, 1320, 1330)** comprising reporter oligonucleotides **(1340)** attached thereto. Labelling agent **1310** (e.g., any of the labelling agents described herein) is attached (either directly, e.g., covalently attached, or indirectly) to reporter oligonucleotide **1340**. Reporter oligonucleotide **1340** may comprise barcode sequence **1342** that identifies labelling agent **1310**. Reporter oligonucleotide **1340** may also comprise one or more functional sequences **1343** that can be used in subsequent processing, such as an adapter sequence, a unique molecular identifier (UMI) sequence, a sequencer specific flow cell attachment sequence (such as an P5, P7, or partial P5 or P7 sequence), a primer or primer binding sequence, or a sequencing primer or primer biding sequence (such as an R1, R2, or partial R1 or R2 sequence).

Referring to **FIG. 13,** in some instances, reporter oligonucleotide **1340** conjugated to a labelling agent (e.g., **1310, 1320, 1330**) comprises a primer sequence **1341,** a barcode sequence **1342** that identifies the labelling agent (e.g., **1310, 1320, 1330),** and functional sequence **1343**. Functional sequence **1343** may be configured to hybridize to a complementary sequence, such as a complementary sequence present on a nucleic acid barcode molecule **1390** (not shown), such as those described elsewhere herein. In some instances, nucleic acid barcode molecule **1390** is attached to a support (e.g., a bead, such as a gel bead), such as those described elsewhere herein. For example, nucleic acid barcode molecule **1390** may be attached to the support via a releasable linkage (e.g., comprising a labile bond), such as those described elsewhere herein. In some instances, reporter oligonucleotide **1340** comprises one or more additional functional sequences, such as those described above.

In some instances, the labelling agent **1310** is a protein or polypeptide (e.g., an antigen or prospective antigen) comprising reporter oligonucleotide **1340**. Reporter oligonucleotide **1340** comprises barcode sequence **1342** that identifies polypeptide **1310** and can be used to infer the presence of an analyte, e.g., a binding partner of polypeptide **1310** (i.e., a molecule or compound to which polypeptide **1310** can bind). In some instances, the labelling agent **1310** is a lipophilic moiety (e.g., cholesterol) comprising reporter oligonucleotide **1340,** where the lipophilic moiety is selected such that labelling agent **1310** integrates into a membrane of a cell or nucleus. Reporter oligonucleotide **1340** comprises barcode sequence **1342** that identifies lipophilic moiety **1310** which in some instances is used to tag cells (e.g., groups of cells, cell samples, etc.) and may be used for multiplex analyses as described elsewhere herein. In some instances, the labelling agent is an antibody **1320** (or an epitope binding fragment thereof) comprising reporter oligonucleotide **1340**. Reporter oligonucleotide **1340** comprises barcode sequence **1342** that identifies antibody **1320** and can be used to infer the presence of, e.g., a target of antibody **1320** (i.e., a molecule or compound to which antibody **1320** binds). In other embodiments, labelling agent **1330** comprises an MHC molecule **1331** comprising peptide **1332** and reporter oligonucleotide **1340** that identifies peptide **1332**. In some instances, the MHC molecule is coupled to a support **1333**. In some instances, support **1333** may be a polypeptide, such as streptavidin, or a polysaccharide, such as dextran. In some instances, reporter oligonucleotide **1340** may be directly or indirectly coupled to MHC labelling agent **1330** in any suitable manner. For example, reporter oligonucleotide **1340** may be coupled to MHC molecule **1331,** support **1333,** or peptide **1332**. In some embodiments, labelling agent **1330** comprises a plurality of MHC molecules, (e.g. is an MHC multimer, which may be coupled to a support (e.g., **1333)).** There are many possible configurations of Class I and/or Class II MHC multimers that can be utilized with the compositions, methods, and systems disclosed herein, e.g., MHC tetramers, MHC pentamers (MHC assembled via a coiled-coil domain, e.g., Pro5® MHC Class I Pentamers, (ProImmune, Ltd.), MHC octamers, MHC dodecamers, MHC decorated dextran molecules (e.g., MHC Dextramer® (Immudex)), etc. For a description of exemplary labelling agents, including antibody and MHC-based labelling agents, reporter oligonucleotides, and methods of use, see, e.g., U.S. Pat. 10,550,429 and U.S. Pat. Pub. 20190367969.

In one aspect, the present disclosure provides reporter agents, as further described herein, that include some of or all of the same structural features of the labelling agents depicted in FIG. 13, including one or more of a functional sequence **1343,** a barcode sequence **1343,** and a primer sequence **1341.** In one embodiment, the reporter agent can be a protein or polypeptide (e.g., an antigen or prospective antigen).

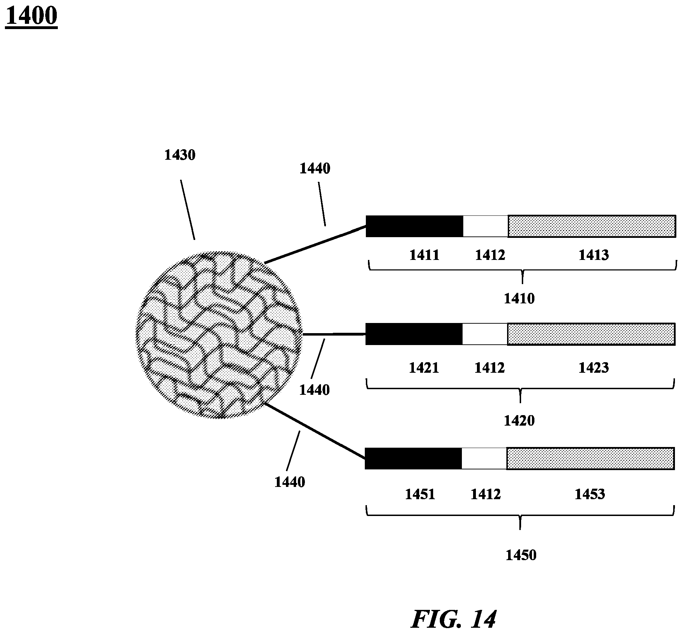

FIG. 14 illustrates another example of a barcode carrying bead. In some embodiments, analysis of multiple analytes (e.g., RNA and one or more analytes using labelling agents described herein) may comprise nucleic acid barcode molecules as generally depicted in FIG. 14. In some embodiments, nucleic acid barcode molecules 1410 and 1420 are attached to support 1430 via a releasable linkage 1440 (e.g., comprising a labile bond) as described elsewhere herein. Nucleic acid barcode molecule 1410 may comprise adapter sequence 1411, barcode sequence 1412 and adapter sequence 1413. Nucleic acid barcode molecule 1420 may comprise adapter sequence 1421, barcode sequence 1412, and adapter sequence 1423, wherein adapter sequence 1423 comprises a different sequence than adapter sequence 1413. In some instances, adapter 1411 and adapter 1421 comprise the same sequence. In some instances, adapter 1411 and adapter 1421 comprise different sequences. Although support 1430 is shown comprising nucleic acid barcode molecules 1410 and 1420, any suitable number of barcode molecules comprising common barcode sequence 1412 are contemplated herein. For example, in some embodiments, support 1430 further comprises nucleic acid barcode molecule 1450. Nucleic acid barcode molecule 1450 may comprise adapter sequence 1451, barcode sequence 1412 and adapter sequence 1453, wherein adapter sequence 1453 comprises a different sequence than adapter sequence 1413 and 1423. In some instances, nucleic acid barcode molecules (e.g., 1410, 1420, 1450) comprise one or more additional functional sequences, such as a UMI or other sequences described herein. The nucleic acid barcode molecules 1410, 1420 or 1450 may interact with analytes as described elsewhere herein, for example, as depicted in FIGS. 15A-C.

Referring to FIG. 15A, in an instance where cells are labelled with labeling agents, sequence 1523 may be complementary to an adapter sequence of a reporter oligonucleotide. Cells may be contacted with one or more reporter oligonucleotide 1510 conjugated labelling agents 1520 (e.g., polypeptide, antibody, or others described elsewhere herein). In one embodiment, sequence 1523 is complementary to a sequence of a reporter oligonucleotide that is provided as part of a reporter agent, as further described herein. The reporter agent may have some of or all of the structural elements of the labelling agent depicted in FIG. 13, including the functional sequence 1343. In some cases, the cells may be further processed prior to barcoding. For example, such processing steps may include one or more washing and/or cell sorting steps. In some instances, a cell that is bound to labelling agent 1520 which is conjugated to oligonucleotide 1510 and support 1530 (e.g., a bead, such as a gel bead) comprising nucleic acid barcode molecule 1590 is partitioned into a partition amongst a plurality of partitions (e.g., a droplet of a droplet emulsion or a well of a microwell array). In some instances, the partition comprises at most a single cell bound to labelling agent 1520. In some instances, reporter oligonucleotide 1510 conjugated to labelling agent 1520 (e.g., polypeptide, an antibody, pMHC molecule such as an MHC multimer, etc.) comprises a first adapter sequence 1511 (e.g., a primer sequence), a barcode sequence 1512 that identifies the labelling agent 1520 (e.g., the polypeptide, antibody, or peptide of a pMHC molecule or complex), and an adapter sequence 1513. Adapter sequence 1513 may be configured to hybridize to a complementary sequence, such as sequence 1523 present on a nucleic acid barcode molecule 1590. In some instances, oligonucleotide 1510 comprises one or more additional functional sequences, such as those described elsewhere herein.

Barcoded nucleic acid molecules may be generated (e.g., via a nucleic acid reaction, such as nucleic acid extension or ligation) from the constructs described in FIGS. 15A-C. For example, sequence 1513 may then be hybridized to complementary sequence 1523 to generate (e.g., via a nucleic acid reaction, such as nucleic acid extension or ligation) a barcoded nucleic acid molecule comprising cell (e.g., partition specific) barcode sequence 1522 (or a reverse complement thereof) and reporter sequence 1512 (or a reverse complement thereof). Barcoded nucleic acid molecules can then be optionally processed as described elsewhere herein, e.g., to amplify the molecules and/or append sequencing platform specific sequences to the fragments. See, e.g., U.S. Pat. Pub. 2018/0105808. Barcoded nucleic acid molecules, or derivatives generated therefrom, can then be sequenced on a suitable sequencing platform.

In some instances, analysis of multiple analytes (e.g., nucleic acids and one or more analytes using labelling agents described herein) may be performed. For example, the workflow may comprise a workflow as generally depicted in any of FIGS. 15A-C, or a combination of workflows for an individual analyte, as described elsewhere herein. For example, by using a combination of the workflows as generally depicted in FIGS. 15A-C, multiple analytes can be analyzed.

In some instances, analysis of an analyte (e.g., a nucleic acid, a polypeptide, a carbohydrate, a lipid, etc.) comprises a workflow as generally depicted in FIG. 15A and/or 15B. A nucleic acid barcode molecule 1590 may be co-partitioned with the one or more analytes. In some instances, nucleic acid barcode molecule 1590 is attached to a support 1530 (e.g., a bead, such as a gel bead or a solid bead, e.g., a magnetic bead), such as those described elsewhere herein. For example, nucleic acid barcode molecule 1590 may be attached to support 1530 via a releasable linkage 1540 (e.g., comprising a labile bond) or linker (such as a non-releasable linker), such as those described elsewhere herein. In one embodiment, the support 1530 may be a solid bead (e.g., a magnetic bead) with nucleic acid barcode molecule 1590 attached via a linker (such as a non-releasable linker).

Nucleic acid barcode molecule **1590** may comprise a barcode sequence **1521** and optionally comprise other additional sequences, for example, a UMI sequence **1522** (or other functional sequences described elsewhere herein). The nucleic acid barcode molecule **1590** may comprise a sequence **1523** that may be complementary to another nucleic acid sequence, such that it may hybridize to a particular sequence.

For example, sequence **1523** may comprise a poly-T sequence and may be used to hybridize to mRNA. Referring to **FIG. 15C,** in some embodiments, nucleic acid barcode molecule **1590** comprises sequence **1523** complementary to a sequence of RNA molecule **1560** from a cell. In some instances, sequence **1523** comprises a sequence specific for an RNA molecule. Sequence **1523** may comprise a known or targeted sequence or a random sequence. In some instances, a nucleic acid extension reaction may be performed, thereby generating a barcoded nucleic acid product comprising sequence **1523,** the barcode sequence **1521,** UMI sequence **1522,** any other functional sequence, and a sequence corresponding to the RNA molecule **1560.**

In another example, sequence **1523** may be complementary to an overhang sequence or an adapter sequence that has been appended to an analyte. For example, referring to **FIG. 15B,** in some embodiments, primer **1550** comprises a sequence complementary to a sequence of nucleic acid molecule **1560** (such as an RNA encoding for a BCR sequence) from a biological particle (or an analyte carrier), such as a cell, a nucleus, or a cell bead. In some instances, primer **1550** comprises one or more sequences **1551** that are not complementary to RNA molecule **1560.** Sequence **1551** may be a functional sequence as described elsewhere herein, for example, an adapter sequence, a sequencing primer sequence, or a sequence the facilitates coupling to a flow cell of a sequencer. In some instances, primer **1550** comprises a poly-T sequence. In some instances, primer **1550** comprises a sequence complementary to a target sequence in an RNA molecule. In some instances, primer **1550** comprises a sequence complementary to a region of an immune molecule, such as the constant region of a TCR or BCR sequence. Primer **1550** is hybridized to nucleic acid molecule **1560** and complementary molecule **1570** is generated. For example, complementary molecule **1570** may be cDNA generated in a reverse transcription reaction. In some instances, an additional sequence may be appended to complementary molecule **1570.** For example, the reverse transcriptase enzyme may be selected such that several non-templated bases **1580** (e.g., a poly-C sequence) are appended to the cDNA. In another example, a terminal transferase may also be used to append the additional sequence. Nucleic acid barcode molecule **1590** comprises a sequence **1524** complementary to the non-templated bases, and the reverse transcriptase performs a template switching reaction onto nucleic acid barcode molecule **1590** to generate a barcoded nucleic acid molecule comprising cell (e.g., partition specific) barcode sequence **1522** (or a reverse complement thereof) and a sequence of complementary molecule **1570** (or a portion thereof). In some instances, sequence **1523** comprises a sequence complementary to a region of an immune molecule, such as the constant region of a TCR or BCR sequence. Sequence **1523** is hybridized to nucleic acid molecule **1560** and a complementary molecule **1570** is generated. For example, complementary molecule 1570 may be generated in a reverse transcription reaction generating a barcoded nucleic acid molecule comprising cell (e.g., partition specific) barcode sequence **1522** (or a reverse complement thereof) and a sequence of complementary molecule **1570** (or a portion thereof). Additional methods and compositions suitable for barcoding cDNA generated from mRNA transcripts including those encoding V(D)J regions of an immune cell receptor and/or barcoding methods and composition including a template switch oligonucleotide are described in International Patent Application WO2018/075693, U.S. Patent Publication No. 2018/0105808, U.S. Patent Publication No. 2015/0376609, filed June 26, 2015, and U.S. Patent Publication No. 2019/03 67969.

In some instances, the analysis of a secreted antibody comprises a workflow as depicted in **FIG. 15D.** Cells may be contacted with one or more reporter agents (as further described herein) that comprise reporter oligonucleotides **1510.** A nucleic acid barcode molecule **1590** may be co-partitioned with a cell (e.g., a plasma B cell) that comprises a complex coupled to the surface of the cell. The complex may include a capture agent, a secreted antibody or antigen binding fragment thereof, and a reporter agent. In some instances, a secreted antibody **1595** (from the complex coupled to the surface of the cell which is not shown in **FIG. 15D)** that is bound to labelling agent **1520** which is conjugated to oligonucleotide **1510** and support **1530** (e.g., a bead, such as a gel bead or a solid bead) comprising nucleic acid barcode molecule **1590** is partitioned into a partition amongst a plurality of partitions (e.g., a droplet of a droplet emulsion or a well of a microwell array). In some instances, the partition includes at most a single cell with the complex coupled to the surface of the cell. The complex includes the capture agent (as further described herein but not shown in **FIG. 15D)** coupled to a secreted antibody **1595,** which is coupled to labelling agent **1520.** In some instances, reporter oligonucleotide **1510** conjugated to labelling agent **1520** (e.g., a protein or polypeptide, such as an antigen or prospective antigen) comprises a first adapter sequence **1511** (e.g., a primer sequence), a barcode sequence **1512** that identifies the labelling agent **1520** (e.g., a protein or polypeptide, such as an antigen or prospective antigen), and an adapter sequence **1513.** Adapter sequence **1513** may be configured to hybridize to a complementary sequence, such as sequence **1523** present on a nucleic acid barcode molecule **1590.** The workflow depicted in **FIG. 15D** for secreted antibodies may be performed simultaneous with the workflow depicted in **FIG.**

**15B,** e.g., for mRNA processing, as further described herein. In one embodiment, the combined workflow provides secreted antibody and mRNA transcript (including those encoding V(D)J regions of immune cell receptors) information on a single cell level. In this case, sequence **1523** of the nucleic acid barcode molecule **1590** is configured to couple to both the complementary molecule **1570** generated as depicted in **FIG. 15B** (e.g., via sequence **1524)** and the adapter sequence **1513.**

In one other aspect, nucleic acid barcode molecules are provided as coupled to one or more beads as further described herein. In one embodiment, a bead may have coupled to it a plurality of nucleic acid barcode molecules (e.g., **1420** in **FIG. 14** or 1590 in FIG. 15A-D) wherein a subset of the plurality of nucleic acid barcode molecules includes a barcode sequence (e.g., **1412** in **FIG. 14** or 1522 in FIG. 15A-D). In such a case, the barcode sequence in the subset of nucleic acid barcode molecules is a common barcode sequence.

### C. Computer Systems

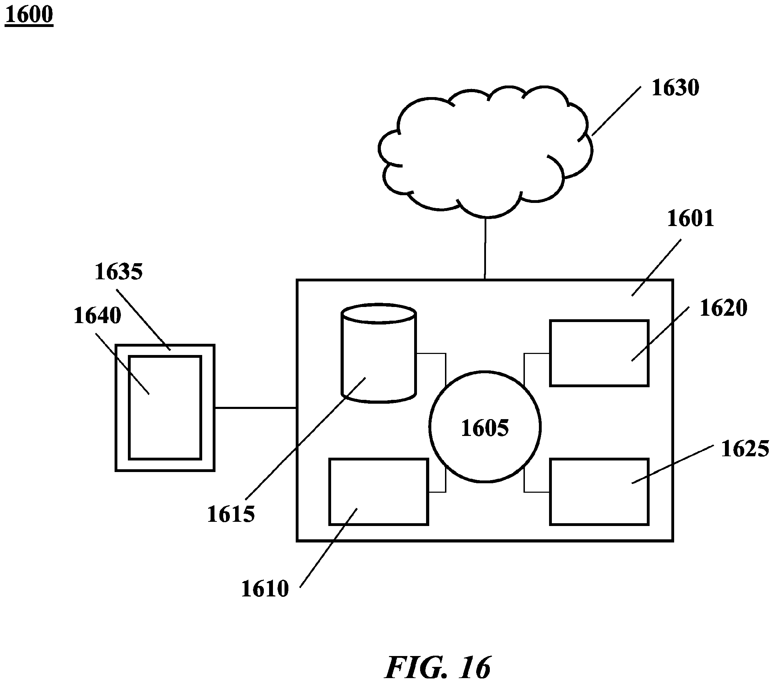

The present disclosure provides computer systems that are programmed to implement methods of the disclosure. **FIG. 16** shows a computer system **1601** that is programmed or otherwise configured to (i) control a microfluidics system (e.g., fluid flow), (ii) sort occupied droplets from unoccupied droplets, (iii) polymerize droplets, (iv) partition cell beads or cells into partitions (e.g., droplets or wells), (v) lysate cells and cell beads, (vi) perform sequencing applications, (vii) generate and maintain libraries of cytokine or other analyte specific antibody barcode sequences, MHC multimer barcode sequences, cell surface protein barcode sequences, and cDNAs generated from mRNAs respectively (vi) analyze such libraries. The computer system **1601** can regulate various aspects of the present disclosure, such as, for example, regulating fluid flow rate in one or more channels in a microfluidic structure, regulating polymerization application units, regulating sequence application unit, etc. The computer system **1601** can be an electronic device of a user or a computer system that is remotely located with respect to the electronic device. The electronic device can be a mobile electronic device.

The computer system **1601** includes a central processing unit (CPU, also "processor" and "computer processor" herein) **1605,** which can be a single core or multi core processor, or a plurality of processors for parallel processing. The computer system **1601** also includes memory or memory location **1610** (e.g., random-access memory, read-only memory, flash memory), electronic storage unit **1615** (e.g., hard disk), communication interface **1620** (e.g., network adapter) for communicating with one or more other systems, and peripheral devices **1625,** such as cache, other memory, data storage and/or electronic display adapters. The memory **1610,** storage unit **1615,** interface **1620** and peripheral devices **1625** are in communication with the CPU **1605** through a communication bus (solid lines), such as a motherboard. The storage unit **1615** can be a data storage unit (or data repository) for storing data. The computer system **1601** can be operatively coupled to a computer network ("network") **1630** with the aid of the communication interface **1620.** The network **1630** can be the Internet, an internet and/or extranet, or an intranet and/or extranet that is in communication with the Internet. The network **1630** in some cases is a telecommunication and/or data network. The network **1630** can include one or more computer servers, which can enable distributed computing, such as cloud computing. The network **1630,** in some cases with the aid of the computer system **1601,** can implement a peer-to-peer network, which may enable devices coupled to the computer system **1601** to behave as a client or a server.

The CPU **1605** can execute a sequence of machine-readable instructions, which can be embodied in a program or software. The instructions may be stored in a memory location, such as the memory **1610.** The instructions can be directed to the CPU **1605,** which can subsequently program or otherwise configure the CPU **1605** to implement methods of the present disclosure. Examples of operations performed by the CPU **1605** can include fetch, decode, execute, and writeback.

The CPU **1605** can be part of a circuit, such as an integrated circuit. One or more other components of the system **1601** can be included in the circuit. In some cases, the circuit is an application specific integrated circuit (ASIC).

The storage unit **1615** can store files, such as drivers, libraries and saved programs. The storage unit **1615** can store user data, e.g., user preferences and user programs. The computer system **1601** in some cases can include one or more additional data storage units that are external to the computer system **1601,** such as located on a remote server that is in communication with the computer system **1601** through an intranet or the Internet.

The computer system **1601** can communicate with one or more remote computer systems through the network **1630.** For instance, the computer system **1601** can communicate with a remote computer system of a user (e.g., operator). Examples of remote computer systems include personal computers (e.g., portable PC), slate or tablet PC's (e.g., Apple® iPad, Samsung® Galaxy Tab), telephones, Smart phones (e.g., Apple® iPhone, Android-enabled device, Blackberry®), or personal digital assistants. The user can access the computer system **1601** via the network **1630.**

Methods as described herein can be implemented by way of machine (e.g., computer processor) executable code stored on an electronic storage location of the computer system **1601,** such as, for example, on the memory **1610** or electronic storage unit **1615.** The machine executable or machine readable code can be provided in the form of software. During use, the code can be executed by the processor **1605.** In some cases, the code can be retrieved from the storage unit **1615** and stored on the memory **1610** for ready access by the processor **1605.** In some situations, the electronic storage unit **1615** can be precluded, and machine-executable instructions are stored on memory **1610.**

The code can be pre-compiled and configured for use with a machine having a processor adapted to execute the code, or can be compiled during runtime. The code can be supplied in a programming language that can be selected to enable the code to execute in a pre-compiled or as-compiled fashion.

Aspects of the systems and methods provided herein, such as the computer system **1601,** can be embodied in programming. Various aspects of the technology may be thought of as "products" or "articles of manufacture" typically in the form of machine (or processor) executable code and/or associated data that is carried on or embodied in a type of machine readable medium. Machine-executable code can be stored on an electronic storage unit, such as memory (e.g., read-only memory, random-access memory, flash memory) or a hard disk. "Storage" type media can include any or all of the tangible memory of the computers, processors or the like, or associated modules thereof, such as various semiconductor memories, tape drives, disk drives and the like, which may provide non-transitory storage at any time for the software programming. All or portions of the software may at times be communicated through the Internet or various other telecommunication networks. Such communications, for example, may enable loading of the software from one computer or processor into another, for example, from a management server or host computer into the computer platform of an application server. Thus, another type of media that may bear the software elements includes optical, electrical and electromagnetic waves, such as used across physical interfaces between local devices, through wired and optical landline networks and over various air-links. The physical elements that carry such waves, such as wired or wireless links, optical links or the like, also may be considered as media bearing the software. As used herein, unless restricted to non-transitory, tangible "storage" media, terms such as computer or machine "readable medium" refer to any medium that participates in providing instructions to a processor for execution.

Hence, a machine readable medium, such as computer-executable code, may take many forms, including but not limited to, a tangible storage medium, a carrier wave medium or physical transmission medium. Non-volatile storage media include, for example, optical or magnetic disks, such as any of the storage devices in any computer(s) or the like, such as may be used to implement the databases, etc. shown in the drawings. Volatile storage media include dynamic memory, such as main memory of such a computer platform. Tangible transmission media include coaxial cables; copper wire and fiber optics, including the wires that comprise a bus within a computer system. Carrier-wave transmission media may take the form of electric or electromagnetic signals, or acoustic or light waves such as those generated during radio frequency (RF) and infrared (IR) data communications. Common forms of computer-readable media therefore include for example: a floppy disk, a flexible disk, hard disk, magnetic tape, any other magnetic medium, a CD-ROM, DVD or DVD-ROM, any other optical medium, punch cards paper tape, any other physical storage medium with patterns of holes, a RAM, a ROM, a PROM and EPROM, a FLASH-EPROM, any other memory chip or cartridge, a carrier wave transporting data or instructions, cables or links transporting such a carrier wave, or any other medium from which a computer may read programming code and/or data. Many of these forms of computer readable media may be involved in carrying one or more sequences of one or more instructions to a processor for execution.

The computer system **1601** can include or be in communication with an electronic display **1635** that comprises a user interface (UI) **1640** for providing, for example, results of sequencing analysis, etc. Examples of UIs include, without limitation, a graphical user interface (GUI) and web-based user interface.

Methods and systems of the present disclosure can be implemented by way of one or more algorithms. An algorithm can be implemented by way of software upon execution by the central processing unit **1605.** The algorithm can, for example, perform nucleotide sequence amplification, sequencing sorting based on barcode sizes, sequencing amplified barcode sequences, analyzing sequencing data, etc.

Devices, systems, compositions and methods of the present disclosure may be used for various applications, such as, for example, processing a single analyte (e.g., RNA, DNA, or protein) or multiple analytes (e.g., DNA and RNA, DNA and protein, RNA and protein, or RNA, DNA and protein) from a single cell. For example, a biological particle (e.g., a cell or cell bead) is partitioned in a partition (e.g., droplet), and multiple analytes from the biological particle are processed for subsequent processing. The multiple analytes (e.g., secreted antibodies or antigen fragments thereof) may be from the single cell (e.g., immune cell, e.g., B cell). This may enable, for example, simultaneous proteomic, transcriptomic and genomic analysis of the cell.

## X. DEFINITIONS

Unless defined otherwise, all terms of art, notations and other technical and scientific terms or terminology used herein are intended to have the same meaning as is commonly understood by one of ordinary skill in the art to which the claimed subject matter pertains. In some cases, terms with commonly understood meanings are defined herein for clarity and/or for ready reference, and the inclusion of such definitions herein should not necessarily be construed to represent a substantial difference over what is generally understood in the art.

The term "barcode," as used herein, generally refers to a label, or identifier, that conveys or is capable of conveying information about an analyte. A barcode can be part of an analyte. A barcode can be independent of an analyte. A barcode can be a tag attached to an analyte (e.g., nucleic acid molecule) or a combination of the tag in addition to an endogenous characteristic of the analyte (e.g., size of the analyte or end sequence(s)). A barcode may be unique. Barcodes can have a variety of different formats. For example, barcodes can include: polynucleotide barcodes; random nucleic acid and/or amino acid sequences; and synthetic nucleic acid and/or amino acid sequences. A barcode can be attached to an analyte in a reversible or irreversible manner. A barcode can be added to, for example, a fragment of a deoxyribonucleic acid (DNA) or ribonucleic acid (RNA) sample before, during, and/or after sequencing of the sample. Barcodes can allow for identification and/or quantification of individual sequencing-reads.

The terms "adaptor(s)", "adapter(s)" and "tag(s)" may be used synonymously. An adaptor or tag can be coupled to a polynucleotide sequence to be "tagged" by any approach, including ligation, hybridization, or other approaches.

"Sequencing," "sequence determination" and the like means determination of information relating to the nucleotide base sequence of a nucleic acid. Such information may include the identification or determination of partial as well as full sequence information of the nucleic acid. Sequence information may be determined with varying degrees of statistical reliability or confidence. In one aspect, the term includes the determination of the identity and ordering of a plurality of contiguous nucleotides in a nucleic acid. "High throughput digital sequencing" or "next generation sequencing" means sequence determination using methods that determine many (typically thousands to billions) of nucleic acid sequences in an intrinsically parallel manner, *i.e.* where DNA templates are prepared for sequencing not one at a time, but in a bulk process, and where many sequences are read out preferably in parallel, or alternatively using an ultra-high throughput serial process that itself may be parallelized. Such methods include but are not limited to pyrosequencing (for example, as commercialized by 454 Life Sciences, Inc., Branford, Conn.); sequencing by ligation (for example, as commercialized in the SOLiD™ technology, Life Technologies, Inc., Carlsbad, Calif.); sequencing by synthesis using modified nucleotides (such as commercialized in TruSeq™ and HiSeq™ technology by Illumina, Inc., San Diego, Calif.; HeliScope™ by Helicos Biosciences Corporation, Cambridge, Ma.; and PacBio RS by Pacific Biosciences of California, Inc., Menlo Park, Calif.), sequencing by ion detection technologies (such as Ion Torrent™ technology, Life Technologies, Carlsbad, Calif.); sequencing of DNA nanoballs (Complete Genomics, Inc., Mountain View, Calif.); nanopore-based sequencing technologies (for example, as developed by Oxford Nanopore Technologies, LTD, Oxford, UK), and like highly parallelized sequencing methods.

The term "bead," as used herein, generally refers to a particle. The bead may be a solid or semi-solid particle. The bead may be a gel bead. The gel bead may include a polymer matrix (e.g., matrix formed by polymerization or cross-linking). The polymer matrix may include one or more polymers (e.g., polymers having different functional groups or repeat units). Polymers in the polymer matrix may be randomly arranged, such as in random copolymers, and/or have ordered structures, such as in block copolymers. Cross-linking can be via covalent, ionic, or inductive, interactions, or physical entanglement. The bead may be a macromolecule. The bead may be formed of nucleic acid molecules bound together. The bead may be formed via covalent or non-covalent assembly of molecules (e.g., macromolecules), such as monomers or polymers. Such polymers or monomers may be natural or synthetic. Such polymers or monomers may be or include, for example, nucleic acid molecules (e.g., DNA or RNA). The bead may be formed of a polymeric material. The bead may be magnetic or non-magnetic. The bead may be rigid. The bead may be flexible and/or compressible. The bead may be disruptable or dissolvable. The bead may be a solid particle (e.g., a metal-based particle including but not limited to iron oxide, gold or silver) covered with a coating comprising one or more polymers. Such coating may be disruptable or dissolvable.

As used herein, the term "barcoded nucleic acid molecule" generally refers to a nucleic acid molecule that results from, for example, the processing of a nucleic acid barcode molecule with a nucleic acid sequence (e.g., nucleic acid sequence complementary to a nucleic acid primer sequence encompassed by the nucleic acid barcode molecule). The nucleic acid sequence may be a targeted sequence or a non-targeted sequence. The nucleic acid barcode molecule may be coupled to or attached to the nucleic acid molecule comprising the nucleic acid sequence. For example, in the methods and systems described herein, hybridization and reverse transcription of a nucleic acid molecule (e.g., a messenger RNA (mRNA) molecule) of a cell with a nucleic acid barcode molecule (e.g., a nucleic acid barcode molecule containing a barcode sequence and a nucleic acid primer sequence complementary to a nucleic acid sequence of the mRNA molecule) results in a barcoded nucleic acid molecule that has a sequence corresponding to the nucleic acid sequence of the mRNA and the barcode sequence (or a reverse complement thereof). The processing of the nucleic acid molecule comprising the nucleic acid sequence, the nucleic acid barcode molecule, or both, can include a nucleic acid reaction, such as, in non-limiting examples, reverse transcription, nucleic acid extension, ligation, etc. The nucleic acid reaction may be performed prior to, during, or following barcoding of the nucleic acid sequence to generate the barcoded nucleic acid molecule. For example, the nucleic acid molecule comprising the nucleic acid sequence may be subjected to reverse transcription and then be attached to the nucleic acid barcode molecule to generate the barcoded nucleic acid molecule, or the nucleic acid molecule comprising the nucleic acid sequence may be attached to the nucleic acid barcode molecule and subjected to a nucleic acid reaction (e.g., extension, ligation) to generate the barcoded nucleic acid molecule. A barcoded nucleic acid molecule may serve as a template, such as a template polynucleotide, that can be further processed (e.g., amplified) and sequenced to obtain the target nucleic acid sequence. For example, in the methods and systems described herein, a barcoded nucleic acid molecule may be further processed (e.g., amplified) and sequenced to obtain the nucleic acid sequence of the nucleic acid molecule (e.g., mRNA).

The term "biological particle," as used herein, generally refers to a discrete biological system derived from a biological sample. The biological particle may be a macromolecule. The biological particle may be a small molecule. The biological particle may be a virus. The biological particle may be a cell or derivative of a cell. The biological particle may be an organelle. The biological particle may be a rare cell from a population of cells. The biological particle may be any type of cell, including without limitation prokaryotic cells, eukaryotic cells, bacterial, fungal, plant, mammalian, or other animal cell type, mycoplasmas, normal tissue cells, tumor cells, or any other cell type, whether derived from single cell or multicellular organisms. The biological particle may be a constituent of a cell. The biological particle may be or may include DNA, RNA, organelles, proteins, or any combination thereof. The biological particle may be or may include a matrix (e.g., a gel or polymer matrix) comprising a cell or one or more constituents from a cell (e.g., cell bead), such as DNA, RNA, organelles, proteins, or any combination thereof, from the cell. The biological particle may be obtained from a tissue of a subject. The biological particle may be a hardened cell. Such hardened cell may or may not include a cell wall or cell membrane. The biological particle may include one or more constituents of a cell, but may not include other constituents of the cell. An example of such constituents is a nucleus or an organelle. A cell may be a live cell. The live cell may be capable of being cultured, for example, being cultured when enclosed in a gel or polymer matrix, or cultured when comprising a gel or polymer matrix.

The terms "coupled," "linked," "conjugated," "associated," "attached," "connected" or "fused," as used herein, may be used interchangeably herein and generally refer to one molecule (e.g., polypeptide, receptor, analyte, etc.) being attached or connected (e.g., chemically bound) to another molecule (e.g., polypeptide, receptor, analyte, etc.).

The term "binding agent," as used herein generally refers to a molecule capable of binding to one or more other molecules (e.g., analytes, receptors, other binding agents, etc.) and that comprises one or more portions. In some cases, a binding agent comprises at least one, at least two, at least three, or at least four portions. Each portion may comprise a polypeptide. The polypeptide of a specific portion may be capable of binding one or more molecules. For example, a polypeptide of a specific portion may bind to a molecule located on a surface of a cell, such as a cell surface protein or receptor (e.g., a CD surface marker such as CD45). A polypeptide of another portion of a binding agent may bind a molecule that may be secreted from a cell (e.g., T cell, B-cell, dendritic cell, etc.). The one or more portions of a binding agent may be directly or indirectly linked to, conjugated to, or fused to one another. For example, a first portion of a binding agent may be directly or indirectly linked to, conjugated to, or fused to a second portion of the binding agent. Moreover, the terms "binding agent," "polypeptide," and "antibody" may be used interchangeably herein.

The term "cell bead," as used herein, generally refers to a hydrogel, polymeric, or crosslinked material that comprises (e.g., encapsulates, contains, etc.) a biological particle (e.g., a cell, a nucleus, a fixed cell, a cross-linked cell), a virus, components of or macromolecular constituents of or derived from a cell or virus. For example, a cell bead may comprise a virus and/or a cell. In some cases, a cell bead comprises a single cell. In some cases, a cell bead may comprise multiple cells adhered together. A cell bead may include any type of cell, including without limitation prokaryotic cells, eukaryotic cells, bacterial, fungal, plant, mammalian, or other animal cell types, mycoplasmas, normal tissue cells, tumor cells, immune cells, e.g., a T-cell (e.g., CD4 T-cell, CD4 T-cell that comprises a dormant copy of human immunodeficiency virus (HIV)), a B cell, or a dendritic cell, a fixed cell, a cross-linked cell, a rare cell from a population of cells, or any other cell type, whether derived from single cell or multicellular organisms. Furthermore, a cell bead may comprise a live cell, such as, for example, a cell may be capable of being cultured. Moreover, in some examples, a cell bead may comprise a derivative of a cell, such as one or more components of the cell (e.g., an organelle, a cell protein, a cellular nucleic acid, genomic nucleic acid, messenger ribonucleic acid, a ribosome, a cellular enzyme, etc.). In some examples, a cell bead may comprise material obtained from a biological tissue, such as, for example, obtained from a subject. In some cases, cells, viruses or macromolecular constituents thereof are encapsulated within a cell bead. Encapsulation can be within a polymer or gel matrix that forms a structural component of the cell bead. In some cases, a cell bead is generated by fixing a cell in a fixation medium or by cross-linking elements of the cell, such as the cell membrane, the cell cytoskeleton, etc.

The term "macromolecular constituent," as used herein, generally refers to a macromolecule contained within or from a biological particle. The macromolecular constituent may comprise a nucleic acid. In some cases, the biological particle may be a macromolecule. The macromolecular constituent may comprise DNA.

The macromolecular constituent may comprise RNA. The RNA may be coding or non-coding. The RNA may be messenger RNA (mRNA), ribosomal RNA (rRNA) or transfer RNA (tRNA), for example. The RNA may be a transcript. The RNA may be small RNA that are less than 200 nucleic acid bases in length, or large RNA that are greater than 200 nucleic acid bases in length. Small RNAs may include 5.8S ribosomal RNA (rRNA), 5S rRNA, transfer RNA (tRNA), microRNA (miRNA), small interfering RNA (siRNA), small nucleolar RNA (snoRNAs), Piwi-interacting RNA (piRNA), tRNA-derived small RNA (tsRNA) and small rDNA-derived RNA (srRNA). The RNA may be double-stranded RNA or single-stranded RNA. The RNA may be circular RNA. The macromolecular constituent may comprise a protein. The macromolecular constituent may comprise a peptide. The macromolecular constituent may comprise a polypeptide.

The terms "antigen binding fragment," "epitope binding fragment," or "antibody fragment," as used herein, may be used interchangeably and generally refer to a portion of a complete antibody (e.g., comprising each domain of the light and heavy chains respectively) capable of binding the same epitope/antigen as the complete antibody, albeit not necessarily to the same extent. Although multiple types of epitope binding fragments are possible, an epitope binding fragment typically comprises at least one pair of heavy and light chain variable regions (VH and VL, respectively) held together (e.g., by disulfide bonds) to preserve the antigen binding site and does not contain all or a portion of the Fc region. Epitope binding fragments of an antibody can be obtained from a given antibody by any suitable technique (e.g., recombinant DNA technology or enzymatic or chemical cleavage of a complete antibody), and typically can be screened for specificity in the same manner in which complete antibodies are screened. In some embodiments, an epitope binding fragment comprises an $F(ab')_2$ fragment, Fab' fragment, Fab fragment, Fd fragment, or Fv fragment. In some embodiments, the term "antibody" includes antibody-derived polypeptides, such as single chain variable fragments (scFv), diabodies or other multimeric scFvs, heavy chain antibodies, single domain antibodies, or other polypeptides comprising a sufficient portion of an antibody (e.g., one or more complementarity determining regions (CDRs)) to confer specific antigen binding ability to the polypeptide.

The term "molecular tag," as used herein, generally refers to a molecule capable of binding to a macromolecular constituent. The molecular tag may bind to the macromolecular constituent with high affinity. The molecular tag may bind to the macromolecular constituent with high specificity. The molecular tag may comprise a nucleotide sequence. The molecular tag may comprise a nucleic acid sequence. The nucleic acid sequence may be at least a portion or an entirety of the molecular tag. The molecular tag may be a nucleic acid molecule or may be part of a nucleic acid molecule. The molecular tag may be an oligonucleotide or a polypeptide. The molecular tag may comprise a DNA aptamer. The molecular tag may be or comprise a primer. The molecular tag may be, or comprise, a protein. The molecular tag may comprise a polypeptide. The molecular tag may be a barcode.

The term "partition," as used herein, generally, refers to a space or volume that may be suitable to contain one or more species or conduct one or more reactions. A partition may be a physical compartment, such as a droplet or well. The partition may isolate space or volume from another space or volume. The droplet may be a first phase (e.g., aqueous phase) in a second phase (e.g., oil) immiscible with the first phase. The droplet may be a first phase in a second phase that does not phase separate from the first phase, such as, for example, a capsule or liposome in an aqueous phase. A partition may comprise one or more other (inner) partitions. In some cases, a partition may be a virtual compartment that can be defined and identified by an index (e.g., indexed libraries) across multiple and/or remote physical compartments. For example, a physical compartment may comprise a plurality of virtual compartments.

The term "analyte," as used herein, generally refers to a species of interest for detection. An analyte may be biological analyte, such as a nucleic acid molecule or protein. An analyte may be an atom or molecule. An analyte be a subunit of a larger unit, such as, e.g., a given sequence of a polynucleotide sequence or a sequence as part of a larger sequence. An analyte of the present disclosure includes a secreted analyte, a soluble analyte, and/or an extracellular analyte.

The term "about" as used herein refers to the usual error range for the respective value readily known to the skilled person in this technical field. Reference to "about" a value or parameter herein comprises (and describes) embodiments that are directed to that value or parameter per se.

As used herein, the singular forms "a," "an," and "the" comprise plural referents unless the context clearly dictates otherwise. For example, "a" or "an" means "at least one" or "one or more."

Throughout this disclosure, various aspects of the claimed subject matter are presented in a range format. It should be understood that the description in range format is merely for convenience and brevity and should not be construed as an inflexible limitation on the scope of the claimed subject matter. Accordingly, the description of a range should be considered to have specifically disclosed all the possible sub-ranges as well as individual numerical values within that range. For example, where a range of values is provided, it is understood that each intervening value, between the upper and lower limit of that range and any other stated or intervening value in that stated range is encompassed within the claimed subject matter. The upper and lower limits of these smaller ranges may independently be comprised in the smaller ranges, and are also encompassed within the claimed subject matter, subject to any specifically excluded limit in the stated range. Where the stated range comprises one or both of the limits, ranges excluding either or both of those comprised limits are also comprised in the claimed subject matter. This applies regardless of the breadth of the range.

Use of ordinal terms such as "first", "second", "third", etc., in the claims to modify a claim element does not by itself connote any priority, precedence, or order of one claim element over another or the temporal order in which acts of a method are performed, but are used merely as labels to distinguish one claim element having a certain name from another element having a same name (but for use of the ordinal term) to distinguish the claim elements. Similarly, use of a), b), etc., or i), ii), etc. does not by itself connote any priority, precedence, or order of steps in the claims. Similarly, the use of these terms in the specification does not by itself connote any required priority, precedence, or order.

**EXAMPLES**

The following examples are included for illustrative purposes only and are not intended to limit the scope of the present disclosure.

**Example 1: Labeling a secreted antibody with a reporter barcode sequence using capture agents**

This example illustrates the use of capture agents to study plasma cell antibodies, outlined in **FIG. 7.** A visual representation of this process is shown in **FIG. 8.**

A cell population of interest (e.g., antigen-specific memory B cells, plasma cells after immunization or vaccination, or memory B cells co-incubated with the antigen of interest) is isolated by flow cytometry, magnetic enrichment with an antigen bound to the surface, BACS, or another alternative method. Isolated B cells are partitioned into droplets to produce single cell suspensions.

The isolated B cell single cell suspension is then incubated with an antigen capture agent (e.g., capture antibody). An antigen capture agent may be any bispecific molecule that recognizes a common antigen on the surface of a B cell and the constant region of a secreted antibody. The antigen capture agent may be an scFv or nanobody reagent, apatamer, bispecific antibody, F(ab')2 reagent, or other biomolecule recognizing both the Fc region of the secreted antibody and a B cell surface antigen, including but not limited to CD19, CD20, CD45, or CD22. In other examples, an antigen capture agent may also be an Fc-binding biomolecule tethered to a lipid, enabling insertion of the biomolecule into a cellular membrane where it acts to tether the secreted antibody to the surface of the cell. The capture agent is optionally conjugated to an oligonucleotide barcode sequence, wherein an oligonucleotide barcode sequence is a unique oligonucleotide that can be amplified downstream.

The capture agent incubated cells are then exposed to one or more reporter barcoded antigens (i.e., reporter agents). Antigens may be conjugated to a fluorophore. In some examples, antigen-specific plasma cells are further enriched by a secondary enrichment against the antigen (e.g., target the fluorophore or another part of the antigen, to produce a single cell suspension for partitioning), or by forming alternative partitions (e.g., cell beads) in a selective fashion. The polymer matrix of a cell bead is formed by addition of hydrogen peroxide, when the antigen has a horseradish peroxidase or similar domain conjugated, exclusively producing partitions around cells binding the antigens of interest.

The single cell suspensions of interest are captured, and barcoding and V(D)J amplification is subsequently performed (e.g., using the Immune Profiling Solution of the 10× Genomics Chromium™ system). This produces a vector of antigen-specific counts for each antigen, for each captured B cell, enabling the identification of the antigen specificity of that cell.

**Example 2: Labeling a secreted antibody with a reporter barcode sequence using cell beads**

This example illustrates the use of cell beads to study plasma cell antibodies, outlined in **FIG. 9.** A visual representation of this process is shown in **FIGS. 10A and 10B.**

A cell population of interest (e.g., antigen-specific memory B cells, plasma cells after immunization or vaccination, or memory B cells co-incubated with the antigen of interest) is isolated by flow cytometry, magnetic enrichment with an antigen bound to the surface, BACS, or another alternative method. Isolated B cells are partitioned into droplets forming a single cell suspension, which subsequently gels into a hydrogel matrix (e.g., cell bead). Secreted antibodies (i.e., IgG) from the encapsulated B cells are secreted and emanate from the cell surface, **FIG. 10A,** are captured by the hydrogel matrix of the cell bead, and remain tethered to the hydrogel matrix, e.g., **FIG. 10B.**

The cell beads with tethered antibodies are then exposed to one or more reporter barcoded antigens (i.e., reporter agents), wherein a reporter barcode sequence of the reporter barcoded antigen is a unique oligonucleotide that can be amplified downstream. Antigens may be conjugated to a fluorophore. In some examples, antigen-specific plasma cells are further enriched by a secondary enrichment against the antigen (i.e., target the fluorophore or another part of the antigen, to produce a single cell suspension for partitioning), or by forming alternative partitions (e.g., cell bead-gel beads) in a selective fashion. The polymer matrix of a cell bead-gel bead is formed by addition of hydrogen peroxide, when the antigen has a horseradish peroxidase or similar domain conjugated, exclusively producing partitions around cells binding the antigens of interest.

The cell beads with tethered antibodies are then exposed to one or more reporter barcoded antigens (i.e., reporter agents), wherein a reporter barcode sequence of the reporter barcoded antigen is a unique oligonucleotide that can be amplified downstream. Antigens may be conjugated to a fluorophore. In some examples, antigen-specific plasma cells are further enriched by a secondary enrichment against the antigen (i.e., target the fluorophore or another part of the antigen, to produce a single cell suspension for partitioning), or by forming alternative partitions (e.g., cell bead-gel beads) in a selective fashion. The polymer matrix of a cell bead-gel bead is formed by addition of hydrogen peroxide, when the antigen has a horseradish peroxidase or similar domain conjugated, exclusively producing partitions around cells binding the antigens of interest.

In some examples, anti-fluorophore or anti-enrichment antibodies on a magnetic bead, optionally possessing a reporter barcode sequence different from that of the antigens, are added to the cell beads. The cell beads of interest are captured, and barcoding and VDJ amplification is subsequently performed (e.g., using the Immune Profiling Solution of the 10× Genomics Chromium™ system). This produces a vector of antigen-specific counts for each antigen, for each captured B cell, enabling the identification of the antigen specificity of that cell using approaches for count transformation and imputation appreciable by those knowledgeable in the art.

## Example 3: Detection and analysis of secreted antibodies

Cells with a reasonable number of antigen-binding counts from Examples 1 and 2 are identified. Within this group, cells which also have additional multiomic counts (e.g., cellular RNA or surface protein) are identified. Transform the antigen-binding count distribution by subtracting the median or mean background for each antigen as detected in empty droplets. Cells that bind the antigen of interest and the control antigen, or just the control antigen, are excluded from further analysis. Then, transform the antigen count vector using any number of approaches (e.g., centered log ratio, PCA, GLMPCA, or more complex machine learning models or neural networks). Finally, antibodies with antigen fingerprints are chosen for therapeutic evaluation

The cDNA library may be used to selectively amplify plasma cell sequences encoding secreted antibodies of interest by using primers that hybridize to a give cell barcode or cell of interest, and the heavy chain and light chain constant regions. Separate heavy chain and light chain amplification occurs, followed by overlap extension PCR to combine them into a single continuous molecule. Heavy chain and light chains are directly paired, enabling an antibody with ideal antigen specific to be rapidly cloned and converted into a format amenable to therapeutic development and testing.

## Claims

1. A method of single cell analysis, comprising:

   a) contacting an antibody-secreting cell with a reporter agent comprising a reporter nucleic acid molecule to provide a labelled cell, wherein the reporter nucleic acid molecule comprises a reporter sequence corresponding to the reporter agent, wherein the labelled cell comprises a complex coupled to a surface of the cell, and wherein the complex comprises (i) a capture agent, (ii) a secreted antibody or antigen binding fragment thereof, and (iii) the reporter agent, wherein the capture agent is configured to couple to both a cell surface molecule and the secreted antibody or antigen binding fragment thereof, and wherein the reporter agent is configured to couple to the secreted antibody or antigen binding fragment thereof;
   b) partitioning the labelled cell in a partition, wherein the partition comprises a plurality of barcode nucleic acid molecules which comprise a common partition-specific barcode sequence, wherein said reporter nucleic acid molecule further comprises a sequence configured to couple to a barcode nucleic acid molecule of the plurality of barcode nucleic acid molecules;
   c) in bulk or in the partition, generating a barcoded nucleic acid molecule from the barcode nucleic acid molecule and the reporter nucleic acid molecule, wherein the barcoded nucleic acid molecule comprises the reporter sequence or a complement thereof and the common partition-specific barcode sequence or a complement thereof; and
   d) sequencing the barcoded nucleic acid molecule to obtain a nucleic acid sequence of the barcoded nucleic acid molecule, optionally wherein the barcoded nucleic acid molecule is amplified prior to sequencing.

2. The method of claim 1, wherein:

   (a) the antibody-secreting cell is of a B-cell lineage, optionally wherein the B-cell lineage cell is a plasma cell;
   (b) said cell surface molecule is a cell surface protein;
   (c) said capture agent is a first antibody binding agent; and/or
   (d) said reporter agent is a second antibody binding agent.

3. The method of claim 1 or claim 2, wherein said labelled cell further comprises a second reporter agent comprising a second reporter nucleic acid molecule, optionally wherein:

(a) said second reporter agent is configured to couple to a second cell surface molecule, wherein the second cell surface molecule is a second cell surface protein of the cell; and/or

(b) said second reporter nucleic acid molecule comprises a second reporter sequence corresponding to the second reporter agent.

4. The method of claim any of claims 1-3, wherein the partition further comprises a second barcode nucleic acid molecule of a plurality of second barcode nucleic acid molecules, wherein the second barcode nucleic acid molecule of the plurality of second barcode nucleic acid molecules comprise the common partition-specific barcode sequence, optionally wherein:

(a) said second reporter nucleic acid molecule comprises a second sequence configured to couple to the second barcode nucleic acid molecule; and/or

(b) step (c) further comprises generating a second barcoded nucleic acid molecule from a second barcode nucleic acid molecule from the plurality of second barcode nucleic acid molecules and the second reporter nucleic acid molecule, wherein the second barcoded nucleic acid molecule comprises the second reporter sequence from the second reporter nucleic acid molecule and the common partition-specific barcode from the second barcode nucleic acid molecule, or complements thereof.

5. The method of any of claims 1-4, wherein said labelled cell further comprises a plurality of nucleic acid analytes, wherein a nucleic acid analyte of said plurality of nucleic acid analytes comprises a nucleic acid analyte sequence.

6. The method of any of claims 1-5, wherein the partition further comprises a plurality of third barcode nucleic acid molecules comprising the common partition-specific barcode sequence, optionally wherein:

(a) a third barcode nucleic acid molecule of said plurality of third barcode nucleic acid molecules comprises a third sequence configured to couple to the nucleic acid analyte sequence; and/or

(b) step (c) further comprises generating a third barcoded nucleic acid molecule from a third barcode nucleic acid molecule from the plurality of third barcode nucleic acid molecules and the nucleic acid analyte, wherein the third barcoded nucleic acid molecule comprises sequences from the nucleic acid analyte and the common partition-specific barcode sequence from the third barcode nucleic acid molecule, or complements thereof.

7. The method of any of claims 1-6, wherein said partition comprises a support that comprises the plurality of barcode nucleic acid molecules, optionally wherein:

(a) the support is a bead, optionally wherein the bead is a gel bead; and/or

(b) said plurality of barcode nucleic acid molecules are releasably attached to the support.

8. A method of single cell analysis, comprising:

a) contacting a cell bead with a reporter agent comprising a reporter nucleic acid molecule to provide a labelled cell bead, wherein the cell bead comprises an antibody-secreting cell in a matrix, wherein the reporter nucleic acid molecule comprises a reporter sequence corresponding to the reporter agent, wherein the labelled cell bead comprises a complex in or on the cell bead, and wherein the complex comprises (i) a capture agent, (ii) a secreted antibody or antigen binding fragment thereof, and (iii) the reporter agent, wherein said capture agent is configured to couple to both the matrix and said secreted antibody or antigen binding fragment thereof, and wherein the reporter agent is configured to couple to the secreted antibody or antigen binding fragment thereof;

b) partitioning the labelled cell bead in a partition, wherein the partition comprises a plurality of barcode nucleic acid molecules which comprise a common partition-specific barcode sequence, wherein said reporter nucleic acid molecule further comprises a sequence configured to couple to a barcode nucleic acid molecule of the plurality of barcode nucleic acid molecules;

c) in bulk or in the partition, generating a barcoded nucleic acid molecule from the barcode nucleic acid molecule and the reporter nucleic acid molecule, wherein the barcoded nucleic acid molecule comprises the reporter sequence or a complement thereof and the common partition-specific barcode sequence or a complement thereof; and

d) sequencing the barcoded nucleic acid molecules to obtain a nucleic acid sequence of the barcoded nucleic acid molecule, optionally wherein the barcoded nucleic acid molecule is amplified prior to sequencing.

9. The method of claim 8, wherein:

(a) the antibody-secreting cell is of a B-cell lineage, optionally wherein the B-lineage cell is a plasma cell;

(b) said matrix is a degradable polymer matrix;

(c) said capture agent is a first antibody binding agent; and/or(d) said reporter agent is a second antibody binding agent.

10. The method of claim 8 or claim 9, wherein said cell or said labelled cell bead further comprises a second reporter agent comprising a second reporter nucleic acid molecule, optionally wherein:

> (a) said second reporter agent is configured to couple to a cell surface protein of the cell; and/or
> (b) said second reporter nucleic acid molecule comprises a second reporter sequence corresponding to the second reporter agent.

11. The method of claim any of claims 8-10, wherein the partition further comprises a second barcode nucleic acid molecule of a plurality of second barcode nucleic acid molecules, wherein the second barcode nucleic acid molecule of the plurality of second barcode nucleic acid molecules comprise the common partition-specific barcode sequence, optionally wherein:

> (a) said second reporter nucleic acid molecule comprises a second sequence configured to couple to the second barcode nucleic acid molecule; and/or
> (b) step (c) further comprises generating a second barcoded nucleic acid molecule of a second barcode nucleic acid molecule from the plurality of second barcode nucleic acid molecules and the second reporter nucleic acid molecule, wherein the second barcoded nucleic acid molecule comprises the second reporter sequence from the second reporter nucleic acid molecule and the common partition-specific barcode sequence of the second barcode nucleic acid molecule, or complements thereof.

12. The method of any of claims 8-11, wherein said cell further comprises a plurality of nucleic acid analytes, wherein a nucleic acid analyte of said plurality of nucleic acid analytes comprises a nucleic acid analyte sequence.

13. The method of any of claims 8-12, wherein the partition further comprises a plurality of third barcode nucleic acid molecules comprising the common partition-specific barcode sequence, optionally wherein:

> (a) a third barcode nucleic acid molecule of said plurality of third barcode nucleic acid molecules comprises a third sequence configured to couple to the nucleic acid analyte sequence; and/or
> (b) step (c) further comprises generating a third barcoded nucleic acid molecule of a third barcode nucleic acid molecule from the plurality of third barcode nucleic acid molecules and the nucleic acid analyte, wherein the third barcoded nucleic acid molecule comprises sequences from the nucleic acid analyte and the common partition-specific barcode sequence of the third barcode nucleic acid molecule, or complements thereof.

14. The method of any of claims 8-13, wherein said partition comprises a support that comprises the plurality of barcode nucleic acid molecules, optionally wherein:

> (a) the support is a bead, optionally wherein the bead is a gel bead; and/or
> (b) said plurality of barcode nucleic acid molecules are releasably attached to the support.

15. The method of any of claims 1-14, wherein the partition is from a plurality of partitions, optionally wherein the partition is a droplet or a microwell.

**Patentansprüche**

1. Verfahren zur Einzelzellanalyse, umfassend:

> a) Inkontaktbringen einer Antikörper sekretierenden Zelle mit einem ein Reporternukleinsäuremolekül umfassenden Reportermittel zum Bereitstellen einer markierten Zelle, wobei das Reporternukleinsäuremolekül eine dem Reportermittel entsprechende Reportersequenz umfasst, wobei die markierte Zelle einen an eine Oberfläche der Zelle gekoppelten Komplex umfasst, und wobei der Komplex (i) ein Einfangmittel, (ii) einen sekretierten Antikörper oder ein antigenbindendes Fragment davon, und (iii) das Reportermittel umfasst, wobei das Einfangmittel zum Koppeln sowohl an ein Zelloberflächenmolekül als auch an den sekretierten Antikörper oder das antigenbindende Fragment davon ausgelegt ist, und wobei das Reportermittel zum Koppeln an den sekretierten Antikörper oder das antigenbindende Fragment davon ausgelegt ist;
> b) Partitionieren der markierten Zelle in eine Partition, wobei die Partition eine Vielzahl von Barcode-Nukleinsäuremolekülen umfasst, die eine gemeinsame partitionsspezifische Barcode-Sequenz umfassen, wobei das Reporternukleinsäuremolekül ferner eine zum Koppeln an ein Barcode-Nukleinsäuremolekül der Vielzahl von Barcode-Nukleinsäuremolekülen ausgelegte Sequenz umfasst;
> c) in der Masse oder in der Partition, Erzeugen eines barcodierten Nukleinsäuremoleküls aus dem Barcode-Nukleinsäuremolekül und dem Reporternukleinsäuremolekül, wobei das barcodierte Nukleinsäuremolekül die Reportersequenz oder ein Komplement davon und die gemeinsame partitionsspezifische Barcode-Sequenz oder ein Komplement davon umfasst; und

d) Sequenzieren des barcodierten Nukleinsäuremoleküls, um eine Nukleinsäuresequenz des barcodierten Nukleinsäuremoleküls zu erhalten, wobei das barcodierte Nukleinsäuremolekül optional vor der Sequenzierung amplifiziert wird.

2. Verfahren nach Anspruch 1, wobei:

   (a) die Antikörper sekretierende Zelle zu einer B-Zelllinie gehört, wobei die Zelle der B-Zelllinie optional eine Plasmazelle ist;
   (b) das Zelloberflächenmolekül ein Zelloberflächenprotein ist;
   (c) das Einfangmittel ein erstes Antikörperbindemittel ist; und/oder
   (d) das Reportermittel ein zweites Antikörperbindemittel ist.

3. Verfahren nach Anspruch 1 oder Anspruch 2, wobei die markierte Zelle ferner ein zweites Reportermittel umfasst, das ein zweites Reporternukleinsäuremolekül umfasst, optional wobei:

   (a) das zweite Reportermittel zum Koppeln an ein zweites Zelloberflächenmolekül ausgelegt ist, wobei das zweite Zelloberflächenmolekül ein zweites Zelloberflächenprotein der Zelle ist; und/oder
   (b) das zweite Reporternukleinsäuremolekül eine dem zweiten Reportermittel entsprechende zweite Reportersequenz umfasst.

4. Verfahren nach einem der Ansprüche 1 bis 3, wobei die Partition ferner ein zweites Barcode-Nukleinsäuremolekül einer Vielzahl von zweiten Barcode-Nukleinsäuremolekülen umfasst, wobei das zweite Barcode-Nukleinsäuremolekül der Vielzahl von zweiten Barcode-Nukleinsäuremolekülen die gemeinsame partitionsspezifische Barcode-Sequenz umfasst, optional wobei:

   (a) das zweite Reporternukleinsäuremolekül eine zweite Sequenz umfasst, die zum Koppeln an das zweite Barcode-Nukleinsäuremolekül ausgelegt ist; und/oder
   (b) Schritt (c) ferner das Erzeugen eines zweiten barcodierten Nukleinsäuremoleküls aus einem zweiten Barcode-Nukleinsäuremolekül aus der Vielzahl von zweiten Barcode-Nukleinsäuremolekülen und dem zweiten Reporternukleinsäuremolekül umfasst, wobei das zweite barcodierte Nukleinsäuremolekül die zweite Reportersequenz aus dem zweiten Reporternukleinsäuremolekül und den gemeinsamen partitionsspezifischen Barcode aus dem zweiten Barcode-Nukleinsäuremolekül oder Komplemente davon umfasst.

5. Verfahren nach einem der Ansprüche 1 bis 4, wobei die markierte Zelle ferner eine Vielzahl von Nukleinsäureanalyten umfasst, wobei ein Nukleinsäureanalyt der Vielzahl von Nukleinsäureanalyten eine Nukleinsäureanalytsequenz umfasst.

6. Verfahren nach einem der Ansprüche 1 bis 5, wobei die Partition ferner eine Vielzahl von dritten Barcode-Nukleinsäuremolekülen umfasst, die die gemeinsame partitionsspezifische Barcode-Sequenz umfassen, optional wobei:

   (a) ein drittes Barcode-Nukleinsäuremolekül der Vielzahl von dritten Barcode-Nukleinsäuremolekülen eine dritte Sequenz umfasst, die zum Koppeln an die Nukleinsäureanalytsequenz ausgelegt ist; und/oder
   (b) Schritt (c) ferner das Erzeugen eines dritten barcodierten Nukleinsäuremoleküls aus einem dritten Barcode-Nukleinsäuremolekül aus der Vielzahl von dritten Barcode-Nukleinsäuremolekülen und dem Nukleinsäureanalyten umfasst, wobei das dritte barcodierte Nukleinsäuremolekül Sequenzen aus dem Nukleinsäureanalyten und die gemeinsame partitionsspezifische Barcode-Sequenz aus dem dritten Barcode-Nukleinsäuremolekül oder Komplemente davon umfasst.

7. Verfahren nach einem der Ansprüche 1 bis 6, wobei die Partition einen Träger umfasst, der die Vielzahl der Barcode-Nukleinsäuremoleküle umfasst, optional, wobei:

   (a) der Träger ein Kügelchen ist, wobei das Kügelchen optional ein Gelkügelchen ist; und/oder
   (b) die Vielzahl von Barcode-Nukleinsäuremolekülen freisetzbar an dem Träger angelagert ist.

8. Verfahren zur Einzelzellanalyse, umfassend:

   a) Inkontaktbringen eines Zellkügelchens mit einem ein Reporternukleinsäuremolekül umfassenden Reportermittel zum Bereitstellen eines markierten Zellkügelchens, wobei das Zellkügelchen eine Antikörper sekretierende Zelle in einer Matrix umfasst, wobei das Reporternukleinsäuremolekül eine dem Reportermittel entsprechende Reportersequenz umfasst, wobei das markierte Zellkügelchen einen Komplex in oder auf dem Zellkügelchen umfasst, und wobei der Komplex (i) ein Einfangmittel, (ii) einen sekretierten Antikörper oder ein antigenbindendes Fragment davon, und (iii) das Reportermittel umfasst, wobei das Einfangmittel zum Koppeln sowohl an die Matrix als auch an den sekretierten Antikörper oder das antigenbindende Fragment davon ausgelegt ist, und wobei das Reportermittel zum Koppeln an den sekretierten Antikörper oder das antigenbindende Fragment davon ausgelegt ist;

b) Partitionieren des markierten Zellkügelchens in eine Partition, wobei die Partition eine Vielzahl von Barcode-Nukleinsäuremolekülen umfasst, die eine gemeinsame partitionsspezifische Barcode-Sequenz umfassen, wobei das Reporternukleinsäuremolekül ferner eine zum Koppeln an ein Barcode-Nukleinsäuremolekül der Vielzahl von Barcode-Nukleinsäuremolekülen ausgelegte Sequenz umfasst;

c) in der Masse oder in der Partition, Erzeugen eines barcodierten Nukleinsäuremoleküls aus dem Barcode-Nukleinsäuremolekül und dem Reporternukleinsäuremolekül, wobei das barcodierte Nukleinsäuremolekül die Reportersequenz oder ein Komplement davon und die gemeinsame partitionsspezifische Barcode-Sequenz oder ein Komplement davon umfasst; und

d) Sequenzieren der barcodierten Nukleinsäuremoleküle, um eine Nukleinsäuresequenz des barcodierten Nukleinsäuremoleküls zu erhalten, wobei das barcodierte Nukleinsäuremolekül optional vor der Sequenzierung amplifiziert wird.

9. Verfahren nach Anspruch 8, wobei:

   (a) die Antikörper sekretierende Zelle zu einer B-Zelllinie gehört, wobei die B-Zelllinie optional eine Plasmazelle ist;
   (b) die Matrix eine abbaubare Polymermatrix ist;
   (c) das Einfangmittel ein erstes Antikörperbindemittel ist; und/oder (d) das Reportermittel ein zweites Antikörperbindemittel ist.

10. Verfahren nach Anspruch 8 oder Anspruch 9, wobei die Zelle oder das markierte Zellkügelchen ferner ein zweites Reportermittel umfasst, das ein zweites Reporternukleinsäuremolekül umfasst, optional, wobei:

   (a) das zweite Reportermittel zum Koppeln an ein Zelloberflächenprotein der Zelle ausgelegt ist; und/oder
   (b) das zweite Reporternukleinsäuremolekül eine dem zweiten Reportermittel entsprechende zweite Reportersequenz umfasst.

11. Verfahren nach einem der Ansprüche 8 bis 10, wobei die Partition ferner ein zweites Barcode-Nukleinsäuremolekül einer Vielzahl von zweiten Barcode-Nukleinsäuremolekülen umfasst, wobei das zweite Barcode-Nukleinsäuremolekül der Vielzahl von zweiten Barcode-Nukleinsäuremolekülen die gemeinsame partitionsspezifische Barcode-Sequenz umfasst, optional wobei:

   (a) das zweite Reporternukleinsäuremolekül eine zweite Sequenz umfasst, die zum Koppeln an das zweite Barcode-Nukleinsäuremolekül ausgelegt ist; und/oder
   (b) Schritt (c) ferner das Erzeugen eines zweiten barcodierten Nukleinsäuremoleküls eines zweiten Barcode-Nukleinsäuremoleküls aus der Vielzahl von zweiten Barcode-Nukleinsäuremolekülen und dem zweiten Reporternukleinsäuremolekül umfasst, wobei das zweite barcodierte Nukleinsäuremolekül die zweite Reportersequenz aus dem zweiten Reporternukleinsäuremolekül und den gemeinsamen partitionsspezifischen Barcode des zweiten Barcode-Nukleinsäuremoleküls oder Komplemente davon umfasst.

12. Verfahren nach einem der Ansprüche 8 bis 11, wobei die Zelle ferner eine Vielzahl von Nukleinsäureanalyten umfasst, wobei ein Nukleinsäureanalyt der Vielzahl von Nukleinsäureanalyten eine Nukleinsäureanalytsequenz umfasst.

13. Verfahren nach einem der Ansprüche 8 bis 12, wobei die Partition ferner eine Vielzahl von dritten Barcode-Nukleinsäuremolekülen umfasst, die die gemeinsame partitionsspezifische Barcode-Sequenz umfassen, optional wobei:

   (a) ein drittes Barcode-Nukleinsäuremolekül der Vielzahl von dritten Barcode-Nukleinsäuremolekülen eine dritte Sequenz umfasst, die zum Koppeln an die Nukleinsäureanalytsequenz ausgelegt ist; und/oder (b) Schritt (c) ferner das Erzeugen eines dritten barcodierten Nukleinsäuremoleküls eines dritten Barcode-Nukleinsäuremoleküls aus der Vielzahl von dritten Barcode-Nukleinsäuremolekülen und dem Nukleinsäureanalyten umfasst, wobei das dritte barcodierte Nukleinsäuremolekül Sequenzen aus dem Nukleinsäureanalyten und die gemeinsame partitionsspezifische Barcode-Sequenz des dritten Barcode-Nukleinsäuremoleküls oder Komplemente davon umfasst.

14. Verfahren nach einem der Ansprüche 8 bis 13, wobei die Partition einen Träger umfasst, der die Vielzahl der Barcode-Nukleinsäuremoleküle umfasst, optional, wobei:

(a) der Träger ein Kügelchen ist, wobei das Kügelchen optional ein Gelkügelchen ist; und/oder
(b) die Vielzahl von Barcode-Nukleinsäuremolekülen freisetzbar an dem Träger angelagert ist.

15. Verfahren nach einem der Ansprüche 1 bis 14, wobei die Partition aus einer Vielzahl von Partitionen besteht, wobei die Partition optional ein Tröpfchen oder eine Mikrovertiefung ist.

**Revendications**

1. Procédé d'analyse de cellule isolée, comprenant :

    a) la mise en contact d'une cellule sécrétrice d'anticorps avec un agent rapporteur comprenant une molécule d'acide nucléique rapporteur pour fournir une cellule marquée, dans lequel la molécule d'acide nucléique rapporteur comprend une séquence rapporteur correspondant à l'agent rapporteur, dans lequel la cellule marquée comprend un complexe couplé à une surface de la cellule, et dans lequel le complexe comprend (i) un agent de capture, (ii) un anticorps sécrété ou un fragment de liaison aux antigènes de celui-ci, et (iii) l'agent rapporteur, dans lequel l'agent de capture est configuré pour se coupler à la fois à une molécule de surface cellulaire et à l'anticorps sécrété ou au fragment de liaison à l'antigène de celui-ci, et dans lequel l'agent rapporteur est configuré pour se coupler à l'anticorps sécrété ou à son fragment de liaison aux antigènes ;
    b) la séparation de la cellule marquée dans un compartiment, dans lequel le compartiment comprend une pluralité de molécules d'acide nucléique à code-barres qui comprennent une séquence de code-barres spécifique à un compartiment commune, dans lequel ladite molécule d'acide nucléique rapporteur comprend en outre une séquence configurée pour se coupler à une molécule d'acide nucléique à code-barres de la pluralité de molécules d'acide nucléique à code-barres ;
    c) dans la masse ou dans le compartiment, la génération d'une molécule d'acide nucléique à code-barres à partir de la molécule d'acide nucléique à code-barres et de la molécule d'acide nucléique rapporteur, dans lequel la molécule d'acide nucléique à code-barres comprend la séquence rapporteur ou un complément de celle-ci et la séquence de code-barres spécifique au compartiment commune ou un complément de celle-ci ; et
    d) le séquençage de la molécule d'acide nucléique à code-barres pour obtenir une séquence d'acide nucléique de la molécule d'acide nucléique à code-barres, facultativement dans lequel la molécule d'acide nucléique à code-barres est amplifiée avant le séquençage.

2. Procédé selon la revendication 1, dans lequel :

    (a) la cellule sécrétrice d'anticorps est d'une lignée de cellules B, facultativement dans lequel la cellule de lignée de cellules B est une cellule plasmatique ;
    (b) ladite molécule de surface cellulaire est une protéine de surface cellulaire ;
    (c) ledit agent de capture est un premier agent de liaison aux anticorps ; et/ou
    (d) ledit agent rapporteur est un deuxième agent de liaison aux antigènes.

3. Procédé selon la revendication 1 ou la revendication 2, dans lequel ladite cellule marquée comprend en outre un deuxième agent rapporteur comprenant une deuxième molécule d'acide nucléique rapporteur, facultativement dans lequel :

    (a) ledit deuxième agent rapporteur est configuré pour se coupler à une deuxième molécule de surface cellulaire, dans lequel la deuxième molécule de surface cellulaire est une deuxième protéine de surface cellulaire de la cellule ; et/ou
    (b) ladite deuxième molécule d'acide nucléique rapporteur comprend une deuxième séquence rapporteur correspondant au deuxième agent rapporteur.

4. Procédé selon l'une quelconque des revendications 1 à 3, dans lequel le compartiment comprend en outre une deuxième molécule d'acide nucléique à code-barres d'une pluralité de deuxièmes molécules d'acide nucléique à code-barres, dans lequel la deuxième molécule d'acide nucléique à code-barres de la pluralité de deuxièmes molécules d'acide nucléique à code-barres comprend la séquence à code-barres spécifique de compartiment commune, facultativement dans lequel :

    (a) ladite deuxième molécule d'acide nucléique rapporteur comprend une deuxième séquence configurée pour se coupler à la deuxième molécule d'acide nucléique à code-barres ; et/ou
    (b) l'étape (c) comprend en outre la génération d'une deuxième molécule d'acide nucléique à code-barres à partir d'une deuxième molécule d'acide nucléique à code-barres de la pluralité de deuxièmes molécules d'acide nucléique à code-barres et de la deuxième molécule d'acide nucléique rapporteur, dans lequel la deuxième molécule d'acide nucléique à code-barres comprend la deuxième séquence rapporteur provenant de la deuxième molécule d'acide nucléique à code-barres et le code-barres spécifique au compartiment commun provenant de la deuxième molécule d'acide nucléique à code-barres, ou de compléments de celle-ci.

**5.** Procédé selon l'une quelconque des revendications 1 à 4, dans lequel ladite cellule marquée comprend en outre une pluralité d'analytes d'acide nucléique, dans lequel un analyte d'acide nucléique de ladite pluralité d'analytes d'acide nucléique comprend une séquence d'analyte d'acide nucléique.

**6.** Procédé selon l'une quelconque des revendications 1 à 5, dans lequel le compartiment comprend en outre une pluralité de troisièmes molécules d'acide nucléique à code-barres comprenant la séquence de code-barres spécifique au compartiment commune, facultativement dans lequel :

(a)une troisième molécule d'acide nucléique à code-barres de ladite pluralité de troisièmes molécules d'acide nucléique à code-barres comprend une troisième séquence configurée pour se coupler à la séquence d'analyte d'acide nucléique ; et/ou
(b)l'étape (c) comprend en outre la génération d'une troisième molécule d'acide nucléique codée par code-barres à partir d'une troisième molécule d'acide nucléique à code-barres de la pluralité de troisièmes molécules d'acide nucléique à code-barres et de l'analyte d'acide nucléique, dans lequel la troisième molécule d'acide nucléique codé par code-barres comprend des séquences provenant de l'analyte d'acide nucléique et de la séquence de code-barres spécifique au compartiment commune provenant de la troisième molécule d'acide nucléique à code-barres, ou de compléments de celles-ci.

**7.** Procédé selon l'une quelconque des revendications 1 à 6, dans lequel ledit compartiment comprend un support qui comprend la pluralité de molécules d'acide nucléique à code-barres, facultativement dans lequel :

(a)le support est une bille, facultativement dans lequel la bille est une bille de gel ; et/ou
(b)ladite pluralité de molécules d'acide nucléique à code-barres sont fixées de manière libérable au support.

**8.** Procédé d'analyse de cellule isolée, comprenant :

a)la mise en contact d'une bille cellulaire avec un agent rapporteur comprenant une molécule d'acide nucléique rapporteur pour fournir une bille cellulaire marquée, dans lequel la bille cellulaire comprend une cellule sécrétrice d'anticorps dans une matrice, dans lequel la molécule d'acide nucléique rapporteur comprend une séquence rapporteur correspondant à l'agent rapporteur, dans lequel la bille cellulaire marquée comprend un complexe dans ou sur la bille cellulaire, et dans lequel le complexe comprend (i) un agent de capture, (ii) un anticorps sécrété ou un fragment de liaison aux antigènes de celui-ci, et (iii) l'agent rapporteur, dans lequel ledit agent de capture est configuré pour se coupler à la fois à la matrice et audit anticorps sécrété ou au fragment de liaison aux antigènes de celui-ci, et dans lequel l'agent rapporteur est configuré pour se coupler à l'anticorps sécrété ou au fragment de liaison aux antigènes de celui-ci ;
b)la séparation de la bille cellulaire marquée dans une partition, dans lequel la partition comprend une pluralité de molécules d'acide nucléique à code-barres qui comprennent une séquence de code-barres spécifique à un compartiment commune, dans lequel ladite molécule d'acide nucléique rapporteur comprend en outre une séquence configurée pour se coupler à une molécule d'acide nucléique à code-barres de la pluralité de molécules d'acide nucléique à code-barres ;
c)dans la masse ou dans le compartiment, la génération d'une molécule d'acide nucléique à code-barres à partir de la molécule d'acide nucléique à code-barres et de la molécule d'acide nucléique rapporteur, dans lequel la molécule d'acide nucléique à code-barres comprend la séquence rapporteur ou un complément de celle-ci et la séquence de code-barres spécifique au compartiment commune ou un complément de celle-ci ; et
d)le séquençage des molécules d'acide nucléique à code-barres pour obtenir une séquence d'acide nucléique de la molécule d'acide nucléique à code-barres, facultativement dans lequel la molécule d'acide nucléique à code-barres est amplifiée avant le séquençage.

**9.** Procédé selon la revendication 8, dans lequel :

(a) la cellule sécrétrice d'anticorps est d'une lignée de cellules B, facultativement dans lequel la cellule de lignée B est une cellule plasmatique ;
(b) ladite matrice est une matrice polymère dégradable ;
(c) ledit agent de capture est un premier agent de liaison aux anticorps ; et/ou (d) ledit agent rapporteur est un deuxième agent de liaison aux antigènes.

**10.** Procédé selon la revendication 8 ou la revendication 9, dans lequel ladite cellule ou ladite bille cellulaire marquée comprend en outre un deuxième agent rapporteur comprenant une deuxième molécule d'acide nucléique rapporteur, facultativement dans lequel :

(a) ledit deuxième agent rapporteur est configuré pour se coupler à une protéine de surface cellulaire de la cellule ; et/ou
(b) ladite deuxième molécule d'acide nucléique rapporteur comprend une deuxième séquence rapporteur correspondant au deuxième agent rapporteur.

**11.** Procédé selon l'une quelconque des revendications 8 à 10, dans lequel le compartiment comprend en outre une deuxième molécule d'acide nucléique à code-barres d'une pluralité de deuxièmes molécules d'acide nucléique à code-barres, dans lequel la deuxième molécule d'acide nucléique à code-barres de la pluralité de deuxièmes molécules d'acide nucléique à code-barres comprend la séquence à code-barres spécifique de compartiment commune, facultativement dans lequel :

(a) ladite deuxième molécule d'acide nucléique rapporteur comprend une deuxième séquence configurée pour se coupler à la deuxième molécule d'acide nucléique à code-barres ; et/ou
(b) l'étape (c) comprend en outre la génération d'une deuxième molécule d'acide nucléique à code-barres d'une deuxième molécule d'acide nucléique à code-barres de la pluralité de deuxièmes molécules d'acide nucléique à code-barres et de la deuxième molécule d'acide nucléique rapporteur, dans lequel la deuxième molécule d'acide nucléique à code-barres comprend la deuxième séquence rapporteur provenant de la deuxième molécule d'acide nucléique à code-barres et la séquence de code-barres spécifique au compartiment commune de la deuxième molécule d'acide nucléique à code-barres, ou de compléments de celle-ci.

**12.** Procédé selon l'une quelconque des revendications 8 à 11, dans lequel ladite cellule comprend en outre une pluralité d'analytes d'acide nucléique, dans lequel un analyte d'acide nucléique de ladite pluralité d'analytes d'acide nucléique comprend une séquence d'analyte d'acide nucléique.

**13.** Procédé selon l'une quelconque des revendications 8 à 12, dans lequel le compartiment comprend en outre une pluralité de troisièmes molécules d'acide nucléique à code-barres comprenant la séquence de code-barres spécifique au compartiment commune, facultativement dans lequel :

(a) une troisième molécule d'acide nucléique à code-barres de ladite pluralité de troisièmes molécules d'acide nucléique à code-barres comprend une troisième séquence configurée pour se coupler à la séquence d'analyte d'acide nucléique ; et/ou
(b) l'étape (c) comprend en outre la génération d'une troisième molécule d'acide nucléique codée par code-barres d'une troisième molécule d'acide nucléique à code-barres de la pluralité de troisièmes molécules d'acide nucléique à code-barres et de l'analyte d'acide nucléique, dans lequel la troisième molécule d'acide nucléique codé par code-barres comprend des séquences provenant de l'analyte d'acide nucléique et de la séquence de code-barres spécifique au compartiment commune de la troisième molécule d'acide nucléique à code-barres, ou de compléments de celles-ci.

**14.** Procédé selon l'une quelconque des revendications 8 à 13, dans lequel ledit compartiment comprend un support qui comprend la pluralité de molécules d'acide nucléique à code-barres, facultativement dans lequel :

(a) le support est une bille, facultativement dans lequel la bille est une bille de gel ; et/ou
(b) ladite pluralité de molécules d'acide nucléique à code-barres sont fixées de manière libérable au support.

**15.** Procédé selon l'une quelconque des revendications 1 à 14, dans lequel le compartiment provient d'une pluralité de compartiments, facultativement dans lequel le compartiment est une gouttelette ou une cupule.

EP 4 225 934 B1

**FIG. 1**

**FIG. 2**

EP 4 225 934 B1

**FIG. 3**

EP 4 225 934 B1

400

404

406

408 410 412

403

470 465

475

472

462

464

405

460

485

482

480

407

*FIG. 4*

**FIG. 5**

EP 4 225 934 B1

**FIG. 6**

**700**

702 — Cells of B-cell lineage, e.g., plasma cells, are stimulated to allow secretion of antibodies

704 — An antigen specific capture agent (e.g., antibody binding agent), configured to couple to both a cell surface molecule and a secreted antibody or antigen binding fragment thereof, creates a complex coupled to the surface of the cell

706 — Cells are incubated with a reporter agent configured to couple to (bind) the secreted antibody or antigen binding fragment thereof (comprising a first barcode), to provide a labelled cell

Cells are partitioned, optionally into droplets (e.g., emulsion droplets); partitions may comprise beads comprising a second barcode sequence (e.g., partition-specific) — 708

Cells are lysed inside the partitions (e.g., droplets or wells) to release intracellular constituent (e.g., secreted antibodies or antigen binding fragment thereof) and barcodes attached to polypeptides — 710

Reverse transcription of the released mRNAs; attaching second (e.g., partition-specific) barcodes to cDNA generated from mRNA and to the released first (analyte-specific) barcodes; amplifying nucleic acid molecules — 712

Pooling partitions (e.g., droplets or wells) into a bulk solution and further processing to convert into sequencing library — 714

*FIG. 7*

EP 4 225 934 B1

*FIG. 8*

**900**

**FIG. 9**

Cells of B-cell lineage, e.g., plasma cells are encapsulated into droplets which subsequently gel into a hydrogel matrix encapsulating the cell, forming a cell bead — **902**

Cell beads are incubated with stimuli to allow secretion of antibodies; secreted antibodies or antigen binding fragments thereof are coupled to, e.g., linked to, the backbone of the polymer forming the hydrogel matrix via a capture agent configured to couple to both the secreted antibodies or antigen binding fragment thereof, and the cell bead matrix — **904**

After capturing the secreted antibodies, the cell beads are partially digested by enzymes so that a plurality of reporter agents with barcodes can have access to the captured secreted antibodies or antigen binding fragments thereof, and label partially digested cell beads with said plurality of reporter agents — **906**

Cell beads are partitioned, optionally into droplets (e.g., emulsion droplets); partitions may comprise beads comprising a second barcode sequence (e.g., partition-specific) — **908**

Cells are lysed inside the partitions (e.g., droplets or wells) to release intracellular constituent (e.g., secreted antibodies or antigen binding fragment thereof) and barcodes attached to polypeptides — **910**

Reverse transcription of the released mRNAs; attaching second (e.g., partition-specific) barcodes to cDNA generated from mRNA and to the released first (analyte-specific) barcodes; amplifying nucleic acid molecules — **912**

Pooling partitions (e.g., droplets or wells) into a bulk solution and further processing to convert into sequencing library — **914**

*FIG. 10A*

1000

1012

1002

1014

1016

FIG. 10B

**FIG. 11**

## 1200

**1201**
Cell Surface
proteins

**1202**
Antigen
Specificity

**1203**
Secreted
Proteins

**1204**
mRNA

*FIG. 12*

EP 4 225 934 B1

1300

FIG. 13

EP 4 225 934 B1

<u>1400</u>

FIG. 14

*FIG. 15A*

*FIG. 15B*

EP 4 225 934 B1

**FIG. 15C**

**FIG. 15D**

EP 4 225 934 B1

**1600**

**FIG. 16**

**EP 4 225 934 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20190100632 A
- US 10590244 B
- US 20180216162 A
- US 10428326 B
- US 20190233878 A
- US 2019023878 A
- WO 2015157567 A
- WO 2020167862 A
- WO 2020176882 A
- US 20190367997 A
- US 20190064173 A
- US 20140155295

- US 20100105112
- US 20140378345
- US 20150292988 A
- US 20140378345 A
- US 20150376609 A
- WO 2019165181 A1
- US 20180105808 A
- WO 2019157529 A
- US 10550429 B
- US 20190177800 A
- US 20190367969 A
- US 20190323088 A
- US 6265552 B
- WO 2018075693 A

### Non-patent literature cited in the description

- Genome Analysis: A Laboratory Manual Series. 1999, vol. I-IV
- Genetic Variation: A Laboratory Manual. 2007
- PCR Primer: A Laboratory Manual; Bowtell and Sambrook. 2003
- DNA Microarrays: A Molecular Cloning Manual. 2004
- **SAMBROOK ; RUSSELL.** Bioinformatics: Sequence and Genome Analysis;. 2006
- **SAMBROOK ; RUSSELL.** Condensed Protocols from Molecular Cloning: A Laboratory Manual. 2002
- Molecular Cloning: A Laboratory Manual. Cold Spring Harbor Laboratory Press
- **STRYER, L.** Biochemistry. W. H. Freeman, 1995
- **GAIT.** Oligonucleotide Synthesis: A Practical Approach. IRL Press, 1984

- **NELSON ; COX.** Principles of Biochemistry. W. H. Freeman Pub, 2000
- **BERG et al.** Biochemistry. W. H. Freeman Pub, 2002
- *Angew. Chem. Int.,* 2001, vol. 40 (11), 2004-2021
- *Pharm Res.,* 2008, vol. 25 (10), 2216-2230
- *Chem. Commun.,* 2011, vol. 47, 6257-6259
- *Nature,* 2015, vol. 519 (7544), 486-90
- **MADL ; HEILSHOM.** Bioorthogonal Strategies for Engineering Extracellular Matrices. *Adv. Funct. Mater,* 2018, vol. 28, 1706046
- **HUGHES L D et al.** *PLoS One,* 04 February 2014, vol. 9 (2), e87649
- **FANG et al.** Fluoride-Cleavable Biotinylation Phosphoramidite for 5'-end-Labelling and Affinity Purification of Synthetic Oligonucleotides. *Nucleic Acids Res.,* 15 January 2003, vol. 3 1 (2), 708-7